(19) Europäisches Patentamt
European Patent Office
Office européen des brevets

(11) EP 3 193 659 B1

(12) # EUROPEAN PATENT SPECIFICATION

(45) Date of publication and mention of the grant of the patent:
**16.12.2020 Bulletin 2020/51**

(21) Application number: **15775802.0**

(22) Date of filing: **26.08.2015**

(51) Int Cl.:
***A45D 34/04*** *(2006.01)* ***A45D 40/26*** *(2006.01)*
***A61Q 19/00*** *(2006.01)* ***A61K 8/92*** *(2006.01)*
***A61K 8/06*** *(2006.01)* ***A61K 8/04*** *(2006.01)*

(86) International application number:
**PCT/IB2015/056473**

(87) International publication number:
**WO 2016/030841 (03.03.2016 Gazette 2016/09)**

(54) **NOVEL CARE AND/OR MAKEUP DEVICE COMPRISING A COMPOSITION OF GEL/GEL ARCHITECTURE**

MAKEUP-VORRICHTUNG MIT EINER GEL/GEL-ZUSAMMENSETZUNG

DISPOSITIF DE MAQUILLAGE COMPRÉNANT UNE COMPOSITION DU TYPE GEL/GEL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.08.2014 FR 1458060**

(43) Date of publication of application:
**26.07.2017 Bulletin 2017/30**

(73) Proprietor: **L'OREAL**
**75008 Paris (FR)**

(72) Inventors:
- **VALVERDE, Elodie**
**26400 Gigors-et-Lozeron (FR)**
- **FERRARI, Véronique**
**94700 Maisons - Alfort (FR)**
- **CAULIER, Eric**
**60420 Ferrieres (FR)**
- **ROUDAUT, Etienne**
**92250 La Garenne Colombes (FR)**

(74) Representative: **Nony**
**11 rue Saint-Georges**
**75009 Paris (FR)**

(56) References cited:
**WO-A1-98/44012**
**WO-A1-2014/128680**
**WO-A2-2013/093869**
**US-A1- 2009 311 035**
**US-B1- 6 186 686**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 193 659 B1

## Description

The present invention relates to the field of caring for and/or making up keratin materials.

The invention is more particularly directed towards proposing a novel care and/or makeup device combining a composition of gel/gel architecture and a specific applicator for giving the user, during the application of such a composition, an unexpected perception of lightness. WO 2013/093869 and US 2009/311035 are some examples of the compositions and dispensers of the prior art.

The term "keratin materials" especially means the skin, the lips, keratin fibres such as the eyebrows and/or the eyelashes, in particular the skin and/or the eyebrows, and preferably the skin.

It is clear that the consumers of products for making up and/or caring for the skin or the lips, more particularly intended for the face, all generally expect to have products that afford a natural and uniform makeup result with prolonged staying power, while at the same time giving them a sensation of lightness or even freshness on application. For obvious reasons, aqueous architectures are preferred architectures for satisfying this last requirement.

However, a composition which has a high content of aqueous phase, which is advantageous with regard to the freshness properties it affords, cannot always provide uniform distribution of the particulate materials it moreover contains.

More recently, the inventors have discovered a novel galenical form that is most particularly advantageous with regard to its technical performance and the sensory perceptions it gives the user during its application in particular to the skin. These are compositions comprising at least one aqueous phase gelled with at least one hydrophilic gelling agent, and at least one oily phase gelled with at least one lipophilic gelling agent; said phases forming therein a macroscopically homogeneous mixture.

Unfortunately, these compositions, with regard precisely to this gel/gel architecture, and thus their slightly thickened fluid appearance, may be less appealing to users, who are always in search of a sensation of lightness on application. In point of fact, this gel/gel architecture runs the risk of being perceived by the user as too heavy and/or too creamy. This preconception is moreover very often unjustified insofar as there is no immediate correlation between the density visually perceived for a composition in its packaging and the spreading qualities of this composition especially in terms of lightness.

For obvious reasons, it is desirable to overcome such a prejudice.

The present invention is precisely directed towards satisfying this need.

Thus, according to one of its aspects, the present invention relates to a device for packaging and applying a cosmetic or dermatological product, comprising:

- a composition, especially a cosmetic composition, for making up and/or caring for keratin materials, in particular the skin and/or the lips, comprising at least one aqueous phase gelled with at least one hydrophilic gelling agent chosen from 2-acrylamido-2-methylpropanesulfonic acid polymers and copolymers and at least one oily phase gelled with at least one lipophilic gelling agent, with said phases forming a macroscopically homogeneous mixture,
- a container containing said composition,
- an applicator head fed with composition by the container, the applicator head being arranged to dispense said composition through a dispensing member having at least one passage for the composition imposing a shear on the composition passing through it, in which the applicator head comprises a grate defining an application surface for applying the product to a surface to be treated and also comprises a partitioned support member for the grate, against which the grate may rest at least during the application, having at least one partition between two zones where the product may pass through the support member.

Contrary to all expectation, the inventors have found that the distribution of a composition as defined above using a device in accordance with the invention makes it possible precisely to give the user the perception of lightness that he is expecting.

Specifically, as emerges from the example below, users of a composition placed in a device according to the invention perceive the application head of this device as being capable of modifying the texture and of significantly increasing the lightness of the composition, or even as being of a nature to give it an airy appearance. The device according to the invention thus makes it possible to erase the high consistency and the thickness that the composition has in the bulk, when it is in a jar.

These users moreover find an ease of metering out and of spreading when compared with a composition packaged in ajar.

According to one embodiment variant, the dispensing member of a device according to the invention has a single passage.

As more precisely concerns the grate, it may be a woven or nonwoven textile or an injection-moulded or extruded net.

It may, for example, be made of thermoplastic material, especially of polyamide 6.6 or of PP, PA or PET.

This grate may especially comprise a plurality of yarns of diameter d between 2 μm and 1000 μm and better still between 50 μm and 250 μm.

By way of example, it may comprise between 20 yarns/cm and 70 yarns/cm and better still between 28 yarns/cm and 35 yarns/cm.

Still as regards said grate, it may be formed from a woven or a net, its meshes each having an aperture of cross section S between 0.01 $mm^2$ and 1 $mm^2$ and better still between 0.01 $mm^2$ and 0. 1 $mm^2$.

According to one embodiment variant, a device according to the invention may comprise a cap for covering the applicator head when the device is not in use.

According to another of its aspects, a subject of the invention is also a process, especially a cosmetic process, for making up and/or caring for keratin materials, in particular the skin and/or the lips, using the device according to the invention, and comprising at least one step that consists in applying to said keratin materials the composition contained in the container of a device as defined above.

The invention may be understood more clearly on reading the detailed description that follows of non-limiting implementation examples thereof, and from examining the attached drawing, in which:

- figure 1 is a partial view, in axial cross section and in perspective, of a device according to the invention,
- figure 2 shows in isolation a supporting part of the device of figure 1,
- figure 3 is a top view of a textile grate that may be used in the device of figure 1, and
- figure 4 is a view of the applicator head of the device of figure 1, after dispensing the product on the grate.

As stated previously, the device according to the invention comprises an applicator head that may comprise one or more passages including at least one that is suitable for shearing the composition that passes through it.

The figures illustrate a first variant of the device according to the invention, in which the applicator head comprises several passages that are all capable of imposing a shear and preferably the same shear on the composition that passes through them. Advantageously, these passages are identical.

More precisely, the packaging and application device 2 shown in figure 1 comprises a container 5 containing the gel/gel composition to be applied (described in greater detail hereinbelow) and an applicator head 10 comprising several passages that are all capable of imposing the same shear on the composition that passes through them.

The applicator head more precisely comprises an insert 13 connected to the neck 25 of the container 5 and a supporting part 16 connected to the insert 13. The supporting part 16 comprises a grate 19 defining an application surface 19a and passages that are capable of imposing a shear on the composition passing through them. The grate 19 is placed on a partitioned support member 22 of the supporting part 16.

The term "partitioned support member" denotes a support member of the grate having a product outlet comprising a plurality of open passage zones for the composition to the grate, separated by one or more partitions. Thus, the partitioned support member has at least one partition between two zones in which the composition may pass through it. Here, the partitioned support member 22 is made from one piece with the supporting part 16.

The container 5 containing the composition may be supple, with a variable internal volume. Feeding the applicator head 10 with composition from the container 5 may then be done by pressing on the wall of the container 5, with the aid of a piston and/or with the aid of a pump.

As illustrated, the neck 25 comprises a flange 28 onto which the insert 13 is click-fastened. The insert 13 comprises a sealing skirt 34 which engages in the neck 25 of the container 5 and forms a throat 35 for extracting the composition contained in the container 5 out of the container. The insert 13 and the container 5 are rotationally rigidly connected.

The insert 13 comprises here an anti-splash nozzle 46 at the outlet of the throat 35 formed by the sealing skirt 34. As illustrated, the anti-splash nozzle 46 comprises a plate 49 arranged perpendicular to the composition outlet axis X, such that the composition leaving the container 5 is diverted in its course and becomes distributed laterally.

The plate 49 has here a diameter substantially equal to that of the throat 35 and is raised relative to the outlet of the throat 35. Preferably, the anti-splash nozzle 46 is placed at a height h of the outlet of the throat 35 of between 0.5 mm and 5 mm.

The supporting part 16 may be click-fastened onto the insert 13 such that the supporting part 16 and the insert 13 are rotationally rigidly connected.

As shown in figure 2, the support member 22 is in the form of an outward dome and comprises partitions in the form of an annular central region 75 and connecting bridges 72 connected to the annular central region 75. The connecting bridges together define disjointed apertures 78, whereas the annular central part 75 defines a central aperture. The grate 19 is in contact with the connecting bridges 72 of the support member 22, against which it rests. The grate 19 is fed with product through the apertures 78. As shown, the grate 19 is convex towards the surface to be treated on account of it bearing against the support member 22.

The grate 19 may, for example, be made of a thermoplastic material, especially of polyamide 6.6 or PP, PA or

PET.

As shown in figures 3 and 4, the grate may be a textile. The textile of the grate 19 is then, preferably, a fabric and comprises a plurality of interlaced yarns 81, arranged along two axes Y and Z that are perpendicular to each other, and that together define meshes 84. The textile of the grate 19 may comprise a plurality of yarns 81 with a diameter d between 2 μm and 1000 μm, better still between 50 μm and 250 μm, even better still between 60 μm and 150 μm. The textile of the grate 19 preferably comprises between 20 yarns/cm and 70 yarns/cm, better still between 28 yarns/cm and 35 yarns/cm. The meshes 84 of the textile of the grate 19 each preferably have an aperture of cross section S between 0.01 $mm^2$ and 1 $mm^2$, better still between 0.01 $mm^2$ and 0.1 $mm^2$ and even better still between 0.03 $mm^2$ and 0.05 $mm^2$. The thickness of the grate 19 is preferably less than 1 mm and better still less than 0.2 mm.

In variants not shown, the grate 19 is formed from a microperforated film or an injection-moulded or extruded net; the grate 19 may also be formed from a perforated nonwoven.

During the distribution of the composition, the composition passes, along the axis X of the applicator head 10, through the apertures 78 of the support member 22 and comes into contact with the grate 19 to pass through it via the apertures 78. Preferably, the product is dispensed on the grate 19 only at its regions that are superposed on the apertures 78 and thus reproduces the pattern defined by the apertures 78. During the application to the surface to be treated, the grate may be moved on the skin. As a variant, the user can take up the product directly on the grate to apply it on the skin, especially by finger.

Moreover, the supporting part 16 can be configured to receive a cap 93, this cap possibly being, for example, screwed onto the supporting part. The cap covers the applicator head 10 to protect the grate 19 from the external medium, and especially to prevent the product from drying out on contact with air, when the device is not in use. The device may comprise a seal that is placed between the supporting part 16 and the cap 93, allowing leaktight closure of the device 2 by the cap 93. Preferably, the insert 13, the supporting part 16 and the cap 93 are made of thermoplastic material, especially of polypropylene.

Needless to say, the device according to the invention is not limited to the example illustrated and is susceptible to numerous variants available to those skilled in the art.

It is possible, for example, to use other materials for making the application head.

The grate may be replaced with a screen or any similar openwork member.

According to a variant of the device also, the applicator head may comprise a single passage, which is capable of imposing a shear on the composition that passes through it. As an illustration of elements capable of featuring a single passage at the outlet of the applicator head, mention may be made especially of a flexible tube, comprising a deformable reservoir and a neck of reduced cross section relative to the reservoir, or a syringe, especially a needleless syringe.

As emerges from the foregoing, the gel/gel composition contained in the device undergoes a mechanical shear during dispensing thereof through the passage(s), when the user presses on the wall of the container or subjects the composition contained in the container to some other pressure. The viscosity of the composition is preferably high enough that, after passing through the passage(s), it does not fully regain its cohesion by resuming a smooth-surfaced appearance. In other words, the passage may give the composition after it has passed therethrough a surface state with a microrelief.

As stated previously, the cosmetic composition under consideration according to the invention has a gel/gel architecture. It may especially have a semi-solid texture, for example having the texture of a cream, a gel or a paste. The composition may have a viscosity at room temperature of between 5 Pa.s and 20 Pa.s and better still between 7 Pa.s and 19 Pa.s, measured at 25°C with a Rheomat 180 viscometer equipped with a No. 4 spindle, the measurement being taken after 10 minutes of rotation of the spindle, at a shear of 200 $min^{-1}$.

Furthermore, it is important to note that a composition according to the invention is different from an emulsion.

An emulsion generally consists of an oily liquid phase and an aqueous liquid phase. It is a dispersion of droplets of one of the two liquid phases in the other. The size of the droplets forming the dispersed phase of the emulsion is typically about a micrometre (0.1 to 100 μm). Furthermore, an emulsion requires the presence of a surfactant or of an emulsifier to ensure its stability over time.

In contrast, a composition according to the invention consists of a macroscopically homogeneous mixture of two immiscible gelled phases. These two phases both have a gel-type texture. This texture is especially reflected visually by a consistent and/or creamy appearance.

The term *"macroscopically homogeneous mixture"* means a mixture in which each of the gelled phases cannot be individualized by the naked eye. More precisely, in a composition according to the invention, the gelled aqueous phase and the gelled oily phase interpenetrate and thus form a stable, consistent product. This consistency is achieved by mixing interpenetrated macrodomains. These interpenetrated macrodomains are not measurable objects. Thus, by microscope, the composition according to the invention is very different from an emulsion. A composition according to the invention cannot be characterized either as having a *"sense"*, i.e. an O/W or W/O sense, this means that a continuous phsase and a dispersed phase cannot be defined.

Thus, a composition according to the invention has a consistency of gel type. The stability of the composition

is long-lasting without surfactant. Consequently, a composition, especially a cosmetic composition according to the invention, does not require any surfactant or silicone emulsifier to ensure its stability over time.

A composition according to the invention is distinguishable from an emulsion by mean of at least one of the following tests: test using a dyestuff, drop test and dilution test.

Test using a dyestuff

It is known practice from the prior art to observe the intrinsic nature of a mixture of aqueous and oily gels in a gel-type composition, for example, by introducing a dyestuff either into the aqueous gelled phase or into the lipophilic gelled phase, before the formation of the gel-type composition. During visual inspection, in a gel-type composition, the dyestuff appears uniformly dispersed, even if the dye is present solely in the gelled aqueous phase or in the gelled oily phase. Specifically, if two different dyes of different colours are introduced, respectively, into the oily phase and into the aqueous phase, before formation of the gel-type composition, the two colours may be observed as being uniformly dispersed throughout the gel-type composition. This is different from an emulsion in which, if a dye, which is soluble in water or soluble in oil, is introduced, respectively, into the aqueous and oily phases, before forming the emulsion, the colour of the dye present will only be observed in the outer phase (Remington: The Science and Practice of Pharmacy, 19th Edition (1995), Chapter 21, page 282).

Drop test

It is also known practice to distinguish a gel-type composition from an emulsion by performing a "drop test". This test consists in demonstrating the bi-continuous nature of a gel-type composition. Specifically, as mentioned previously, the consistency of a composition is obtained by means of the interpenetration of the aqueous and oily gelled domains. Consequently, the bi-continuous nature of a gel-type composition may be demonstrated by means of a simple test with, respectively, hydrophilic and hydrophobic solvents. This test consists in depositing, firstly, one drop of a hydrophilic solvent on a first sample of the test composition, and, secondly, one drop of a hydrophobic solvent on a second sample of the same test composition, and in analysing the behaviour of the two drops of solvents. In the case of an O/W emulsion, the drop of hydrophilic solvent diffuses into the sample and the drop of hydrophobic solvent remains at the surface of the sample. In the case of a W/O emulsion, the drop of hydrophilic solvent remains at the surface of the sample and the drop of hydrophobic solvent diffuses throughout the sample. Finally, in the case of a gel-type composition (bi-continuous system), the hydrophilic and hydrophobic drops diffuse throughout the sample.

Dilution test

In the case of the present invention, the test that will be preferred for distinguishing a gel-type composition from an emulsion is a dilution test. Specifically, in a gel-type composition, the aqueous and oily gelled domains interpenetrate and form a consistent and stable composition, in which the behaviour in water and in oil is different from the behaviour of an emulsion. Consequently, the behaviour during dilution of a gel-type composition (bi-continuous system) may be compared to that of an emulsion, obviously the behaviour during dilution of a gel/gel-type composition and the one of a emulsion will be different.

More specifically, the dilution test consists in placing 40 g of product and 160 g of dilution solvent (water or oil) in a 500 mL plastic beaker. The dilution is performed with controlled stirring to avoid any emulsification. In particular, this is performed using a planetary mixer: Speed Mixer TM DAC400FVZ. The speed of the mixer is set at 1500 rpm for 4 minutes. Finally, observation of the resulting sample is performed using an optical microscope at a magnification of $\times 100$ ($\times 10 \times 10$). It may be noted that oils such as Parleam® and Xiameter PMX-200 Silicone Fluid 5CS® sold by Dow Corning are suitable as dilution solvent, in the same respect as one of the oils contained in the composition.

In the case of a gel-type composition (bi-continuous system), when it is diluted in oil or in water, a heterogeneous appearance is always observed. When a gel-type composition (bi-continuous system) is diluted in water, pieces of oily gel in suspension are observed, and when a gel-type composition (bi-continuous system) is diluted in oil, pieces of aqueous gel in suspension are observed.

In contrast, during dilution, emulsions have a different behaviour. When an O/W emulsion is diluted in an aqueous solvent, it gradually reduces without having a heterogeneous and lumpy appearance. This same O/W emulsion, on dilution with oil, has a heterogeneous appearance (pieces of O/W emulsion suspended in the oil). When a W/O emulsion is diluted with an aqueous solvent, it has a heterogeneous appearance (pieces of W/O emulsion suspended in the water). This same W/O emulsion, when diluted in oil, gradually reduces without having a heterogeneous and lumpy appearance.

According to the present invention, the aqueous gelled phase and the oily gelled phase forming a composition according to the invention are present therein in a weight ratio ranging from 95/5 to 5/95. More preferentially, the aqueous

phase and the oily phase are present in a weight ratio ranging from 30/70 to 80/20.

The ratio between the two gelled phases is adjusted according to the desired cosmetic properties.

According to one embodiment, the aqueous gelled phase/oily gelled phase weight ratio is greater than 1, especially ranging from 60/40 to 90/10, preferably ranging from 60/40 to 80/20, preferentially from 60/40 to 70/30 and even more preferentially the aqueous gelled phase/oily gelled phase weight ratio is 60/40 or 70/30.

Such gelled aqueous phase/gelled oily phase weight ratios are particularly advantageous in the case of a makeup composition, in particular for the face.

These preferred ratios are particularly advantageous for obtaining fresh and light compositions.

Advantageously, a composition according to the invention may thus be in the form of a creamy gel with a minimum stress below which it does not flow unless it has been subjected to an external mechanical stress.

As emerges from the text hereinbelow, a composition according to the invention may have a minimum threshold stress of 1.5 Pa and in particular greater than 10 Pa.

The composition according to the invention may have a maximum threshold stress of 10 000 Pa preferably of 5 000 Pa.

It also advantageously has a stiffness modulus G* at least equal to 400 Pa and preferably greater than 1000 Pa. The composition according to the invention may have a stiffness modulus G* preferably lower than 50 000Pa, more preferably lower than 5 000 Pa.

The ratio of the hydrophilic phase viscosity / lipophilic phase viscosity (measured at 25°C and 100 $s^{-1}$) preferably ranges from 0.2 and 3.

According to an advantageous embodiment variant, the gelled phases under consideration to form a composition according to the invention have, respectively, a threshold stress of greater than 1.5 Pa and preferably greater than 10 Pa.

The gelled phases under consideration to form a composition according to the invention may have a threshold stress lower than 10 000 Pa preferably lower than 5 000 Pa.

Characterization of the threshold stresses is performed by oscillating rheology measurements. Methodology is proposed in the illustrative chapter of the present text.

In general, the corresponding measurements are taken at 25°C using a Haake RS600 imposed-stress rheometer equipped with a plate-plate measuring body (60 mm diameter) fitted with an anti-evaporation device (bell jar). For each measurement, the sample is placed delicately in position and the measurements start 5 minutes after placing the sample in the jaws (2 mm). The test composition is then subjected to a stress ramp from $10^{-2}$ to $10^3$ Pa at a set frequency of 1 Hz.

A composition according to the invention may also have a certain elasticity. This elasticity may be characterized by a stiffness modulus G* which, under this minimum stress threshold, may be at least equal to 400 Pa and preferably greater than 1000 Pa. The value G* of a composition may be obtained by subjecting the composition under consideration to a stress ramp from $10^{-2}$ to $10^3$ Pa at a set frequency of 1 Hz.

## HYDROPHILIC GELLING AGENT

For the purposes of the present invention, the term *"hydrophilic gelling agent"* means a compound that is capable of gelling the aqueous phase of the compositions according to the invention.

The gelling agent is hydrophilic and is thus present in the aqueous phase of the composition.

The gelling agent may be water-soluble or water-dispersible.

As stated above, the aqueous phase of a composition according to the invention is gelled with at least one hydrophilic gelling agent.

The hydrophilic gelling agent may be chosen from synthetic polymeric gelling agents, polymeric gelling agents that are natural or of natural origin, mixed silicates and fumed silicas, and mixtures thereof.

Preferably, the hydrophilic gelling agent may be chosen from synthetic polymeric gelling agents.

At least one hydrophilic gelling agent is chosen from 2-acrylamido-2-methylpropanesulfonic acid polymers and copolymers.

### I. Polymeric gelling agents that are natural or of natural origin

The polymeric hydrophilic gelling agents that are suitable for use in the invention may be natural or of natural origin.

For the purposes of the invention, the term *"of natural origin"* is intended to denote polymeric gelling agents obtained by modification of natural polymeric gelling agents.

These gelling agents may be particulate or non-particulate.

More specifically, these gelling agents fall within the category of polysaccharides.

In general, polysaccharides may be divided into several categories.

Thus, polysaccharides that are suitable for use in the invention may be homopolysaccharides such as fructans,

glucans, galactans and mannans or heteropolysaccharides such as hemicellulose.

Similarly, they may be linear polysaccharides such as pullulan or branched polysaccharides such as gum arabic and amylopectin, or mixed polysaccharides such as starch.

More particularly, the polysaccharides that are suitable for use in the invention may be distinguished according to whether or not they are starchy.

*I.A. Starchy polysaccharides*

As representatives of this category, mention may be made most particularly of native starches, modified starches and particulate starches.

Native starches

The starches that may be used in the present invention are more particularly macromolecules in the form of polymers consisting of elementary moieties which are anhydroglucose units (dextrose), linked via $\alpha(1,4)$ bonds of chemical formula $(C_6H_{10}O_5)_n$. The number of these moieties and their assembly make it possible to distinguish amylose, a molecule formed from about 600 to 1000 linearly linked glucose molecules, and amylopectin, a polymer branched approximately every 25 glucose residues ($\alpha(1,6)$ bond). The total chain may include between 10 000 and 100 000 glucose residues.

Starch is described in particular in Kirk-Othmer's Encyclopaedia of Chemical Technology, 3rd edition, volume 21, pages 492-507, Wiley Interscience, 1983.

The relative proportions of amylose and of amylopectin, and their degree of polymerization, vary as a function of the botanical origin of the starches. On average, a sample of native starch consists of about 25% amylose and 75% amylopectin.

Occasionally, phytoglycogen is present (between 0% and 20% of the starch), which is an analogue of amylopectin but branched every 10 to 15 glucose residues.

Starch may be in the form of semi-crystalline granules: amylopectin is organized in leaflets, amylose forms a less well organized amorphous zone between the various leaflets.

Amylose is organized in a straight helix with six glucoses per turn. It dissociates into assimilable glucose under the action of enzymes, amylases, all the more easily when it is in amylopectin form. Specifically, the helical formation does not promote the accessibility of starch to the enzymes.

Starches are generally in the form of a white powder, which is insoluble in cold water, whose elemental particle size ranges from 3 to 100 microns.

By treating it with hot water, starch paste is obtained. It is exploited in industry for its thickening and gelling properties.

The botanical origin of the starch molecules used in the present invention may be cereals or tubers. Thus, the starches are chosen, for example, from corn starch, rice starch, cassava starch, tapioca starch, barley starch, potato starch, wheat starch, sorghum starch and pea starch.

The native starches are represented, for example, by the products sold under the names C*AmilogelTM, Cargill GelTM, C* GelTM, Cargill GumTM, DryGelTM and C*Pharm GelTM by the company Cargill, under the name Corn Starch by the company Roquette, and under the name Tapioca Pure by the company National Starch.

Modified starches

The modified starches used in the composition of the invention may be modified via one or more of the following reactions: pregelatinization, degradation (acid hydrolysis, oxidation, dextrinization), substitution (esterification, etherification), crosslinking (esterification), bleaching.

More particularly, these reactions may be performed in the following manner:

- pregelatinization by splitting the starch granules (for example drying and cooking in a drying drum);
- acid hydrolysis giving rise to very rapid retrogradation on cooling;
- oxidation with strong oxidizing agents (alkaline medium, in the presence of sodium hypochlorite NaOCl for example) leading to depolymerization of the starch molecule and to the introduction of carboxyl groups into the starch molecule (mainly oxidation of the hydroxyl group at $C_6$);
- dextrinization in acid medium at high temperature (hydrolysis followed by repolymerization);
- crosslinking with functional agents capable of reacting with the hydroxyl groups of the starch molecules, which will thus bond together (for example with glyceryl and/or phosphate groups);
- esterification in alkaline medium for the grafting of functional groups, especially $C_1$-$C_6$ acyl (acetyl), $C_1$-$C_6$ hydroxy-

alkyl (hydroxyethyl or hydroxypropyl), carboxymethyl or octenylsuccinic.

Monostarch phosphates (of the type St-O-PO-$(OX)_2$), distarch phosphates (of the type St-O-PO-(OX)-O-St) or even tristarch phosphates (of the type St-O-PO-$(O\text{-}St)_2$) or mixtures thereof may especially be obtained by crosslinking with phosphorus compounds.

X in particular denotes alkali metals (for example sodium or potassium), alkaline-earth metals (for example calcium or magnesium), ammonium salts, amine salts, for instance those of monoethanolamine, diethanolamine, triethanolamine, 3-amino-1,2-propanediol, or ammonium salts derived from basic amino acids such as lysine, arginine, sarcosine, ornithine or citrulline.

The phosphorus compounds may be, for example, sodium tripolyphosphate, sodium orthophosphate, phosphorus oxychloride or sodium trimetaphosphate.

According to the invention, it is also possible to use amphoteric starches, these amphoteric starches containing one or more anionic groups and one or more cationic groups. The anionic and cationic groups may be linked to the same reactive site of the starch molecule or to different reactive sites; they are preferably linked to the same reactive site. The anionic groups may be of carboxylic, phosphate or sulfate type, preferably carboxylic. The cationic groups may be of primary, secondary, tertiary or quaternary amine type.

The amphoteric starches are especially chosen from the compounds having the following formulae:

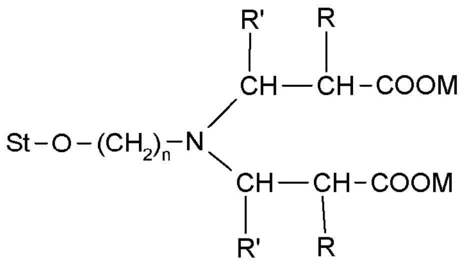

(I)

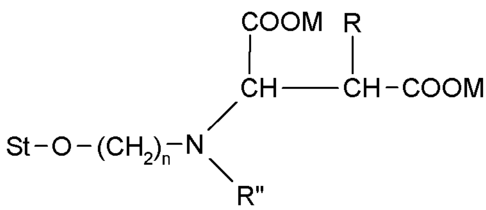

(II)

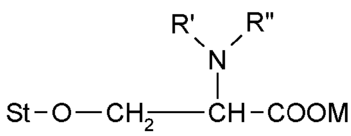

(III)

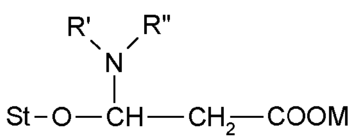

(IV)

in which:

- St-O represents a starch molecule;
- R, which may be identical or different, represents a hydrogen atom or a methyl radical;
- R', which may be identical or different, represents a hydrogen atom, a methyl radical or a -COOH group;
- n is an integer equal to 2 or 3;
- M, which may be identical or different, denotes a hydrogen atom, an alkali metal or alkaline-earth metal such as Na, K, Li or $NH_4$, a quaternary ammonium or an organic amine,
- R" represents a hydrogen atom or an alkyl radical containing from 1 to 18 carbon atoms.

These compounds are especially described in patents US 5 455 340 and US 4 017 460.

The starch molecules may be derived from any plant source of starch, especially such as corn, potato, oat, rice, tapioca, sorghum, barley or wheat. It is also possible to use the starch hydrolysates mentioned above.

The modified starches are represented, for example, by the products sold under the names C*Tex-Instant (pregelatinized adipate), C*StabiTex-Instant (pregelatinized phosphate), C*PolarTex-Instant (pregelatinized hydroxypropyl), C*Set (acid hydrolysis, oxidation), C*size (oxidation), C*BatterCrisp (oxidation), C*DrySet (dextrinization), C*TexTM (acetyl distarch adipate), C*PolarTexTM (hydroxypropyl distarch phosphate), C* StabiTexTM (distarch phosphate, acetyl distarch phosphate) by the company Cargill, by distarch phosphates or compounds rich in distarch phosphate such as the product sold under the references Prejel VA-70-T AGGL (gelatinized hydroxypropyl cassava distarch phosphate) or Prejel TK1 (gelatinized cassava distarch phosphate) or Prejel 200 (gelatinized acetyl cassava distarch phosphate) by the company Avebe or Structure Zea from National Starch (gelatinized corn distarch phosphate).

As examples of oxidized starches, use will be made especially of those sold under the name C*size from the company Cargill.

The native or modified starches described above may be advantageously used in a proportion of from 0.1% to 8% by weight of solids and preferably at about 1% by weight, relative to the total weight of the aqueous phase.

Particulate starches

Particulate starches that may be mentioned in particular include:

- starches grafted with an acrylic polymer (homopolymer or copolymer) and especially with sodium polyacrylate, for instance those sold under the names Sanfresh ST-100MC by the company Sanyo Chemical Industries or Makimousse 25, Makimousse 12 by the company Daito Kasei (INCI name: Sodium polyacrylate starch),
- hydrolysed starches grafted with an acrylic polymer (homopolymer or copolymer) and especially acryloacrylamide/sodium acrylate copolymer, for instance those sold under the names Water Lock A-240, A-180, B-204, D-223, A-100, C-200 and D-223 by the company Grain Processing (INCI name: Starch/acrylamide/sodium acrylate copolymer);
- polymers based on starch, gum and cellulose derivative, such as the product containing starch, and sodium carboxymethylcellulose, for instance the product sold under the name Lysorb 220 by the company Lysac.

Mention may be made most particularly of $C_1$-$C_4$ carboxyalkyl starches, also referred to hereinbelow as *carboxyalkyl starch*. These compounds are obtained by grafting carboxyalkyl groups onto one or more alcohol functions of starch, especially by reaction of starch and of sodium monochloroacetate in alkaline medium.

The carboxyalkyl groups are generally attached via an ether function, more particularly to carbon 1. The degree of substitution with carboxyalkyl units of the $C_1$-$C_4$ carboxyalkyl starch preferably ranges from 0.1 to 1 and more particularly from 0.15 to 0.5. The degree of substitution is defined according to the present invention as being the mean number of hydroxyl groups substituted with an ester or ether group per monosaccharide unit of the polysaccharide.

The carboxyalkyl starches are advantageously used in the form of salts and especially of salts of alkali metals or alkaline-earth metals such as Na, K, Li, NH4, or salts of a quaternary ammonium or of an organic amine such as monoethanolamine, diethanolamine or triethanolamine. The $C_1$-$C_4$ carboxyalkyl starches are advantageously, in the context of the present invention, carboxymethyl starches. The carboxymethyl starches preferably comprise units having the following formula:

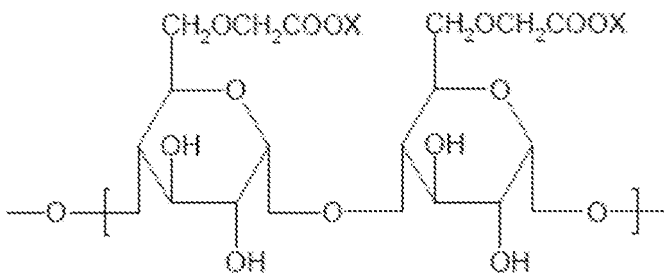

in which X, optionally covalently bonded to the carboxylic unit, denotes a hydrogen atom, an alkali metal or alkaline-earth metal such as Na, K, Li, $NH_4$, a quaternary ammonium or an organic amine, for instance monoethanolamine, diethanolamine or triethanolamine.

Preferably, X denotes a cation $Na^+$. The carboxyalkyl starches that may be used according to the present invention are preferably non-pregelatinized carboxyalkyl starches. The carboxyalkyl starches that may be used according to the present invention are preferably partially or totally crosslinked carboxyalkyl starches.

In general, a crosslinked carboxyalkyl starch has, in contrast with a non-crosslinked carboxyalkyl starch, an increased, controllable viscosity and increased stability. The crosslinking thus makes it possible to reduce the syneresis phenomena and to increase the resistance of the gel to shear effects.

The carboxyalkyl starches under consideration according to the invention are more particularly potato carboxyalkyl starches. Thus, the carboxyalkyl starches that may be used according to the present invention are preferably sodium salts of carboxyalkyl starches, in particular a sodium salt of potato carboxymethyl starch, sold especially under the name Primojel® by the company DMV International or Glycolys® and Glycolys® LV by the company Roquette.

According to a particular mode, use will be made of the potato carboxymethyl starches sold especially under the name Glycolys® by the company Roquette. As stated previously, the $C_1$-$C_4$ carboxyalkyl starch particles are present in the compositions according to the invention in a swollen and non-split form. This swelling may be characterized by a swelling power Q which may advantageously be between 10 and 30 ml/g and preferably between 15 and 25 ml (volume of absorbed liquid)/g of dry particulate material.

Thus, the size of the swollen carboxyalkyl starch particles used according to the present invention generally ranges from 25 to 300 μm. For example, the gel Primojel® containing 10% by weight of potato carboxyalkyl starch and sodium salt in water contains more than 80% of swollen particles of this starch with a diameter of greater than 50 microns and more particularly greater than 100 microns.

According to a preferred embodiment variant of the invention, these particles are used for the preparation of the compositions according to the invention, in this swollen particulate state. To do so, these particles are advantageously used in the form of an aqueous gel either prepared beforehand or already commercially available. The gels under consideration according to the invention are advantageously translucent.

For example, a carboxymethyl starch gel such as Primojel® which is at a concentration of 10% by weight may be adjusted to the required concentration before being used for preparing the expected composition.

Such a particulate starch may be used in a proportion of from 0.1% to 5% by weight of solids relative to the total weight of the aqueous phase, preferably between 0.5% and 2.5% by weight and in particular in a proportion of about 1.5% by weight, relative to the total weight of the aqueous phase.

According to one embodiment variant, the hydrophilic gelling agent is non-starchy.

### *I.B. Non-starchy polysaccharides*

In general, the non-starchy polysaccharides may be chosen from polysaccharides produced by microorganisms; polysaccharides isolated from algae, and higher plant polysaccharides, such as homogeneous polysaccharides, in particular celluloses and derivatives thereof or fructosans, heterogeneous polysaccharides such as gum arabics, galactomannans, glucomannans and pectins, and derivatives thereof; and mixtures thereof.

In particular, the polysaccharides may be chosen from fructans, gellans, glucans, amylose, amylopectin, glycogen, pullulan, dextrans, celluloses and derivatives thereof, in particular methylcelluloses, hydroxyalkylcelluloses, ethylhydroxyethylcelluloses and carboxymethylcelluloses, mannans, xylans, lignins, arabans, galactans, galacturonans, alginate-based compounds, chitin, chitosans, glucuronoxylans, arabinoxylans, xyloglucans, glucomannans, pectic acids and pectins, arabinogalactans, carrageenans, agars, glycosaminoglucans, gum arabics, tragacanth gums, ghatti gums, karaya gums, locust bean gums, galactomannans such as guar gums and nonionic derivatives thereof, in particular hydroxypropyl guar, and ionic derivatives thereof, biopolysaccharide gums of microbial origin, in particular scleroglucan or xanthan gums, mucopolysaccharides, and in particular chondroitin sulfates, and mixtures thereof.

These polysaccharides may be chemically modified, especially with urea or urethane groups or by hydrolysis, oxidation, esterification, etherification, sulfatation, phosphatation, amination, amidation or alkylation reaction, or by several of these modifications.

The derivatives obtained may be anionic, cationic, amphoteric or nonionic.

Advantageously, the polysaccharides may be chosen from carrageenans, in particular kappa carrageenan, gellan gum, agar-agar, xanthan gum, alginate-based compounds, in particular sodium alginate, scleroglucan gum, guar gum, inulin and pullulan, and mixtures thereof.

In general, the compounds of this type that may be used in the present invention are chosen from those described especially in Kirk-Othmer's Encyclopaedia of Chemical Technology, Third Edition, 1982, volume 3, pp. 896-900, and volume 15, pp. 439-458, in Polymers in Nature by E.A. MacGregor and C.T. Greenwood, published by John Wiley & Sons, Chapter 6, pp. 240-328, 1980, in the book by Robert L. Davidson entitled Handbook of Water-Soluble Gums and Resins published by McGraw Hill Book Company (1980) and in Industrial Gums - Polysaccharides and their Derivatives, edited by Roy L. Whistler, Second Edition, published by Academic Press Inc.

Such a gelling agent may be used in a proportion of from 0.1% to 8% by weight of solids relative to the total weight of the aqueous phase, especially from 0.1% to 6% by weight, preferably between 0.5% and 2.5% by weight and in particular in a proportion of about 1%, or alternatively in a proportion of about 1.5% by weight, relative to the total weight of the aqueous phase.

More precisely, these polysaccharides that are suitable for use in the invention may be distinguished according to whether they are derived from microorganisms, from algae or from higher plants, and are detailed below.

Polysaccharides produced by microorganisms

*Xanthan*

Xanthan is a heteropolysaccharide produced at the industrial scale by the aerobic fermentation of the bacterium *Xanthomonas campestris*. Its structure consists of a main chain of β(1,4)-linked β-D-glucoses, similar to cellulose. One glucose molecule in two bears a trisaccharide side chain composed of an α-D-mannose, a β-D-glucuronic acid and a terminal β-D-mannose. The internal mannose residue is generally acetylated on carbon 6. About 30% of the terminal mannose residues bear a pyruvate group linked in chelated form between carbons 4 and 6. The charged pyruvic acids and glucuronic acids are ionizable, and are thus responsible for the anionic nature of xanthan (negative charge down to a pH equal to 1). The content of pyruvate and acetate residues varies according to the bacterial strain, the fermentation process, the conditions after fermentation and the purification steps. These groups may be neutralized in commercial products with $Na^+$, $K^+$ or $Ca^{2+}$ ions (Satia company, 1986). The neutralized form may be converted into the acid form by ion exchange or by dialysis of an acidic solution.

Xanthan gums have a molecular weight of between 1 000 000 and 50 000 000 and a viscosity of between 0.6 and 1.65 Pa.s for an aqueous composition containing 1% of xanthan gum (measured at 25°C on a Brookfield viscometer of LVT type at 60 rpm).

Xanthan gums are represented, for example, by the products sold under the names Rhodicare by the company Rhodia Chimie, under the name Satiaxane™ by the company Cargill Texturizing Solutions (for the food, cosmetic and pharmaceutical industries), under the name Novaxan™ by the company ADM, and under the names Kelzan® and Keltrol® by the company CP-Kelco.

*Pullulan*

Pullulan is a polysaccharide consisting of maltotriose units, known under the name α(1,4)-α(1,6)-glucan. Three glucose units in maltotriose are connected via an α(1,4) glycoside bond, whereas the consecutive maltotriose units are connected to each other via an α(1,6) glycoside bond.

Pullulan is produced, for example, under the reference Pullulan PF 20 by the group Hayashibara in Japan.

*Dextran and dextran sulfate*

Dextran is a neutral polysaccharide not bearing any charged groups, which is biologically inert, prepared by fermentation of beet sugar containing solely hydroxyl groups.

It is possible to obtain dextran fractions of different molecular weights from native dextran by hydrolysis and purification. Dextran may in particular be in the form of dextran sulfate.

Dextran is represented, for example, by the products sold under the name Dextran or Dextran T by the company Pharmacosmos, or under the name Dextran 40 Powder or Dextran 70 Powder by the company Meito Sangyo Co. Dextran sulfate is sold by the company PK Chemical A/S under the name Dextran sulfate.

*Succinoglycan*

Succinoglycan is an extracellular polymer of high molecular weight produced by bacterial fermentation, consisting of octasaccharide repeating units (repetition of 8 sugars). Succinoglycans are sold, for example, under the name Rheozan by the company Rhodia.

*Scleroglucan*

Scleroglucan is a nonionic branched homopolysaccharide consisting of β-D-glucan units. The molecules consist of a linear main chain formed from D-glucose units linked via β(1,3) bonds and of which one in three is linked to a side D-glucose unit via a β(1,6) bond.

A more complete description of scleroglucans and of their preparation may be found in patent US 3 301 848.

Scleroglucan is sold, for example, under the name Amigel by the company Alban Müller, or under the name Actigum™ CS by the company Cargill.

*Gellan gum*

Gellan gum is an anionic linear heteropolyoside based on oligoside units composed of 4 saccharides (tetraoside). D-Glucose, L-rhamnose and D-glucuronic acid in 2:1:1 proportions are present in gellan gum in the form of monomer elements.
It is sold, for example, under the name Kelcogel CG LA by the company CP Kelco.

Polysaccharides isolated from algae

*Galactans*

The polysaccharide according to the invention may be a galactan chosen especially from agar and carrageenans.
Carrageenans are anionic polysaccharides constituting the cell walls of various red algae (Rhodophyceae) belonging to the Gigartinacae, Hypneaceae, Furcellariaceae and Polyideaceae families. They are generally obtained by hot aqueous extraction from natural strains of said algae. These linear polymers, formed by disaccharide units, are composed of two D-galactopyranose units linked alternately by $\alpha$(1,3) and $\beta$(1,4) bonds. They are highly sulfated polysaccharides (20-50%) and the $\alpha$-D-galactopyranosyl residues may be in 3,6-anhydro form. Depending on the number and position of sulfate-ester groups on the repeating disaccharide of the molecule, several types of carrageenans are distinguished, namely: kappa-carrageenans, which bear one sulfate-ester group, iota-carrageenans, which bear two sulfate-ester groups, and lambda-carrageenans, which bear three sulfate-ester groups.
Carrageenans are composed essentially of potassium, sodium, magnesium, triethanolamine and/or calcium salts of polysaccharide sulfate esters.
Carrageenans are sold especially by the company SEPPIC under the name Solagum®, by the company Gelymar under the names Carragel®, Carralact® and Carrasol®, by the company Cargill, under the names Satiagel™ and Satiagum™, and by the company CP-Kelco under the names Genulacta®, Genugel® and Genuvisco®.
Galactans of agar type are galactose polysaccharides contained in the cell wall of some of these species of red algae (rhodophyceae). They are formed from a polymer group whose base backbone is a $\beta$(1,3) D-galactopyranose and $\alpha$(1,4) L 3-6 anhydrogalactose chain, these units repeating regularly and alternately. The differences within the agar family are due to the presence or absence of solvated methyl or carboxyethyl groups. These hybrid structures are generally present in variable percentage, depending on the species of algae and the harvest season.
Agar-agar is a mixture of polysaccharides (agarose and agaropectin) of high molecular mass, between 40 000 and 300 000 $g.mol^{-1}$. It is obtained by manufacturing algal extraction liquors, generally by autoclaving, and by treating these liquors which comprise about 2% of agar-agar, so as to extract the latter.
Agar is produced, for example, by the group B&V Agar Producers under the names Gold Agar, Agarite and Grand Agar by the company Hispanagar, and under the names Agar-Agar, QSA (Quick Soluble Agar), and Puragar by the company Setexam.

*Furcellaran*

Furcellaran is obtained commercially from red algae *Furcellaria fasztigiata.* Furcellaran is produced, for example, by the company Est-Agar.

*Alginate-based compound*

For the purposes of the invention, the term *"alginate-based compound'* means alginic acid, alginic acid derivatives and salts of alginic acid (alginates) or of said derivatives.
Preferably, the alginate-based compound is water-soluble.
Alginic acid, a natural substance resulting from brown algae or certain bacteria, is a polyuronic acid composed of 2 uronic acids linked by 1,4-glycosidic bonds: $\beta$-D-mannuronic (M) acid and $\alpha$-L-glucuronic (G) acid.
Alginic acid is capable of forming water-soluble salts (alginates) with alkali metals such as sodium, potassium or lithium, substituted cations of lower amines and of substituted ammonium such as methylamine, ethanolamine, diethanolamine or triethanolamine. These alginates are water-soluble in aqueous medium at a pH equal to 4, but dissociate into alginic acid at a pH below 4.
This (these) alginate-based compound(s) is/are capable of crosslinking in the presence of at least one crosslinking agent, by formation of ionic bonds between said alginate-based compound(s) and said crosslinking agent(s). The formation of multiple crosslinking between several molecules of said alginate-based compound(s) leads to the formation of a water-insoluble gel.

Use is preferably made of alginate-based compounds with a weight-average molecular mass ranging from 10 000 to 1 000 000, preferably from 15 000 to 500 000 and better still from 20 000 to 250 000.
According to a preferred embodiment, the alginate-based compound is alginic acid and/or a salt thereof.
Advantageously, the alginate-based compound is an alginate salt, and preferably sodium alginate.
The alginate-based compound may be chemically modified, especially with urea or urethane groups or by hydrolysis, oxidation, esterification, etherification, sulfatation, phosphatation, amination, amidation or alkylation reaction, or by several of these modifications.
The derivatives obtained may be anionic, cationic, amphoteric or nonionic.
The alginate-based compounds that are suitable for use in the invention may be represented, for example, by the products sold under the names Kelcosol, Satialgine™, Cecalgum™ or Algogel™ by the company Cargill Products, under the name Protanal™ by the company FMC Biopolymer, under the name Grindsted® Alginate by the company Danisco, under the name Kimica Algin by the company Kimica, and under the names Manucol® and Manugel® by the company ISP.

Polysaccharides of higher plants

This category of polysaccharides may be divided into homogeneous polysaccharides (only one saccharide species) and heterogeneous polysaccharides composed of several types of saccharides.

a) Homogeneous polysaccharides and derivatives thereof

The polysaccharide according to the invention may be chosen from celluloses and derivatives or fructosans.

*Cellulose and derivatives*

The polysaccharide according to the invention may also be a cellulose or a derivative thereof, especially cellulose ethers or esters (e.g.: methylcellulose, carboxymethylcellulose, hydroxymethylcellulose, hydroxyethylcellulose, hydroxypropylcellulose, hydroxymethylpropylcellulose, cellulose acetate, cellulose nitrate, nitrocellulose).
The invention may also contain a cellulose-based associative polymer. According to the invention, the term *"cellulose-based compound'* means any polysaccharide compound bearing in its structure linear sequences of anhydroglucopyranose residues (AGU) linked together via β(1,4) bonds. The repeating unit is the cellobiose dimer. The AGUs are in chair conformation and bear 3 hydroxyl functions: 2 secondary alcohols (in position 2 and 3) and a primary alcohol (in position 6). The polymers thus formed combine together via intermolecular bonds of hydrogen bond type, thus giving the cellulose a fibrillar structure (about 1500 molecules per fibre).
The degree of polymerization differs enormously depending on the origin of the cellulose; its value may range from a few hundred to several tens of thousands.
Cellulose has the following chemical structure:

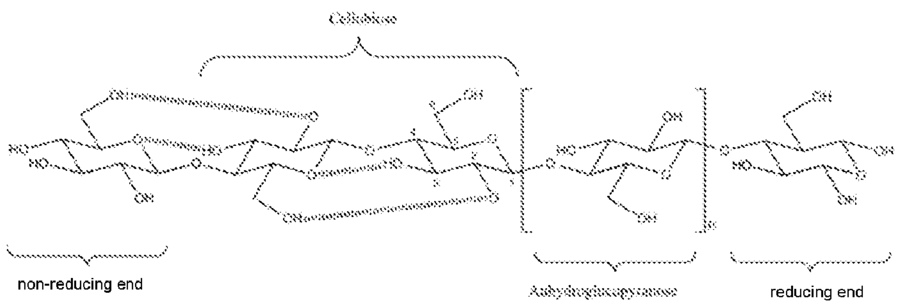


The hydroxyl groups of cellulose may react partially or totally with various chemical reagents to give cellulose derivatives having intrinsic properties. The cellulose derivatives may be anionic, cationic, amphoteric or nonionic. Among these derivatives, cellulose ethers, cellulose esters and cellulose ester ethers are distinguished.
Among the nonionic cellulose ethers, mention may be made of alkylcelluloses such as methylcelluloses and ethylcelluloses; hydroxyalkylcelluloses such as hydroxymethylcelluloses, hydroxyethylcelluloses and hydroxypropylcelluloses; and mixed hydroxyalkylalkylcelluloses such as hydroxypropylmethylcelluloses, hydroxy-ethylmethylcelluloses, hydroxyethylethylcelluloses and hydroxybutylmethylcelluloses.
Among the anionic cellulose ethers, mention may be made of carboxyalkylcelluloses and salts thereof. By way of example, mention may be made of carboxymethylcelluloses, carboxymethylmethylcelluloses and carboxymethylhydroxy-ethylcelluloses and sodium salts thereof.

Among the cationic cellulose ethers, mention may be made of crosslinked or non-crosslinked, quaternized hydroxyethylcelluloses.

The quaternizing agent may in particular be glycidyltrimethylammonium chloride or a fatty amine such as laurylamine or stearylamine. Another cationic cellulose ether that may be mentioned is hydroxyethylcellulosehydroxypropyltrimethylammonium.

The quaternized cellulose derivatives are, in particular:

- quaternized celluloses modified with groups comprising at least one fatty chain, such as alkyl, arylalkyl or alkylaryl groups comprising at least 8 carbon atoms, or mixtures thereof,
- quaternized hydroxyethylcelluloses modified with groups comprising at least one fatty chain, such as alkyl, arylalkyl or alkylaryl groups comprising at least 8 carbon atoms, or mixtures thereof.

The alkyl radicals borne by the above quaternized celluloses or hydroxyethylcelluloses preferably contain from 8 to 30 carbon atoms. The aryl radicals preferably denote phenyl, benzyl, naphthyl or anthryl groups.

Examples of quaternized alkylhydroxyethylcelluloses containing $C_8$-$C_{30}$ fatty chains that may be indicated include the products Quatrisoft LM 200, Quatrisoft LM-X 529-18-A, Quatrisoft LM-X 529-18B ($C_{12}$ alkyl) and Quatrisoft LM-X 529-8 ($C_{18}$ alkyl) sold by the company Amerchol and the products Crodacel QM, Crodacel QL ($C_{12}$ alkyl) and Crodacel QS ($C_{18}$ alkyl) sold by the company Croda.

Among the cellulose derivatives, mention may also be made of:

- celluloses modified with groups comprising at least one fatty chain, for instance hydroxyethylcelluloses modified with groups comprising at least one fatty chain, such as alkyl groups, especially of $C_8$-$C_{22}$, arylalkyl and alkylaryl groups, such as Natrosol Plus Grade 330 CS ($C_{16}$ alkyls) sold by the company Aqualon, and
- celluloses modified with polyalkylene glycol alkylphenyl ether groups, such as the product Amercell Polymer HM-1500 (nonylphenyl polyethylene glycol (15) ether) sold by the company Amerchol.

Among the cellulose esters are mineral esters of cellulose (cellulose nitrates, sulfates, phosphates, etc.), organic cellulose esters (cellulose monoacetates, triacetates, amidopropionates, acetatebutyrates, acetatepropionates and acetatetrimellitates, etc.), and mixed organic/mineral esters of cellulose, such as cellulose acetatebutyrate sulfates and cellulose acetatepropionate sulfates. Among the cellulose ester ethers, mention may be made of hydroxypropylmethylcellulose phthalates and ethylcellulose sulfates.

The cellulose-based compounds of the invention may be chosen from unsubstituted celluloses and substituted celluloses.

The celluloses and derivatives are represented, for example, by the products sold under the names Avicel® (microcrystalline cellulose, MCC) by the company FMC Biopolymers, under the name Cekol (carboxymethylcellulose) by the company Noviant (CP-Kelco), under the name Akucell AF (sodium carboxymethylcellulose) by the company Akzo Nobel, under the name Methocel™ (cellulose ethers) and Ethocel™ (ethylcellulose) by the company Dow, and under the names Aqualon® (carboxymethylcellulose and sodium carboxymethylcellulose), Benecel® (methylcellulose), Blanose™ (carboxymethylcellulose), Culminal® (methylcellulose, hydroxypropylmethylcellulose), Klucel® (hydroxypropylcellulose), Polysurf® (cetylhydroxyethylcellulose) and Natrosol® CS (hydroxyethylcellulose) by the company Hercules Aqualon.

*Fructosans*

The polysaccharide according to the invention may especially be a fructosan chosen from inulin and derivatives thereof (especially dicarboxy and carboxymethyl inulins).

Fructans or fructosans are oligosaccharides or polysaccharides comprising a sequence of anhydrofructose units optionally combined with several saccharide residues other than fructose. Fructans may be linear or branched. Fructans may be products obtained directly from a plant or microbial source or alternatively products whose chain length has been modified (increased or decreased) by fractionation, synthesis or hydrolysis, in particular enzymatic. Fructans generally have a degree of polymerization from 2 to about 1000 and preferably from 2 to about 60.

Three groups of fructans are distinguished. The first group corresponds to products whose fructose units are for the most part linked via $\beta(2,1)$ bonds. These are essentially linear fructans such as inulins.

The second group also corresponds to linear fructoses, but the fructose units are essentially linked via $\beta(2,6)$ bonds. These products are levans.

The third group corresponds to mixed fructans, i.e. containing $\beta(2,6)$ and $\beta(2,1)$ sequences. These are essentially branched fructans, such as graminans.

The preferred fructans in the compositions according to the invention are inulins. Inulin may be obtained, for example, from chicory, dahlia or Jerusalem artichoke, preferably from chicory.

In particular, the polysaccharide, especially the inulin, has a degree of polymerization from 2 to about 1000 and preferably from 2 to about 60, and a degree of substitution of less than 2 on the basis of one fructose unit.

The inulin used for this invention is represented, for example, by the products sold under the name Beneo™ inulin by the company Orafti, and under the name Frutafit® by the company Sensus.

b) Heterogeneous polysaccharides and derivatives thereof

The polysaccharides that may be used according to the invention may be gums, for instance cassia gum, karaya gum, konjac gum, gum tragacanth, tara gum, acacia gum or gum arabic.

*Gum arabic*

Gum arabic is a highly branched acidic polysaccharide which is in the form of mixtures of potassium, magnesium and calcium salts. The monomer elements of the free acid (arabic acid) are D-galactose, L-arabinose, L-rhamnose and D-glucuronic acid.

*Galactomannans (guar, locust bean, fenugreek, tara gum) and derivatives (guar phosphate, hydroxypropyl guar, etc.)*

Galactomannans are nonionic polyosides extracted from the endosperm of leguminous seeds, of which they constitute the storage carbohydrate.

Galactomannans are macromolecules consisting of a main chain of β(1,4)-linked D-mannopyranose units, bearing side branches consisting of a single D-galactopyranose unit α(1,6)-linked to the main chain. The various galactomannans differ, firstly, by the proportion of α-D-galactopyranose units present in the polymer, and secondly, by significant differences in terms of distribution of galactose units along the mannose chain.

The mannose/galactose (M/G) ratio is about 2 for guar gum, 3 for tara gum and 4 for locust bean gum.

Galactomannans have the following chemical structure:

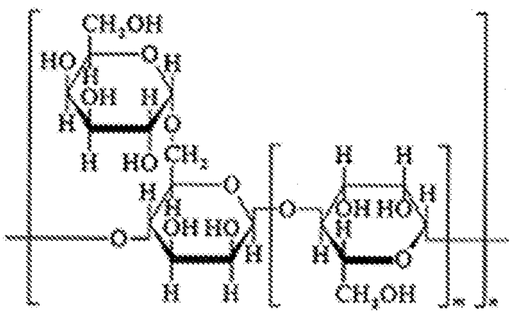

m = 3: Locust bean gum
m = 1: Guar gum
m = 2: Tara gum

*Guar*

Guar gum is characterized by a mannose/galactose ratio of the order of 2/1. The galactose group is regularly distributed along the mannose chain.

The guar gums that may be used according to the invention may be nonionic, cationic or anionic. According to the invention, use may be made of chemically modified or unmodified nonionic guar gums.

The unmodified nonionic guar gums are, for example, the products sold under the names Vidogum GH, Vidogum G and Vidocrem by the company Unipektin and under the name Jaguar by the company Rhodia, under the name Meypro® Guar by the company Danisco, under the name Viscogum™ by the company Cargill, and under the name Supercol® guar gum by the company Aqualon.

The hydrolysed nonionic guar gums that may be used according to the invention are represented, for example, by the products sold under the name Meyprodor® by the company Danisco.

The modified nonionic guar gums that may be used according to the invention are preferably modified with $C_1$-$C_6$ hydroxyalkyl groups, among which mention may be made, for example, of hydroxymethyl, hydroxyethyl, hydroxypropyl and hydroxybutyl groups.

Such nonionic guar gums optionally modified with hydroxyalkyl groups are sold, for example, under the trade names Jaguar HP 60, Jaguar HP 105 and Jaguar HP 120 (hydroxypropyl guar) by the company Rhodia or under the name N-Hance® HP (hydroxypropyl guar) by the company Aqualon.

The cationic galactomannan gums preferably have a cationic charge density of less than or equal to 1.5 meq./g, more particularly between 0.1 and 1 meq./g. The charge density may be determined by the Kjeldahl method. It generally corresponds to a pH of the order of 3 to 9.

In general, for the purposes of the present invention, the term *"cationic galactomannan gum"* means any galactomannan gum containing cationic groups and/or groups that can be ionized into cationic groups.

The preferred cationic groups are chosen from those comprising primary, secondary, tertiary and/or quaternary amine groups.

The cationic galactomannan gums used generally have a weight-average molecular mass of between 500 and $5 \times 10^6$ approximately and preferably between $10^3$ and $3 \times 10^6$ approximately.

The cationic galactomannan gums that may be used according to the present invention are, for example, gums comprising tri(C1-C4)alkylammonium cationic groups. Preferably, 2% to 30% by number of the hydroxyl functions of these gums bear trialkylammonium cationic groups.

Among these trialkylammonium groups, mention may be made most particularly of trimethylammonium and triethylammonium groups.

Even more preferentially, these groups represent from 5% to 20% by weight relative to the total weight of the modified galactomannan gum.

According to the invention, the cationic galactomannan gum is preferably a guar gum comprising hydroxypropyltrimethylammonium groups, i.e. a guar gum modified, for example, with 2,3-epoxypropyltrimethylammonium chloride.

These galactomannan gums, in particular guar gums modified with cationic groups are products already known per se and are, for example, described in patents US 3 589 578 and US 4 031 307. Such products are moreover sold especially under the trade names Jaguar EXCEL, Jaguar C13 S, Jaguar C 15, Jaguar C 17 and Jaguar C162 (Guar Hydroxypropyltrimonium Chloride) by the company Rhodia, under the name Amilan® Guar (Guar Hydroxypropyltrimonium Chloride) by the company Degussa, and under the name N-Hance® 3000 (Guar Hydroxypropyltrimonium Chloride) by the company Aqualon.

The anionic guar gums that may be used according to the invention are polymers comprising groups derived from carboxylic, sulfonic, sulfenic, phosphoric, phosphonic or pyruvic acid. The anionic group is preferably a carboxylic acid group. The anionic group may also be in the form of an acid salt, especially a sodium, calcium, lithium or potassium salt.

The anionic guar gums that may be used according to the invention are preferentially carboxymethyl guar derivatives (carboxymethyl guar or carboxymethyl hydroxypropyl guar).

*Locust bean*

Locust bean gum is extracted from the seeds of the locust bean tree *(Ceratonia siliqua).*

The unmodified locust bean gum that may be used in this invention is sold, for example, under the name Viscogum™ by the company Cargill, under the name Vidogum L by the company Unipektin and under the name Grinsted® LBG by the company Danisco.

The chemically modified locust bean gums that may be used in this invention may be represented, for example, by the cationic locust beans sold under the name Catinal CLB (locust bean hydroxypropyltrimonium chloride) by the company Toho.

*Tara gum*

The tara gum that may be used in the context of this invention is sold, for example, under the name Vidogum SP by the company Unipektin.

*Glucomannans (konjac gum)*

Glucomannan is a polysaccharide of high molecular weight (500 000 < Mglucomannan < 2 000 000) composed of D-mannose and D-glucose units with a branch every 50 or 60 units approximately. It is found in wood, but is also the main constituent of konjac gum. Konjac (*Amorphophallus konjac*) is a plant of the Araceae family.

The products that may be used according to the invention are sold, for example, under the names Propol® and Rheolex® by the company Shimizu.

*LM and HM pectins, and derivatives*

Pectins are linear polymers of α-D-galacturonic acid (at least 65%) linked in positions 1 and 4 with a certain proportion of carboxylic groups esterified with a methanol group. About 20% of the sugars constituting the pectin molecule are neutral sugars (L-rhamnose, D-glucose, D-galactose, L-arabinose, D-xylose). L-Rhamnose residues are found in all pectins, incorporated into the main chain in positions 1,2.

Uronic acid molecules bear carboxyl functions. This function gives pectins the capacity for exchanging ions, when they are in $COO^-$ form. Divalent ions (in particular calcium) have the capacity of forming ionic bridges between two carboxyl groups of two different pectin molecules.

In the natural state, a certain proportion of the carboxylic groups are esterified with a methanol group. The natural degree of esterification of a pectin may range between 70% (apple, lemon) and 10% (strawberry) depending on the source used. Using pectins with a high degree of esterification it is possible to hydrolyse the $-COOCH_3$ groups, so as to obtain weakly esterified pectins. Depending on the proportion of methylated or non-methylated monomers, the chain is thus more or less acidic. HM (high-methoxy) pectins are thus defined as having a degree of esterification of greater than 50%, and LM (low-methoxy) pectins are defined as having a degree of esterification of less than 50%.

In the case of amidated pectins, the $-OCH_3$ group is substituted with a $-NH_2$ group.

Pectins are especially sold by the company Cargill under the name Unipectine™, by the company CP-Kelco under the name Genu, and by Danisco under the name Grinsted Pectin.

Other polysaccharides

Among the other polysaccharides that may be used according to the invention, mention may also be made of chitin (poly-N-acetyl-D-glucosamine, β(1,4)-2-acetamido-2-deoxy-D-glucose), chitosan and derivatives (chitosan-beta-glycerophosphate, carboxymethylchitin, etc.) such as those sold by the company France-Chitine; glycosaminoglycans (GAG) such as hyaluronic acid, chondroitin sulfate, dermatan sulfate, keratan sulfate, and preferably hyaluronic acid; xylans (or arabinoxylans) and derivatives.

Arabinoxylans are polymers of xylose and arabinose, all grouped under the name *pentosans.*

Xylans consist of a main chain of β(1,4)-linked D-xylose units and on which are found three substituents (Rouau & Thibault, 1987): acid units, α-L-arabinofuranose units, side chains which may contain arabinose, xylose, galactose and glucuronic acid.

According to this variant, the polysaccharide is preferably hyaluronic acid, or a salt thereof such as the sodium salt (sodium hyaluronate).

**II. Synthetic polymeric gelling agents**

For the purposes of the invention, the term "synthetic" means that the polymer is neither naturally existing nor a derivative of a polymer of natural origin.

The synthetic polymeric hydrophilic gelling agent under consideration according to the invention may or may not be particulate.

For the purposes of the invention, the term "particulate" means that the polymer is in the form of particles, preferably spherical particles.

As emerges from the text hereinbelow, the polymeric hydrophilic gelling agent is advantageously chosen from crosslinked acrylic homopolymers or copolymers; associative polymers, in particular associative polymers of polyurethane type; polyacrylamides and crosslinked and/or neutralized 2-acrylamido-2-methylpropanesulfonic acid polymers and copolymers; modified or unmodified carboxyvinyl polymers, and mixtures thereof, especially as defined below. At least one hydrophilic gelling agent is chosen from 2-acrylamido-2-methylpropanesulfonic acid polymers and copolymers.

*II.A. Particulate synthetic polymeric gelling agents*

They are preferably chosen from crosslinked polymers.

They may especially be crosslinked acrylic homopolymers or copolymers, which are preferably partially neutralized or neutralized, and which are in particulate form.

According to one embodiment, the particulate gelling agent according to the present invention is chosen from crosslinked sodium polyacrylates. Preferably, it has in the dry or non-hydrated state a mean size of less than or equal to 100 μm and preferably less than or equal to 50 μm. The mean size of the particles corresponds to the mass-average diameter (D50) measured by laser particle size analysis or another equivalent method known to those skilled in the art.

Thus, preferably, additional particulate gelling agents according to the present invention can be further chosen from crosslinked sodium polyacrylates, preferably in the form of particles with a mean size (or mean diameter) of less

than or equal to 100 microns, more preferably in the form of spherical particles.

As examples of crosslinked sodium polyacrylates, mention may be made of those sold under the brand names Octacare XI00, X110 and RM100 by the company Avecia, those sold under the names Flocare GB300 and Flosorb 500 by the company SNF, those sold under the names Luquasorb 1003, Luquasorb 1010, Luquasorb 1280 and Luquasorb 1110 by the company BASF, those sold under the names Water Lock G400 and G430 (INCI name: Acrylamide/Sodium acrylate copolymer) by the company Grain Processing.

Mention may also be made of crosslinked polyacrylate microspheres, for instance those sold under the name Aquakeep® 10 SH NF by the company Sumitomo Seika.

Such gelling agents may be used in a proportion of from 0.1% to 10% by weight of solids relative to the total weight of the aqueous phase, especially from 0.5% to 7% by weight and in particular in a proportion of about from 0.8% to 5% by weight, relative to the total weight of the aqueous phase.

***II.B. Non-particulate synthetic polymeric gelling agents***

This family of gelling agents may be detailed under the following subfamilies:

1. Associative polymers,
2. Polyacrylamides and crosslinked and/or neutralized 2-acrylamido-2-methylpropanesulfonic acid polymers and copolymers, and
3. Modified or unmodified carboxyvinyl polymers.

*II.B.1 Associative polymers*

For the purposes of the present invention, the term *"associative polymer"* means any amphiphilic polymer comprising in its structure at least one fatty chain and at least one hydrophilic portion. The associative polymers in accordance with the present invention may be anionic, cationic, nonionic or amphoteric.

Associative anionic polymers

Among the associative anionic polymers that may be mentioned are those comprising at least one hydrophilic unit, and at least one fatty-chain allyl ether unit, more particularly those whose hydrophilic unit is formed by an unsaturated ethylenic anionic monomer, more particularly by a vinylcarboxylic acid and most particularly by an acrylic acid or a methacrylic acid or mixtures thereof, and whose fatty-chain allyl ether unit corresponds to the monomer of formula (I) below:

$$CH_2 = C(R')CH_2\ O\ B_n\ R \qquad (I)$$

in which R' denotes H or $CH_3$, B denotes the ethylenoxy radical, n is zero or denotes an integer ranging from 1 to 100, R denotes a hydrocarbon-based radical chosen from alkyl, arylalkyl, aryl, alkylaryl and cycloalkyl radicals, comprising from 8 to 30 carbon atoms, preferably from 10 to 24 and even more particularly from 12 to 18 carbon atoms.

Anionic amphiphilic polymers of this type are described and prepared, according to an emulsion polymerization process, in patent EP 0 216 479.

Among the associative anionic polymers that may also be mentioned are maleic anhydride/$C_{30}$-$C_{38}$-$\alpha$-olefin/alkyl maleate terpolymers, such as the product maleic anhydiide/$C_{30}$-$C_{38}$-$\alpha$-olefin/isopropyl maleate copolymer sold under the name Performa V 1608 by the company New Phase Technologies.

Among the associative anionic polymers, mention may be made, according to a preferred embodiment, of copolymers comprising among their monomers an $\alpha,\beta$-monoethylenically unsaturated carboxylic acid and an ester of an $\alpha,\beta$-monoethylenically unsaturated carboxylic acid and of an oxyalkylenated fatty alcohol.

Preferentially, these compounds also comprise as monomer an ester of an $\alpha,\beta$-monoethylenically unsaturated carboxylic acid and of a $C_1$-$C_4$ alcohol.

Examples of compounds of this type that may be mentioned include Aculyn 22® sold by the company Rohm & Haas, which is a methacrylic acid/ethyl acrylate/oxyalkylenated stearyl methacrylate (comprising 20 EO units) terpolymer or Aculyn 28® (methacrylic acid/ethyl acrylate/oxyethylenated behenyl methacrylate (25 EO) terpolymer).

Associative anionic polymers that may also be mentioned include anionic polymers comprising at least one hydrophilic unit of unsaturated olefinic carboxylic acid type, and at least one hydrophobic unit exclusively of the type such as a ($C_{10}$-$C_{30}$) alkyl ester of an unsaturated carboxylic acid. Examples that may be mentioned include the anionic polymers described and prepared according to patents US 3 915 921 and US 4 509 949.

Associative anionic polymers that may also be mentioned include anionic terpolymers.

The anionic terpolymer used according to the invention is a linear or branched and/or crosslinked terpolymer, of at least one monomer (1) bearing an acid function in free form, which is partially or totally salified with a nonionic monomer (2) chosen from N,N-dimethylacrylamide and 2-hydroxyethyl acrylate and at least one polyoxyethylenated alkyl acrylate monomer (3) of formula (I) below:

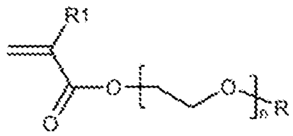

(I)

in which R1 represents a hydrogen atom, R represents a linear or branched $C_2$-$C_8$ alkyl radical and n represents a number ranging from 1 to 10.

The term *"branched polymer"* denotes a non-linear polymer which bears pendent chains so as to obtain, when this polymer is dissolved in water, a high degree of entanglement leading to very high viscosities, at a low speed gradient.

The term *"crosslinked polymer"* denotes a non-linear polymer which is in the form of a three-dimensional network that is insoluble in water but swellable in water, leading to the production of a chemical gel.

The acid function of the monomer (1) is especially a sulfonic acid or phosphonic acid function, said functions being in free or partially or totally salified form.

The monomer (1) may be chosen from styrenesulfonic acid, ethylsulfonic acid and 2-methyl-2-[(1-oxo-2-propenyl]amino]-1-propanesulfonic acid (also known as acryloyldimethyl taurate), in free or partially or totally salified form. It is present in the anionic terpolymer preferably in molar proportions of between 5 mol% and 95 mol% and more particularly between 10 mol% and 90 mol%. The monomer (1) will more particularly be 2-methyl-2-[(1-oxo-2-propenyl)amino]-1-propanesulfonic acid in free or partially or totally salified form.

The acid function in partially or totally salified form will preferably be an alkali metal salt such as a sodium or potassium salt, an ammonium salt, an amino alcohol salt such as a monoethanolamine salt, or an amino acid salt such as a lysine salt.

The monomer (2) is preferably present in the anionic terpolymer in molar proportions of between 4.9 mol% and 90 mol%, more particularly between 9.5 mol% and 85 mol% and even more particularly between 19.5 mol% and 75 mol%.

In formula (I), examples of linear $C_8$-$C_{16}$ alkyl radicals that may be mentioned include octyl, decyl, undecyl, tridecyl, tetradecyl, pentadecyl and hexadecyl.

In formula (I), examples of branched $C_8$-$C_{16}$ alkyl radicals that may be mentioned include 2-ethylhexyl, 2-propylheptyl, 2-butyloctyl, 2-pentylnonyl, 2-hexyldecyl, 4-methylpentyl, 5-methylhexyl, 6-methylheptyl, 15-methylpentadecyl, 16-methylheptadecyl and 2-hexyloctyl.

According to a particular form of the invention, in formula (I), R denotes a $C_{12}$-$C_{16}$ alkyl radical.

According to a particular form of the invention, in formula (I), n ranges from 3 to 5.

Tetraethoxylated lauryl acrylate will more particularly be used as monomer of formula (I).

The monomer (3) of formula (I) is preferably present in the anionic terpolymer in molar proportions of between 0.1 mol% and 10 mol% and more particularly between 0.5 mol% and 5 mol%.

According to a particular mode of the invention, the anionic terpolymer is crosslinked and/or branched with a diethylenic or polyethylenic compound in the proportion expressed relative to the total amount of monomers used, from 0.005 mol% to 1 mol%, preferably from 0.01 mol% to 0.5 mol% and more particularly from 0.01 mol% to 0.25 mol%.

The crosslinking agent and/or branching agent is preferably chosen from ethylene glycol dimethacrylate, diallyloxyacetic acid or a salt thereof, such as sodium diallyloxyacetate, tetraallyloxyethane, ethylene glycol diacrylate, diallylurea, triallylamine, trimethylolpropane triacrylate and methylenebis(acrylamide), or mixtures thereof.

The anionic terpolymer may contain additives such as complexing agents, transfer agents or chain-limiting agents.

Use will be made more particularly of an anionic terpolymer of 2-methyl-2-[(1-oxo-2-propenyl)amino]-1-propanesulfonic acid partially or totally salified in the form of the ammonium salt, N,N-dimethylacrylamide and tetraethoxylated lauryl acrylate crosslinked with trimethylolpropane triacrylate, of INCI name Polyacrylate Crosspolymer-6, such as the product sold under the trade name Sepimax Zen® by the company SEPPIC.

Cationic associative polymers

Cationic associative polymers that may be mentioned include polyacrylates bearing amine side groups.

The polyacrylates bearing quaternized or non-quaternized amino side groups contain, for example, hydrophobic groups of the type such as steareth-20 (polyoxyethylenated (20) stearyl alcohol).

Examples of polyacrylates bearing amino side chains that may be mentioned are the polymers 8781-121B or 9492-103 from the company National Starch.

Nonionic associative polymers

The nonionic associative polymers may be chosen from:

- copolymers of vinylpyrrolidone and of fatty-chain hydrophobic monomers;
- copolymers of $C_1$-$C_6$ alkyl methacrylates or acrylates and of amphiphilic monomers comprising at least one fatty chain;
- copolymers of hydrophilic methacrylates or acrylates and of hydrophobic monomers comprising at least one fatty chain, for instance the polyethylene glycol methacrylate/lauryl methacrylate copolymer;
- associative polyurethanes.

Associative polyurethanes are nonionic block copolymers comprising in the chain both hydrophilic blocks usually of polyoxyethylene nature (polyurethanes may also be referred to as polyurethane polyethers), and hydrophobic blocks that may be aliphatic sequences alone and/or cycloaliphatic and/or aromatic sequences.

In particular, these polymers comprise at least two hydrocarbon-based lipophilic chains containing from 6 to 30 carbon atoms, separated by a hydrophilic block, the hydrocarbon-based chains possibly being pendent chains or chains at the end of the hydrophilic block. In particular, it is possible for one or more pendent chains to be envisaged. In addition, the polymer may comprise a hydrocarbon-based chain at one end or at both ends of a hydrophilic block.

Associative polyurethanes may be block polymers, in triblock or multiblock form. The hydrophobic blocks may thus be at each end of the chain (for example: triblock copolymer containing a hydrophilic central block) or distributed both at the ends and in the chain (for example: multiblock copolymer). These polymers may also be graft polymers or star polymers. Preferably, the associative polyurethanes are triblock copolymers in which the hydrophilic block is a polyoxyethylene chain comprising from 50 to 1000 oxyethylene groups. In general, associative polyurethanes comprise a urethane bond between the hydrophilic blocks, whence arises the name.

According to one preferred embodiment, a nonionic associative polymer of polyurethane type is used as gelling agent.

As examples of nonionic fatty-chain polyurethane polyethers that may be used in the invention, it is also possible to use Rheolate® FX 1100 (Steareth-100/PEG 136/HDI (hexamethyl diisocyanate) copolymer), Rheolate® 205 containing a urea function, sold by the company Elementis, or Rheolate® 208, 204 or 212, and also Acrysol® RM 184 or Acrysol® RM 2020.

Mention may also be made of the product Elfacos® T210 containing a $C_{12}$-$C_{14}$ alkyl chain, and the product Elfacos® T212 containing a $C_{16-18}$ alkyl chain (PPG-14 Palmeth-60 Hexyl Dicarbamate), from Akzo.

The product DW 1206B® from Rohm & Haas containing a $C_{20}$ alkyl chain and a urethane bond, sold at a solids content of 20% in water, may also be used.

Use may also be made of solutions or dispersions of these polymers, especially in water or in aqueous-alcoholic medium. Examples of such polymers that may be mentioned are Rheolate® 255, Rheolate® 278 and Rheolate® 244 sold by the company Elementis. The products DW 1206F and DW 1206J sold by the company Rohm & Haas may also be used.

The associative polyurethanes that may be used according to the invention are in particular those described in the article by G. Fonnum, J. Bakke and Fk. Hansen, Colloid Polym. Sci., 271, 380-389 (1993).

Even more particularly, according to the invention, use may also be made of an associative polyurethane that may be obtained by polycondensation of at least three compounds comprising (i) at least one polyethylene glycol comprising from 150 to 180 mol of ethylene oxide, (ii) stearyl alcohol or decyl alcohol, and (iii) at least one diisocyanate.

Such polyurethane polyethers are sold in particular by the company Rohm & Haas under the names Aculyn® 46 and Aculyn® 44. Aculyn® 46 is a polycondensate of polyethylene glycol containing 150 or 180 mol of ethylene oxide, of stearyl alcohol and of methylenebis(4-cyclohexyl isocyanate) (SMDI), at 15% by weight in a matrix of maltodextrin (4%) and water (81%), and Aculyn® 44 is a polycondensate of polyethylene glycol containing 150 or 180 mol of ethylene oxide, of decyl alcohol and of methylenebis(4-cyclohexyl isocyanate) (SMDI), at 35% by weight in a mixture of propylene glycol (39%) and water (26%).

Use may also be made of solutions or dispersions of these polymers, especially in water or in aqueous-alcoholic medium. Examples of such polymers that may be mentioned include SER AD FX1010, SER AD FX1035 and SER AD 1070 from the company Elementis, and Rheolate® 255, Rheolate® 278 and Rheolate® 244 sold by the company Elementis. Use may also be made of the products Aculyn® 44, Aculyn® 46, DW 1206F and DW 1206J, and also Acrysol® RM 184 from the company Rohm & Haas, or alternatively Borchigel LW 44 from the company Borchers, and mixtures thereof.

Amphoteric associative polymers

Among the associative amphoteric polymers of the invention, mention may be made of crosslinked or non-crosslinked, branched or unbranched amphoteric polymers, which may be obtained by copolymerization:

1) of at least one monomer of formula (IVa) or (IVb):

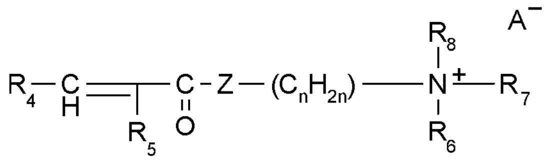

(IVa)

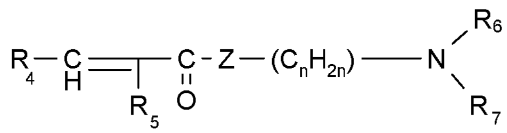

(IVb)

in which $R_4$ and $R_5$, which may be identical or different, represent a hydrogen atom or a methyl radical,
$R_6$, $R_7$ and $R_8$, which may be identical or different, represent a linear or branched alkyl radical containing from 1 to 30 carbon atoms;
Z represents an NH group or an oxygen atom;
n is an integer from 2 to 5;
$A^-$ is an anion derived from a mineral or organic acid, such as a methosulfate anion or a halide such as chloride or bromide;

2) of at least one monomer of formula (V):

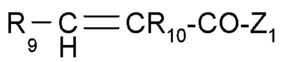

(V)

in which $R_9$ and $R_{10}$, which may be identical or different, represent a hydrogen atom or a methyl radical;
$Z_1$ represents a group OH or a group $NHC(CH_3)_2CH_2SO_3H$;

3) of at least one monomer of formula (VI):

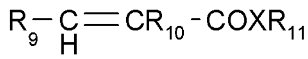

(VI)

in which $R_9$ and $R_{10}$, which may be identical or different, represent a hydrogen atom or a methyl radical, X denotes an oxygen or nitrogen atom and $R_{11}$ denotes a linear or branched alkyl radical containing from 1 to 30 carbon atoms;
4) optionally at least one crosslinking or branching agent; at least one of the monomers of formula (IVa), (IVb) or (VI) comprising at least one fatty chain containing from 8 to 30 carbon atoms and said compounds of the monomers of formulae (IVa), (IVb), (V) and (VI) possibly being quaternized, for example with a $C_1$-$C_4$ alkyl halide or a $C_1$-$C_4$ dialkyl sulfate.

The monomers of formulae (IVa) and (IVb) of the present invention are preferably chosen from the group consisting of:

- dimethylaminoethyl methacrylate, dimethylaminoethyl acrylate,
- diethylaminoethyl methacrylate, diethylaminoethyl acrylate,
- dimethylaminopropyl methacrylate, dimethylaminopropyl acrylate,
- dimethylaminopropylmethacrylamide, dimethylaminopropylacrylamide,

which are optionally quaternized, for example with a $C_1$-$C_4$ alkyl halide or a $C_1$-$C_4$ dialkyl sulfate.

More particularly, the monomer of formula (IVa) is chosen from acrylamidopropyltrimethylammonium chloride

and methacrylamidopropyl-trimethylammonium chloride.

The compounds of formula (V) of the present invention are preferably chosen from the group formed by acrylic acid, methacrylic acid, crotonic acid, 2-methylcrotonic acid, 2-acrylamido-2-methylpropanesulfonic acid and 2-methacrylamido-2-methylpropanesulfonic acid. More particularly, the monomer of formula (V) is acrylic acid.

The monomers of formula (VI) of the present invention are preferably chosen from the group formed by $C_{12}$-$C_{22}$ and more particularly $C_{16}$-$C_{18}$ alkyl acrylates or methacrylates.

The crosslinking or branching agent is preferably chosen from N,N'-methylenebisacrylamide, triallylmethylammonium chloride, allyl methacrylate, n-methylolacrylamide, polyethylene glycol dimethacrylates, ethylene glycol dimethacrylate, diethylene glycol dimethacrylate, 1,6-hexanediol dimethacrylate and allyl sucrose.

The polymers according to the invention may also contain other monomers such as nonionic monomers and in particular such as $C_1$-$C_4$ alkyl acrylates or methacrylates.

The ratio of the number of cationic charges/anionic charges in these amphoteric polymers is preferably equal to about 1.

The weight-average molecular weights of the associative amphoteric polymers have a weight-average molecular mass of greater than 500, preferably between 10 000 and 10 000 000 and even more preferentially between 100 000 and 8 000 000.

Preferably, the associative amphoteric polymers of the invention contain from 1 mol% to 99 mol%, more preferentially from 20 mol% to 95 mol% and even more preferentially from 25 mol% to 75 mol% of compound(s) of formula (IVa) or (IVb). They also preferably contain from 1 mol% to 80 mol%, more preferentially from 5 mol% to 80 mol% and even more preferentially from 25 mol% to 75 mol% of compound(s) of formula (V). The content of compound(s) of formula (VI) is preferably between 0.1 mol% and 70 mol%, more preferentially between 1 mol% and 50 mol% and even more preferentially between 1 mol% and 10 mol%. The crosslinking or branching agent, when it is present, is preferably between 0.0001 mol% and 1 mol% and even more preferentially between 0.0001 mol% and 0.1 mol%.

Preferably, the mole ratio between the compound(s) of formula (IVa) or (IVb) and the compound(s) of formula (V) ranges from 20/80 to 95/5 and more preferentially from 25/75 to 75/25.

The associative amphoteric polymers according to the invention are described, for example, in patent application WO 98/44012.

The amphoteric polymers that are particularly preferred according to the invention are chosen from acrylic acid/acrylamidopropyltrimethylammonium chloride/stearyl methacrylate copolymers.

According to a preferred embodiment, the associative polymer is chosen from nonionic associative polymers and more particularly from associative polyurethanes, such as Steareth-100/PEG-136/HDI Copolymer sold under the name Rheolate FX 1100 by Elementis.

Such an associative polymer is advantageously used in a proportion of from 0.1% to 8% by weight of solids and preferably between 0.5% and 4% by weight, relative to the total weight of the aqueous phase.

*II.B.2 Polyacrylamides and 2-acrylamido-2-methylpropanesulfonic acid polymers and copolymers*

The polymers used that are suitable as aqueous gelling agent for the invention may be crosslinked or non-crosslinked homopolymers or copolymers comprising at least the 2-acrylamido-2-methylpropanesulfonic acid (AMPS®) monomer, in a form partially or totally neutralized with a mineral base other than aqueous ammonia, such as sodium hydroxide or potassium hydroxide.

They are preferably totally or almost totally neutralized, i.e. at least 90% neutralized.

These AMPS® polymers according to the invention may be crosslinked or non-crosslinked.

When the polymers are crosslinked, the crosslinking agents may be chosen from the polyolefinically unsaturated compounds commonly used for crosslinking polymers obtained by radical polymerization.

Examples of crosslinking agents that may be mentioned include divinylbenzene, diallyl ether, dipropylene glycol diallyl ether, polyglycol diallyl ethers, triethylene glycol divinyl ether, hydroquinone diallyl ether, ethylene glycol or tetraethylene glycol di(meth)acrylate, trimethylolpropane triacrylate, methylenebisacrylamide, methylenebismethacrylamide, triallylamine, triallyl cyanurate, diallyl maleate, tetraallylethylenediamine, tetraallyloxyethane, trimethylolpropane diallyl ether, allyl (meth)acrylate, allylic ethers of alcohols of the sugar series, or other allylic or vinyl ethers of polyfunctional alcohols, and also the allylic esters of phosphoric and/or vinylphosphonic acid derivatives, or mixtures of these compounds.

According to one preferred embodiment of the invention, the crosslinking agent is chosen from methylenebisacrylamide, allyl methacrylate and trimethylolpropane triacrylate (TMPTA). The degree of crosslinking generally ranges from 0.01 mol% to 10 mol% and more particularly from 0.2 mol% to 2 mol% relative to the polymer.

The AMPS® polymers that are suitable for use in the invention are water-soluble or water-dispersible. In this case, they are:

- either "homopolymers" comprising only AMPS monomers and, if they are crosslinked, one or more crosslinking agents such as those defined above;
- or copolymers obtained from AMPS® and from one or more hydrophilic or hydrophobic ethylenically unsaturated monomers and, if they are crosslinked, one or more crosslinking agents such as those defined above. When said copolymers comprise hydrophobic ethylenically unsaturated monomers, these monomers do not comprise a fatty chain and are preferably present in small amounts.

For the purpose of the present invention, the term *"fatty chain"* is intended to mean any hydrocarbon-based chain comprising at least 7 carbon atoms.

The term *"water-soluble or water-dispersible"* means polymers which, when introduced into an aqueous phase at 25°C, at a mass concentration equal to 1%, make it possible to obtain a macroscopically homogeneous and transparent solution, i.e. a solution with a maximum light transmittance value, at a wavelength equal to 500 nm, through a sample 1 cm thick, of at least 60% and preferably of at least 70%.

The *"homopolymers"* according to the invention are preferably crosslinked and neutralized, and they may be obtained according to the preparation process comprising the following steps:

(a) the monomer such as AMPS in free form is dispersed or dissolved in a solution of tert-butanol or of water and tert-butanol;
(b) the monomer solution or dispersion obtained in (a) is neutralized with one or more mineral or organic bases, preferably aqueous ammonia $NH_3$, in an amount making it possible to obtain a degree of neutralization of the sulfonic acid functions of the polymer ranging from 90% to 100%;
(c) the crosslinking monomer(s) are added to the solution or dispersion obtained in (b);
(d) a standard free-radical polymerization is performed in the presence of free-radical initiators at a temperature ranging from 10°C to 150°C; the polymer precipitates from the tert-butanol-based solution or dispersion.

The water-soluble or water-dispersible AMPS® copolymers according to the invention contain water-soluble ethylenically unsaturated monomers, hydrophobic monomers, or mixtures thereof.

The water-soluble comonomers may be ionic or nonionic.

Among the ionic water-soluble comonomers, examples that may be mentioned include the following compounds, and salts thereof:

- (meth)acrylic acid,
- styrenesulfonic acid,
- vinylsulfonic acid and (meth)allylsulfonic acid,
- vinylphosphonic acid,
- maleic acid,
- itaconic acid,
- crotonic acid,
- water-soluble vinyl monomers of formula (A) below:

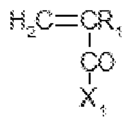
(A)

in which:

- $R_1$ is chosen from H, $-CH_3$, $-C_2H_5$ and $-C_3H_7$,
- $X_1$ is chosen from:
- alkyl oxides of type $-OR_2$ where $R_2$ is a linear or branched, saturated or unsaturated hydrocarbon-based radical containing from 1 to 6 carbon atoms, substituted with at least one sulfonic ($-SO_3-$) and/or sulfate ($-SO_4-$) and/or phosphate ($-PO_4H_2-$) group.

Among the nonionic water-soluble comonomers, examples that may be mentioned include:

- (meth)acrylamide,
- N-vinylacetamide and N-methyl-N-vinylacetamide,
- N-vinylformamide and N-methyl-N-vinylformamide,

- maleic anhydride,
- vinylamine,
- N-vinyllactams comprising a cyclic alkyl group containing from 4 to 9 carbon atoms, such as N-vinylpyrrolidone, N-butyrolactam and N-vinylcaprolactam,
- vinyl alcohol of formula $CH_2$=CHOH,
- water-soluble vinyl monomers of formula (B) below:

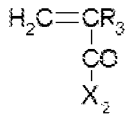
(B)

in which:

  - $R_3$ is chosen from H, $-CH_3$, $-C_2H_5$ and $-C_3H_7$,
  - $X_2$ is chosen from alkyl oxides of the type $-OR_4$ where $R_4$ is a linear or branched, saturated or unsaturated hydrocarbon-based radical containing from 1 to 6 carbon atoms, optionally substituted with a halogen (iodine, bromine, chlorine or fluorine) atom; a hydroxyl (-OH) group; ether.

Mention is made, for example, of glycidyl (meth)acrylate, hydroxyethyl methacrylate, and (meth)acrylates of ethylene glycol, of diethylene glycol or of polyalkylene glycol.
Among the hydrophobic co-monomers without a fatty chain, mention may be made, for example, of:

- styrene and derivatives thereof, such as 4-butylstyrene, $\alpha$-methylstyrene and vinyltoluene;
- vinyl acetate of formula $CH_2$=CH-$OCOCH_3$;
- vinyl ethers of formula $CH_2$=CHOR in which R is a linear or branched, saturated or unsaturated hydrocarbon-based radical containing from 1 to 6 carbons;
- acrylonitrile;
- caprolactone;
- vinyl chloride and vinylidene chloride;
- silicone derivatives, which, after polymerization, result in silicone polymers such as methacryloxypropyltris(trimethylsiloxy)silane and silicone methacrylamides;
- hydrophobic vinyl monomers of formula (C) below:

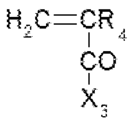
(C)

in which:

  - $R_4$ is chosen from H, $-CH_3$, $-C_2H_5$ and $-C_3H_7$;
  - $X_3$ is chosen from:
  - alkyl oxides of the type $-OR_5$ where $R_5$ is a linear or branched, saturated or unsaturated hydrocarbon-based radical containing from 1 to 6 carbon atoms.

Mention is made, for example, of methyl methacrylate, ethyl methacrylate, n-butyl (meth)acrylate, tert-butyl (meth)acrylate, cyclohexyl acrylate, isobornyl acrylate and 2-ethylhexyl acrylate.
The water-soluble or water-dispersible AMPS® polymers of the invention preferably have a molar mass ranging from 50 000 g/mol to 10 000 000 g/mol, preferably from 80 000 g/mol to 8 000 000 g/mol, and even more preferably from 100 000 g/mol to 7 000 000 g/mol.
As water-soluble or water-dispersible AMPS homopolymers suitable for use in the invention, mention may be made, for example, of crosslinked or non-crosslinked polymers of sodium acrylamido-2-methylpropanesulfonate, such as that used in the commercial product Simulgel 800 (CTFA name: Sodium Polyacryloyldimethyl Taurate), crosslinked ammonium acrylamido-2-methylpropanesulfonate polymers (INCI name: Ammonium Polyacryldimethyltauramide) such as those described in patent EP 0 815 928 B1 and such as the product sold under the trade name Hostacerin AMPS® by the company Clariant.
As water-soluble or water-dispersible AMPS copolymers in accordance with the invention, examples that may

be mentioned include:

- crosslinked acrylamide/sodium acrylamido-2-methylpropanesulfonate copolymers, such as that used in the commercial product Sepigel 305® (CTFA name: Polyacrylamide/$C_{13}$-$C_{14}$ Isoparaffin/ Laureth-7) or that used in the commercial product sold under the name Simulgel 600 (CTFA name: Acrylamide/Sodium acryloyldimethyltaurate/Isohexadecane/Polysorbate-80) by the company SEPPIC;
- copolymers of AMPS® and of vinylpyrrolidone or vinylformamide, such as that used in the commercial product sold under the name Aristoflex AVC® by the company Clariant (CTFA name: Ammonium Acryloyldimethyltaurate/VP copolymer) but neutralized with sodium hydroxide or potassium hydroxide;
- copolymers of AMPS® and of sodium acrylate, for instance the AMPS/sodium acrylate copolymer, such as that used in the commercial product sold under the name Simulgel EG® by the company SEPPIC;
- copolymers of AMPS® and of hydroxyethyl acrylate, for instance the AMPS®/hydroxyethyl acrylate copolymer, such as that used in the commercial product sold under the name Simulgel NS® by the company SEPPIC (CTFA name: Hydroxyethyl acrylate/Sodium acryloyldimethyltaurate copolymer (and) Squalane (and) Polysorbate 60), or such as the product sold under the name Sodium acrylamido-2-methylpropanesulfonate/Hydroxyethyl acrylate copolymer, such as the commercial product Sepinov EMT 10 or under the trade name Sepinov EM (INCI name: Hydroxyethyl acrylate/Sodium acryloyldimethyltaurate copolymer).

As preferred water-soluble or water-dispersible AMPS copolymers in accordance with the invention, mention may be made of copolymers of AMPS® and of hydroxyethyl acrylate.
In general, an aqueous phase according to the invention may comprise from 0.1% to 8% by weight, preferably from 0.2% to 5% by weight and more preferentially from 0.7% to 5% by weight of solids of polyacrylamide(s) and/or of crosslinked and/or neutralized 2-acrylamido-2-methylpropanesulfonic acid polymer(s) and copolymer(s) relative to its total weight.

*II.B. 3 Modified or unmodified carboxyvinyl polymers*

The modified or unmodified carboxyvinyl polymers may be copolymers derived from the polymerization of at least one monomer (a) chosen from $\alpha,\beta$-ethylenically unsaturated carboxylic acids or esters thereof, with at least one ethylenically unsaturated monomer (b) comprising a hydrophobic group.
The term *"copolymers"* means both copolymers obtained from two types of monomer and those obtained from more than two types of monomer, such as terpolymers obtained from three types of monomer.
Their chemical structure more particularly comprises at least one hydrophilic unit and at least one hydrophobic unit. The term "hydrophobic group or unit" means a radical with a saturated or unsaturated, linear or branched hydrocarbon-based chain, comprising at least 8 carbon atoms, preferably from 10 to 30 carbon atoms, in particular from 12 to 30 carbon atoms and more preferentially from 18 to 30 carbon atoms.
Preferably, these copolymers are chosen from copolymers derived from the polymerization:

- of at least one monomer of formula (1) below:

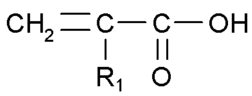
(1)

in which $R_1$ denotes H or $CH_3$ or $C_2H_5$, i.e. acrylic acid, methacrylic acid or ethacrylic acid monomers, and
- of at least one monomer of unsaturated carboxylic acid ($C_{10}$-$C_{30}$)alkyl ester type corresponding to the monomer of formula (2) below:

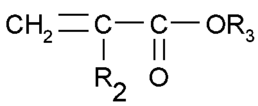
(2)

in which $R_2$ denotes H or $CH_3$ or $C_2H_5$ (i.e. acrylate, methacrylate or ethacrylate units) and preferably H (acrylate units) or $CH_3$ (methacrylate units), $R_3$ denoting a $C_{10}$-$C_{30}$ and preferably $C_{12}$-$C_{22}$ alkyl radical.

The unsaturated carboxylic acid ($C_{10}$-$C_{30}$)alkyl esters are preferably chosen from lauryl acrylate, stearyl acrylate,

decyl acrylate, isodecyl acrylate and dodecyl acrylate, and the corresponding methacrylates, such as lauryl methacrylate, stearyl methacrylate, decyl methacrylate, isodecyl methacrylate and dodecyl methacrylate, and mixtures thereof.

According to a preferred embodiment, these polymers are crosslinked.

Among the copolymers of this type that will be used more particularly are polymers derived from the polymerization of a monomer mixture comprising:

- essentially acrylic acid,
- an ester of formula (2) described above in which $R_2$ denotes H or $CH_3$, $R_3$ denoting an alkyl radical containing from 12 to 22 carbon atoms, and
- a crosslinking agent, which is a well-known copolymerizable unsaturated polyethylenic monomer, such as diallyl phthalate, allyl (meth)acrylate, divinylbenzene, (poly)ethylene glycol dimethacrylate and methylenebisacrylamide.

Among the copolymers of this type, use will more particularly be made of those consisting of from 95% to 60% by weight of acrylic acid (hydrophilic unit), 4% to 40% by weight of $C_{10}$-$C_{30}$ alkyl acrylate (hydrophobic unit) and 0 to 6% by weight of crosslinking polymerizable monomer, or alternatively those consisting of from 98% to 96% by weight of acrylic acid (hydrophilic unit), 1% to 4% by weight of $C_{10}$-$C_{30}$ alkyl acrylate (hydrophobic unit) and 0.1% to 0.6% by weight of crosslinking polymerizable monomer such as those described previously.

Among the abovementioned polymers, the ones that are most particularly preferred according to the present invention are acrylate/$C_{10}$-$C_{30}$-alkyl acrylate copolymers (INCI name: Acrylates/$C_{10-30}$ Alkyl acrylate Crosspolymer) such as the products sold by the company Lubrizol under the trade names Pemulen TR-1, Pemulen TR-2, Carbopol 1382, Carbopol EDT 2020 and Carbopol Ultrez 20 Polymer, and even more preferentially Pemulen TR-2.

Among the modified or unmodified carboxyvinyl polymers, mention may also be made of sodium polyacrylates such as those sold under the name Cosmedia SP® containing 90% solids and 10% water, or Cosmedia SPL® as an inverse emulsion containing about 60% solids, an oil (hydrogenated polydecene) and a surfactant (PPG-5 Laureth-5), both sold by the company Cognis.

Mention may also be made of partially neutralized sodium polyacrylates that are in the form of an inverse emulsion comprising at least one polar oil, for example the product sold under the name Luvigel® EM sold by the company BASF.

The modified or unmodified carboxyvinyl polymers may also be chosen from crosslinked (meth)acrylic acid homopolymers.

For the purposes of the present patent application, the term *"(meth)acrylic"* means *"acrylic or methacrylic"*.

Examples that may be mentioned include the products sold by Lubrizol under the names Carbopol 910, 934, 940, 941, 934 P, 980, 981, 2984, 5984 and Carbopol Ultrez 10 Polymer, or by 3V-Sigma under the name Synthalen® K, Synthalen® L or Synthalen® M.

Among the modified or unmodified carboxyvinyl polymers, mention may be made in particular of Carbopol (INCI name: carbomer) and Pemulen (CTFA name: Acrylates/$C_{10-30}$ alkyl acrylate crosspolymer) sold by the company Lubrizol.

The modified or unmodified carboxyvinyl polymers may be present in a proportion of from 0.1% to 5% by weight of solids relative to the weight of the aqueous phase, in particular from 0.3% to 2% by weight, preferably between 0.4% and 2% and preferentially between 0.4% and 1% by weight, relative to the weight of the aqueous phase.

Advantageously, a composition according to the invention comprises a synthetic polymeric hydrophilic gelling agent chosen from 2-acrylamido-2-methylpropanesulfonic acid polymers and copolymers.

According to a preferred variant, the synthetic polymeric hydrophilic gelling agent is a crosslinked sodium polyacrylate or, preferably, a copolymer of 2-acrylamido-2-methylpropanesulfonic acid and of hydroxy ethyl acrylate.

## III. Other hydrophilic gelling agents

These gelling agents are more particularly chosen from mixed silicates and fumed silicas.

### ***III.A.*** *Mixed silicate*

For the purposes of the present invention, the term "mixed silicate" means all silicates of natural or synthetic origin containing several (two or more) types of cations chosen from alkali metals (for example Na, Li, K) or alkaline-earth metals (for example Be, Mg, Ca), transition metals and aluminium.

According to a particular embodiment, the mixed silicate(s) are in the form of solid particles containing at least 10% by weight of at least one silicate relative to the total weight of the particles. In the rest of the present description, these particles are referred to as *"silicate particles"*.

Preferably, the silicate particles contain less than 1% by weight of aluminium relative to the total weight of the particles. Even more preferably, they contain from 0 to 1% by weight of aluminium relative to the total weight of the particles.

Preferably, the silicate particles contain at least 50% by weight of silicate and better still at least 70% by weight relative to the total weight of the particles. Particles containing at least 90% by weight of silicates, relative to the total weight of the particles, are particularly preferred.
In particular, it is an alkali metal or alkaline-earth metal, aluminium or iron silicate or mixture of silicates.
Preferably, it is sodium, magnesium and/or lithium silicate.
To ensure good cosmetic properties, these silicates are generally in a finely divided form, and in particular in the form of particles with a mean size ranging from 2 nm to 1 μm (from 2 nm to 1000 nm), preferably from 5 nm to 600 nm and even more preferentially from 20 to 250 nm.
The silicate particles may have any form, for example the form of spheres, flakes, needles, platelets, discs, leaflets, or totally random forms. Preferably, the silicate particles are in the form of discs or leaflets.
Thus, the term "mean size" of the particles means the numerical mean size of the largest dimension (length) that it is possible to measure between two diametrically opposite points on an individual particle. The size may be determined, for example, by transmission electron microscopy or by measuring the specific surface area via the BET method or by laser particle size analysis.
When the particles are in the form of discs or leaflets, they generally have a thickness ranging from about 0.5 nm to 5 nm.
The silicate particles may consist of an alloy with metal or metalloid oxides, obtained, for example, by thermal melting of the various constituents thereof. When the particles also comprise such a metal or metalloid oxide, this oxide is preferably chosen from silicon, boron or aluminium oxide.
According to a particular embodiment of the invention, the silicates are phyllosilicates, namely silicates having a structure in which the $SiO_4$ tetrahedra are organized in leaflets between which the metal cations are enclosed.
The mixed silicates that are suitable for use in the invention may be chosen, for example, from montmorillonites, hectorites, bentonites, beidellite and saponites. According to a preferred embodiment of the invention, the mixed silicates used are more particularly chosen from hectorites and bentonites, and better still from laponites.
A family of silicates that is particularly preferred in the compositions of the present invention is thus the laponite family. Laponites are sodium magnesium silicates also possibly containing lithium, which have a layer structure similar to that of montmorillonites. Laponite is the synthetic form of the natural mineral known as *hectorite.* The synthetic origin of this family of silicates is of considerable advantage over the natural form, since it allows good control of the composition of the product. In addition, laponites have the advantage of having a particle size that is much smaller than that of the natural minerals hectorite and bentonite.
Laponites that may especially be mentioned include the products sold under the following names: Laponite® XLS, Laponite® XLG, Laponite® RD, Laponite® RDS, Laponite® XL21 (these products are sodium magnesium silicates and sodium lithium magnesium silicates) by the company Rockwood Additives Limited.
Such gelling agents may be used in a proportion of from 0.1% to 8% by weight of solids relative to the total weight of the aqueous phase, especially from 0.1% to 5% by weight and in particular from 0.5% to 5% by weight, relative to the total weight of the aqueous phase.

### *III.B. Hydrophilic fumed silica*

The fumed silicas according to the present invention are hydrophilic.
The hydrophilic fumed silicas are obtained by pyrolysis of silicon tetrachloride ($SiCl_4$) in a continuous flame at 1000°C in the presence of hydrogen and oxygen. Among the fumed silicas of hydrophilic nature that may be used according to the present invention, mention may be made in particular of those sold by the company Degussa or Evonik Degussa under the trade names Aerosil® 90, 130, 150, 200, 300 and 380 or alternatively by the company Cabot under the name Carbosil H5.
Such gelling agents may be used in a proportion of from 0.1% to 10% by weight of solids relative to the total weight of the aqueous phase, especially from 0.1% to 5% by weight and in particular from 0.5% to 3% by weight, relative to the total weight of the aqueous phase.

## LIPOPHILIC GELLING AGENT

For the purposes of the present invention, the term *"lipophilic gelling agent"* means a compound that is capable of gelling the oily phase of the compositions according to the invention.
The gelling agent is lipophilic and is thus present in the oily phase of the composition.
The gelling agent is liposoluble or lipodispersible.
As emerges from the text hereinbelow, the lipophilic gelling agent is advantageously chosen from particulate gelling agents, organopolysiloxane elastomers, semi-crystalline polymers, dextrin esters and hydrogen bonding polymers, hydrocarbon-based block copolymers, and mixtures thereof.

## I. Particulate gelling agents

The particulate gelling agent used in the composition according to the invention is in the form of particles, preferably spherical particles.

As representative lipophilic particulate gelling agents that are suitable for use in the invention, mention may be made most particularly of polar and apolar waxes, modified clays, and silicas such as fumed silicas and hydrophobic silica aerogels.

### Waxes

The term "wax" under consideration in the context of the present invention generally means a lipophilic compound that is solid at room temperature (25°C), with a solid/liquid reversible change of state, having a melting point of greater than or equal to 30°C, which may be up to 200°C and in particular up to 120°C.

For the purposes of the invention, the melting point corresponds to the temperature of the most endothermic peak observed in thermal analysis (DSC) as described in the standard ISO 11357-3; 1999. The melting point of the wax may be measured using a differential scanning calorimeter (DSC), for example the calorimeter sold under the name MDSC 2920 by the company TA Instruments.

The measuring protocol is as follows:
A 5 mg sample of wax placed in a crucible is subjected to a first temperature increase from -20°C to 100°C, at a heating rate of 10°C/minute, and then is cooled from 100°C to -20°C at a cooling rate of 10°C/minute and is finally subjected to a second temperature rise from -20°C to 100°C at a heating rate of 5°C/minute. During the second temperature rise, the variation in the difference in power absorbed by the empty crucible and by the crucible containing the sample of wax is measured as a function of the temperature. The melting point of the compound is the temperature value corresponding to the top of the peak of the curve representing the variation in the difference in power absorbed as a function of the temperature.

The waxes that may be used in the compositions according to the invention are chosen from waxes that are solid at room temperature of animal, plant, mineral or synthetic origin, and mixtures thereof.

The waxes, for the purposes of the invention, may be those used generally in the cosmetic or dermatological fields. They may in particular be polar or apolar, and hydrocarbon-based, silicone and/or fluoro waxes, optionally comprising ester or hydroxyl functions. They may also be of natural or synthetic origin.

#### a) Apolar waxes

For the purposes of the present invention, the term "apolar wax" means a wax whose solubility parameter at 25°C as defined below, $\delta_a$, is equal to 0 $(J/cm^3)^{½}$.

The definition and calculation of the solubility parameters in the Hansen three-dimensional solubility space are described in the article by C.M. Hansen: The three-dimensional solubility parameters, J. Paint Technol. 39, 105 (1967).

According to this Hansen space:

- $\delta_D$ characterizes the London dispersion forces derived from the formation of dipoles induced during molecular impacts;
- $\delta_p$ characterizes the Debye interaction forces between permanent dipoles and also the Keesom interaction forces between induced dipoles and permanent dipoles;
- $\delta_h$ characterizes the specific interaction forces (such as hydrogen bonding, acid/base, donor/acceptor, etc.); and
- $\delta_a$ is determined by the equation: $\delta_a = (\delta_p^2 + \delta_h^2)^{½}$.

The parameters $\delta_p$, $\delta_h$, $\delta_D$ and $\delta_a$ are expressed in $(J/cm^3)^{½}$.

The apolar waxes are in particular hydrocarbon-based waxes constituted solely of carbon and hydrogen atoms, and free of heteroatoms such as N, O, Si and P.

The apolar waxes are chosen from microcrystalline waxes, paraffin waxes, ozokerite and polyethylene waxes, and mixtures thereof.

An ozokerite that may be mentioned is Ozokerite Wax SP 1020 P.

As microcrystalline waxes that may be used, mention may be made of Multiwax W 445® sold by the company Sonneborn, and Microwax HW® and Base Wax 30540® sold by the company Paramelt, and Cerewax® No. 3 sold by the company Baerlocher.

As microwaxes that may be used in the compositions according to the invention as apolar wax, mention may be made in particular of polyethylene microwaxes such as those sold under the names Micropoly 200®, 220®, 220L® and 250S® by the company Micro Powders.

Polyethylene waxes that may be mentioned include Performalene 500-L Polyethylene and Performalene 400 Polyethylene sold by New Phase Technologies, and Asensa® SC 211 sold by the company Honeywell.

b) Polar wax

For the purposes of the present invention, the term "*polar wax"* means a wax whose solubility parameter at 25°C, δa, is other than 0 $(J/cm^3)^{½}$.

In particular, the term "*polar* wax" means a wax whose chemical structure is formed essentially from, or even consists of, carbon and hydrogen atoms, and comprising at least one highly electronegative heteroatom such as an oxygen, nitrogen, silicon or phosphorus atom.

The polar waxes may in particular be hydrocarbon-based, fluoro or silicone waxes.

Preferentially, the polar waxes may be hydrocarbon-based waxes.

The term "hydrocarbon-based wax" is intended to mean a wax formed essentially from, or even constituted of, carbon and hydrogen atoms, and optionally oxygen and nitrogen atoms, and that does not contain any silicon or fluorine atoms. It may also contain alcohol, ester, ether, carboxylic acid, amine and/or amide groups.

According to the invention, the term "ester wax" is intended to mean a wax comprising at least one ester function. According to the invention, the term "alcohol wax" is intended to mean a wax comprising at least one alcohol function, i.e. comprising at least one free hydroxyl (OH) group.

The following may especially be used as ester wax:

- ester waxes such as those chosen from:

  i) waxes of formula $R_1COOR_2$ in which $R_1$ and $R_2$ represent linear, branched or cyclic aliphatic chains in which the number of atoms ranges from 10 to 50, which may contain a heteroatom such as O, N or P and whose melting point ranges from 25 to 120°C;

  ii) bis(1,1,1-trimethylolpropane) tetrastearate, sold under the name Hest 2T-4S® by the company Heterene;

  iii) diester waxes of a dicarboxylic acid of general formula $R^3$-(-OCO-$R^4$-COO-$R^5$), in which $R^3$ and $R^5$ are identical or different, preferably identical, and represent a $C_4$-$C_{30}$ alkyl group (alkyl group comprising from 4 to 30 carbon atoms) and $R^4$ represents a linear or branched $C_4$-$C_{30}$ aliphatic group (alkyl group comprising from 4 to 30 carbon atoms) which may or may not comprise one or more unsaturations and which is preferably linear and unsaturated;

  iv) mention may also be made of the waxes obtained by catalytic hydrogenation of animal or vegetable oils having linear or branched $C_8$-$C_{32}$ fatty chains, for example such as hydrogenated jojoba oil, hydrogenated sunflower oil, hydrogenated castor oil, hydrogenated coconut oil, and also the waxes obtained by hydrogenation of castor oil esterified with cetyl alcohol;

  v) beeswax, synthetic beeswax, polyglycerolated beeswax, carnauba wax, candelilla wax, oxypropylenated lanolin wax, rice bran wax, ouricury wax, esparto grass wax, cork fibre wax, sugar cane wax, Japan wax, sumac wax, montan wax, orange wax, laurel wax, hydrogenated jojoba wax, sunflower wax, lemon wax, olive wax or berry wax.

According to another embodiment, the polar wax may be an alcohol wax. According to the invention, the term *"alcohol wax"* means a wax comprising at least one alcohol function, i.e. comprising at least one free hydroxyl (OH) group. Alcohol waxes that may be mentioned include for example the $C_{30}$-$_{50}$ alcohol wax Performacol® 550 Alcohol sold by the company New Phase Technologies, stearyl alcohol and cetyl alcohol.

It is also possible to use silicone waxes, which may advantageously be substituted polysiloxanes, preferably of low melting point.

The term "silicone wax" is intended to mean an oil comprising at least one silicon atom, and in particular comprising Si-O groups.

Among the commercial silicone waxes of this type, mention may be made in particular of those sold under the names Abilwax 9800, 9801 or 9810 (Goldschmidt), KF910 and KF7002 (Shin-Etsu), or 176-1118-3 and 176-11481 (General Electric).

The silicone waxes that may be used may also be alkyl or alkoxy dimethicones, and also ($C_{20}$-$C_{60}$)alkyl dimethicones, in particular ($C_{30}$-$C_{45}$)alkyl dimethicones, such as the silicone wax sold under the name SF-1642 by the company GE-Bayer Silicones or $C_{30}$-$_{45}$ alkyl dimethylsilyl polypropylsilsesquioxane under the name SW-8005® C30 Resin Wax sold by the company Dow Corning.

In the context of the present invention, particularly advantageous waxes that may be mentioned include polyethylene waxes, jojoba wax, candelilla wax and silicone waxes, in particular candelilla wax.

They may be present in the oily phase in a proportion of from 0.5% to 30% by weight relative to the weight of

the oily phase, for example between 5% and 20% of the oily phase and more particularly from 2% to 15% by weight relative to the weight of the oily phase.

Modified clays

The composition according to the invention may comprise at least one lipophilic clay.
The clays may be natural or synthetic, and they are made lipophilic by treatment with an alkylammonium salt such as a $C_{10}$ to $C_{22}$ ammonium chloride, for example distearyldimethylammonium chloride.
They may be chosen from bentonites, in particular hectorites and montmorillonites, beidellites, saponites, nontronites, sepiolites, biotites, attapulgites, vermiculites and zeolites.
They are preferably chosen from hectorites.
Hectorites modified with a $C_{10}$ to $C_{22}$ ammonium chloride, such as hectorite modified with distearyldimethylammonium chloride, for instance the product sold under the name Bentone 38V® by the company Elementis or bentone gel in isododecane sold under the name Bentone Gel ISD V® (87% isododecane/10% disteardimonium hectorite/3% propylene carbonate) by the company Elementis, are preferably used as lipophilic clays.
Lipophilic clay may especially be present in a content ranging from 0.1% to 30% by weight, in particular from 0.5% to 20% and more particularly from 1% to 18% by weight relative to the total weight of the oily phase.

Silicas

The oily phase of a composition according to the invention may also comprise, as gelling agent, a fumed silica or silica aerogel particles.

a) Fumed silica

Fumed silica which has undergone a hydrophobic surface treatment is most particularly suitable for use in the invention. Specifically, it is possible to chemically modify the surface of the silica, by chemical reaction generating a reduced number of silanol groups present at the surface of the silica. It is possible in particular to replace silanol groups with hydrophobic groups: a hydrophobic silica is then obtained.
The hydrophobic groups may be:

- trimethylsiloxyl groups, which are obtained in particular by treating fumed silica in the presence of hexamethyldisilazane. Silicas thus treated are known as Silica Silylate according to the CTFA ($8^{th}$ edition, 2000). They are sold, for example, under the references Aerosil R812® by the company Degussa and Cab-O-Sil TS-530® by the company Cabot.
- dimethylsilyloxyl or polydimethylsiloxane groups, which are obtained in particular by treating fumed silica in the presence of polydimethylsiloxane or dimethyldichlorosilane. Silicas thus treated are known as Silica dimethyl silylate according to the CTFA (8th edition, 2000). They are sold, for example, under the references Aerosil R972® and Aerosil R974® by the company Degussa, and Cab-O-Sil TS-610® and Cab-O-Sil TS-720® by the company Cabot.

The fumed silicas may be present in a composition according to the present invention in a content of between 0.1% and 40% by weight, more particularly between 1% and 15% by weight and even more particularly between 2% and 10% by weight relative to the total weight of the oily phase.

b) Hydrophobic silica aerogels

The oily phase of a composition according to the invention may also comprise, as gelling agent, at least silica aerogel particles.
Silica aerogels are porous materials obtained by replacing (by drying) the liquid component of a silica gel with air.
They are generally synthesized via a sol-gel process in a liquid medium and then dried, usually by extraction with a supercritical fluid, the one most commonly used being supercritical $CO_2$. This type of drying makes it possible to avoid shrinkage of the pores and of the material. The sol-gel process and the various drying operations are described in detail in Brinker C.J. and Scherer G.W., Sol-Gel Science, New York: Academic Press, 1990.
The hydrophobic silica aerogel particles used in the present invention have a specific surface area per unit mass (SM) ranging from 500 to 1500 $m^2/g$, preferably from 600 to 1200 $m^2/g$ and better still from 600 to 800 $m^2/g$, and a size expressed as the volume-mean diameter (D[0.5]) ranging from 1 to 1500 $\mu m$, better still from 1 to 1000 $\mu m$, preferably from 1 to 100 $\mu m$, in particular from 1 to 30 $\mu m$, more preferably from 5 to 25 $\mu m$, better still from 5 to 20 $\mu m$ and even better still from 5 to 15 $\mu m$.

According to one embodiment, the hydrophobic silica aerogel particles used in the present invention have a size expressed as volume-mean diameter (D[0.5]) ranging from 1 to 30 μm, preferably from 5 to 25 μm, better still from 5 to 20 μm and even better still from 5 to 15 μm.

The specific surface area per unit mass may be determined by the nitrogen absorption method, known as the BET (Brunauer-Emmett-Teller) method, described in the Journal of the American Chemical Society, vol. 60, page 309, February 1938, which corresponds to International Standard ISO 5794/1 (appendix D). The BET specific surface area corresponds to the total specific surface area of the particles under consideration.

The sizes of the silica aerogel particles may be measured by static light scattering using a commercial particle size analyser such as the MasterSizer 2000 machine from Malvern. The data are processed on the basis of the Mie scattering theory. This theory, which is exact for isotropic particles, makes it possible to determine, in the case of non-spherical particles, an "effective" particle diameter. This theory is especially described in the publication by Van de Hulst, H.C., Light Scattering by Small Particles, Chapters 9 and 10, Wiley, New York, 1957.

According to an advantageous embodiment, the hydrophobic silica aerogel particles used in the present invention have a specific surface area per unit of mass (SM) ranging from 600 to 800 $m^2/g$.

The silica aerogel particles used in the present invention may advantageously have a tapped density ρ ranging from 0.02 $g/cm^3$ to 0.10 $g/cm^3$, preferably from 0.03 $g/cm^3$ to 0.08 $g/cm^3$ and in particular ranging from 0.05 $g/cm^3$ to 0.08 $g/cm^3$.

In the context of the present invention, this density, known as the tapped density, may be assessed according to the following protocol:

40 g of powder are poured into a measuring cylinder; the measuring cylinder is then placed on a Stav 2003 machine from Stampf Volumeter; the measuring cylinder is then subjected to a series of 2500 tapping actions (this operation is repeated until the difference in volume between two consecutive tests is less than 2%); the final volume Vf of tapped powder is then measured directly on the measuring cylinder. The tapped density is determined by the ratio m/Vf, in this instance 40/Vf (Vf being expressed in $cm^3$ and m in g).

According to one preferred embodiment, the hydrophobic silica aerogel particles used in the present invention have a specific surface area per unit of volume SV ranging from 5 to 60 $m^2/cm^3$, preferably from 10 to 50 $m^2/cm^3$ and better still from 15 to 40 $m^2/cm^3$.

The specific surface area per unit of volume is given by the relationship: $S_V = S_M \times \rho$; where ρ is the tapped density, expressed in $g/cm^3$, and $S_M$ is the specific surface area per unit of mass, expressed in $m^2/g$, as defined above.

Preferably, the hydrophobic silica aerogel particles according to the invention have an oil-absorbing capacity, measured at the wet point, ranging from 5 to 18 ml/g, preferably from 6 to 15 ml/g and better still from 8 to 12 ml/g.

The absorbing capacity measured at the wet point, noted Wp, corresponds to the amount of oil that needs to be added to 100 g of particles in order to obtain a homogeneous paste.

It is measured according to the wet point method or the method for determining the oil uptake of a powder described in standard NF T 30-022. It corresponds to the amount of oil adsorbed onto the available surface of the powder and/or absorbed by the powder by measurement of the wet point, described below:

An amount m = 2 g of powder is placed on a glass plate, and the oil (isononyl isononanoate) is then added dropwise. After addition of 4 to 5 drops of oil to the powder, mixing is carried out using a spatula, and addition of oil is continued until conglomerates of oil and powder have formed. From this point, the oil is added at the rate of one drop at a time and the mixture is subsequently triturated with the spatula. The addition of oil is stopped when a firm, smooth paste is obtained. This paste must be able to be spread on the glass plate without cracking or forming lumps. The volume Vs (expressed in ml) of oil used is then noted.

The oil uptake corresponds to the ratio Vs/m.

The aerogels used according to the present invention are aerogels of hydrophobic silica, preferably of silylated silica (INCI name: silica silylate).

The term *"hydrophobic silica"* means any silica whose surface is treated with silylating agents, for example with halogenated silanes such as alkylchlorosilanes, siloxanes, in particular dimethylsiloxanes such as hexamethyldisiloxane, or silazanes, so as to functionalize the OH groups with silyl groups Si-Rn, for example trimethylsilyl groups.

As regards the preparation of hydrophobic silica aerogel particles that have been surface-modified by silylation, reference may be made to document US 7 470 725.

Use will preferably be made of hydrophobic silica aerogel particles surface-modified with trimethylsilyl groups, preferably of the INCI name Silica silylate.

As hydrophobic silica aerogels that may be used in the invention, an example that may be mentioned is the aerogel sold under the name VM-2260 or VM-2270 (INCI name: Silica silylate) by the company Dow Corning, the particles of which have a mean size of about 1000 microns and a specific surface area per unit of mass ranging from 600 to 800 $m^2/g$.

Mention may also be made of the aerogels sold by the company Cabot under the references Aerogel TLD 201, Aerogel OGD 201 and Aerogel TLD 203, Enova® Aerogel MT 1100 and Enova Aerogel MT 1200.

Use will preferably be made of the aerogel sold under the name VM-2270 (INCI name: Silica silylate) by the

company Dow Corning, the particles of which have an average size ranging from 5-15 microns and a specific surface area per unit of mass ranging from 600 to 800 $m^2/g$.

Such an aerogel advantageously makes it possible to promote the resistance of the deposit to sebum and to sweat.

Preferably, the hydrophobic silica aerogel particles are present in the composition according to the invention in a solids content ranging from 0.1% to 15% by weight, preferably from 0.2% to 12% by weight relative to the total weight of the oily phase.

## II. Organopolysiloxane elastomer

The organopolysiloxane elastomer that may be used as lipophilic gelling agent has the advantage of giving the composition according to the invention good application properties. It affords a very soft feel and a matt effect after application, which is advantageous especially for application to the skin. It may also allow efficient filling of the hollows present on keratin materials.

The term *"organopolysiloxane elastomer"* or *"silicone elastomer"* means a supple, deformable organopolysiloxane with viscoelastic properties and especially with the consistency of a sponge or a supple sphere. Its modulus of elasticity is such that this material withstands deformation and has a limited ability to extend and to contract. This material is capable of regaining its original shape after stretching.

It is more particularly a crosslinked organopolysiloxane elastomer.

Thus, the organopolysiloxane elastomer may be obtained by crosslinking addition reaction of diorganopolysiloxane containing at least one hydrogen bonded to silicon and of diorganopolysiloxane containing ethylenically unsaturated groups bonded to silicon, especially in the presence of a platinum catalyst; or by dehydrogenation crosslinking condensation reaction between a diorganopolysiloxane comprising hydroxyl end groups and a diorganopolysiloxane containing at least one hydrogen bonded to silicon, especially in the presence of an organotin; or by crosslinking condensation reaction of a diorganopolysiloxane comprising hydroxyl end groups and of a hydrolysable organopolysilane; or by thermal crosslinking of organopolysiloxane, especially in the presence of an organoperoxide catalyst; or by crosslinking of organopolysiloxane via high-energy radiation such as gamma rays, ultraviolet rays or an electron beam.

Preferably, the organopolysiloxane elastomer is obtained by crosslinking addition reaction (A) of diorganopolysiloxane containing at least two hydrogens each bonded to a silicon, and (B) of diorganopolysiloxane containing at least two ethylenically unsaturated groups bonded to silicon, especially in the presence (C) of a platinum catalyst, as described, for instance, in patent application EP-A-295 886.

In particular, the organopolysiloxane elastomer may be obtained by reaction of dimethylpolysiloxane comprising dimethylvinylsiloxy end groups and of methylhydrogenopolysiloxane comprising trimethylsiloxy end groups, in the presence of a platinum catalyst.

Compound (A) is the base reagent for the formation of organopolysiloxane elastomer, and the crosslinking is performed by addition reaction of compound (A) with compound (B) in the presence of the catalyst (C).

Compound (A) is in particular an organopolysiloxane containing at least two hydrogen atoms bonded to different silicon atoms in each molecule.

Compound (A) may have any molecular structure, in particular a linear-chain or branched-chain structure or a cyclic structure.

Compound (A) may have a viscosity at 25°C ranging from 1 to 50 000 centistokes, especially so as to be miscible with compound (B).

The organic groups bonded to the silicon atoms of compound (A) may be alkyl groups such as methyl, ethyl, propyl, butyl, octyl; substituted alkyl groups such as 2-phenylethyl, 2-phenylpropyl or 3,3,3-trifluoropropyl; aryl groups such as phenyl, tolyl, xylyl; substituted aryl groups such as phenylethyl; and substituted monovalent hydrocarbon-based groups such as an epoxy group, a carboxylate ester group or a mercapto group.

Compound (A) can thus be chosen from methylhydrogenopolysiloxanes comprising trimethylsiloxy end groups, dimethylsiloxane-methylhydrosiloxane copolymers comprising trimethylsiloxy end groups, and dimethylsiloxane-methylhydrosiloxane cyclic copolymers.

Compound (B) is advantageously a diorganopolysiloxane containing at least two lower alkenyl groups (for example $C_2$-$C_4$); the lower alkenyl group may be chosen from vinyl, allyl and propenyl groups. These lower alkenyl groups may be located at any position on the organopolysiloxane molecule but are preferably located at the ends of the organopolysiloxane molecule. The organopolysiloxane (B) may have a branched-chain, linear-chain, cyclic or network structure but the linear-chain structure is preferred. Compound (B) may have a viscosity ranging from the liquid state to the gum state. Preferably, compound (B) has a viscosity of at least 100 centistokes at 25°C.

Besides the abovementioned alkenyl groups, the other organic groups bonded to the silicon atoms in compound (B) may be alkyl groups such as methyl, ethyl, propyl, butyl or octyl; substituted alkyl groups such as 2-phenylethyl, 2-phenylpropyl or 3,3,3-trifluoropropyl; aryl groups such as phenyl, tolyl or xylyl; substituted aryl groups such as phenylethyl;

and substituted monovalent hydrocarbon-based groups such as an epoxy group, a carboxylate ester group or a mercapto group.

The organopolysiloxanes (B) can be chosen from methylvinylpolysiloxanes, methylvinylsiloxane-dimethylsiloxane copolymers, dimethylpolysiloxanes comprising dimethylvinylsiloxy end groups, dimethylsiloxane-methylphenylsiloxane copolymers comprising dimethylvinylsiloxy end groups, dimethylsiloxane-diphenylsiloxane-methylvinylsiloxane copolymers comprising dimethylvinylsiloxy end groups, dimethylsiloxane-methylvinylsiloxane copolymers comprising trimethylsiloxy end groups, dimethylsiloxane-methylphenylsiloxane-methylvinylsiloxane copolymers comprising trimethylsiloxy end groups, methyl(3,3,3-trifluoropropyl)polysiloxanes comprising dimethylvinylsiloxy end groups, and dimethylsiloxane-methyl(3,3,3-trifluoropropyl)siloxane copolymers comprising dimethylvinylsiloxy end groups.

In particular, the organopolysiloxane elastomer can be obtained by reaction of dimethylpolysiloxane comprising dimethylvinylsiloxy end groups and of methylhydrogenopolysiloxane comprising trimethylsiloxy end groups, in the presence of a platinum catalyst.

Advantageously, the sum of the number of ethylenic groups per molecule of compound (B) and of the number of hydrogen atoms bonded to silicon atoms per molecule of compound (A) is at least 5.

It is advantageous for compound (A) to be added in an amount such that the molecular ratio of the total amount of hydrogen atoms bonded to silicon atoms in compound (A) to the total amount of all the ethylenically unsaturated groups in compound (B) is within the range from 1.5/1 to 20/1.

Compound (C) is the catalyst for the crosslinking reaction, and is especially chloroplatinic acid, chloroplatinic acid-olefin complexes, chloroplatinic acid-alkenylsiloxane complexes, chloroplatinic acid-diketone complexes, platinum black and platinum on a support.

Catalyst (C) is preferably added in an amount of from 0.1 to 1000 parts by weight and better still from 1 to 100 parts by weight, as clean platinum metal, per 1000 parts by weight of the total amount of compounds (A) and (B).

The elastomer is advantageously a non-emulsifying elastomer.

The term "*non-emulsifying*" defines organopolysiloxane elastomers not containing any hydrophilic chains, and in particular not containing any polyoxyalkylene units (especially polyoxyethylene or polyoxypropylene) or any polyglyceryl units. Thus, according to one particular mode of the invention, the composition comprises an organopolysiloxane elastomer free of polyoxyalkylene units and of polyglyceryl units.

In particular, the silicone elastomer used in the present invention is chosen from Dimethicone Crosspolymer (INCI name), Vinyl Dimethicone Crosspolymer (INCI name), Dimethicone/Vinyl Dimethicone Crosspolymer (INCI name), Dimethicone Crosspolymer-3 (INCI name).

The organopolysiloxane elastomer particles may be conveyed in the form of a gel formed from an elastomeric organopolysiloxane included in at least one hydrocarbon-based oil and/or one silicone oil. In these gels, the organopolysiloxane particles are often non-spherical particles.

Non-emulsifying elastomers are described especially in patents EP 242 219, EP 285 886 and EP 765 656 and in patent application JP-A-61-194009.

The silicone elastomer is generally in the form of a gel, a paste or a powder, but advantageously in the form of a gel in which the silicone elastomer is dispersed in a linear silicone oil (dimethicone) or cyclic silicone oil (e.g.: cyclopentasiloxane), advantageously in a linear silicone oil.

Non-emulsifying elastomers that may be used more particularly include those sold under the names KSG-6, KSG-15, KSG-16, KSG-18, KSG-41, KSG-42, KSG-43 and KSG-44 by the company Shin-Etsu, DC9040, DC9041 and DC9042 by the company Dow Corning, and SFE 839 by the company General Electric.

According to a particular mode, use is made of a gel of silicone elastomer dispersed in a silicone oil chosen from a non-exhaustive list comprising cyclopentadimethylsiloxane, dimethicones, dimethylsiloxanes, methyl trimethicone, phenyl methicone, phenyl dimethicone, phenyl trimethicone and cyclomethicone, preferably a linear silicone oil chosen from polydimethylsiloxanes (PDMS) or dimethicones with a viscosity at 25°C ranging from 1 to 500 cSt, optionally modified with optionally fluorinated aliphatic groups, or with functional groups such as hydroxyl, thiol and/or amine groups.

Mention may be made especially of the compounds having the following INCI names:

- dimethicone/vinyl dimethicone crosspolymer, such as USG-105 and USG-107A from the company Shin-Etsu; DC9506 and DC9701 from the company Dow Corning;
- dimethicone/vinyl dimethicone crosspolymer (and) dimethicone, such as KSG-6 and KSG-16 from the company Shin-Etsu;
- dimethicone/vinyl dimethicone crosspolymer (and) cyclopentasiloxane, such as KSG-15;
- cyclopentasiloxane (and) dimethicone crosspolymer, such as DC9040, DC9045 and DC5930 from the company Dow Corning;
- dimethicone (and) dimethicone crosspolymer, such as DC9041 from the company Dow Corning.
- dimethicone (and) dimethicone crosspolymer, such as Dow Corning EL-9240® Silicone Elastomer Blend from the company Dow Corning (mixture of polydimethylsiloxane crosslinked with hexadiene/polydimethylsiloxane (2 cSt));

- $C_{4-24}$ alkyl dimethicone/divinyl dimethicone crosspolymer, such as NuLastic Silk MA from the company Alzo.

As examples of silicone elastomers dispersed in a linear silicone oil that may advantageously be used according to the invention, mention may especially be made of the following references:

- dimethicone/vinyl dimethicone crosspolymer (and) dimethicone, such as KSG-6 and KSG-16 from the company Shin-Etsu;
- dimethicone (and) dimethicone crosspolymer, such as DC9041 from the company Dow Corning; and
- dimethicone (and) dimethicone crosspolymer, such as Dow Corning EL-9240® Silicone Elastomer Blend from the company Dow Corning (mixture of polydimethylsiloxane crosslinked with hexadiene/polydimethylsiloxane (2 cSt)).

According to a preferred embodiment, the composition according to the invention comprises at least one crosslinked silicone elastomer having the INCI name "dimethicone crosspolymer" or "dimethicone (and) dimethicone crosspolymer", with, preferably, a dimethicone having a viscosity ranging from 1 to 100 cSt, in particular from 1 to 10 cSt at 25°C, such as the mixture of polydimethylsiloxane crosslinked with hexadiene/polydimethylsiloxane (5 cSt) sold under the name DC 9041 by the company Dow Corning or the mixture of polydimethylsiloxane crosslinked with hexadiene/polydimethylsiloxane (2 cSt) sold under the name EL-9240® by the company Dow Corning.
According to a particularly preferred embodiment, the composition according to the invention comprises at least one crosslinked silicone elastomer having the INCI name "dimethicone (and) dimethicone crosspolymer", preferably with a dimethicone having a viscosity ranging from 1 to 100 cSt, in particular from 1 to 10 cSt at 25°C, such as the mixture of polydimethylsiloxane crosslinked with hexadiene/polydimethylsiloxane (5 cSt) sold under the name DC 9041 by the company Dow Corning.
The organopolysiloxane elastomer particles may also be used in powder form: mention may be made especially of the powders sold under the names Dow Corning 9505 Powder and Dow Corning 9506 Powder by the company Dow Corning, these powders having the INCI name: dimethicone/vinyl dimethicone crosspolymer.
The organopolysiloxane powder may also be coated with silsesquioxane resin, as described, for example, in patent US 5 538 793. Such elastomeric powders are sold under the names KSP-100, KSP-101, KSP-102, KSP-103, KSP-104 and KSP-105 by the company Shin-Etsu, and have the INCI name: vinyl dimethicone/methicone silsesquioxane crosspolymer.
As examples of organopolysiloxane powders coated with silsesquioxane resin that may advantageously be used according to the invention, mention may be made especially of the reference KSP-100 from the company Shin-Etsu.
As preferred lipophilic gelling agent of organopolysiloxane elastomer type, mention may be made especially of crosslinked organopolysiloxane elastomers chosen from Dimethicone Crosspolymer (INCI name), Dimethicone (and) Dimethicone Crosspolymer (INCI name), Vinyl Dimethicone Crosspolymer (INCI name), Dimethicone/Vinyl Dimethicone Crosspolymer (INCI name), Dimethicone Crosspolymer-3 (INCI name), and in particular Dimethicone Crosspolymer (INCI name).
The organopolysiloxane elastomer may be present in a composition of the present invention in a content of between 0.1% and 80% by weight of solids, especially between 1% and 75% and more particularly between 2% and 70% by weight relative to the total weight of the oily phase.

## III. Semi-crystalline polymers

The composition according to the invention may comprise at least one semi-crystalline polymer. Preferably, the semi-crystalline polymer has an organic structure, and a melting point of greater than or equal to 30°C.
For the purposes of the invention, the term "semi-crystalline polymer" is intended to mean polymers comprising a crystallizable portion and an amorphous portion and having a first-order reversible change of phase temperature, in particular of melting point (solid-liquid transition). The crystallizable part is either a side chain (or pendent chain) or a block in the backbone.
When the crystallizable portion of the semi-crystalline polymer is a block of the polymer backbone, this crystallizable block has a chemical nature different than that of the amorphous blocks; in this case, the semi-crystalline polymer is a block copolymer, for example of the diblock, triblock or multiblock type. When the crystallizable part is a chain that is pendent on the backbone, the semi-crystalline polymer may be a homopolymer or a copolymer.
The melting point of the semi-crystalline polymer is preferably less than 150°C.
The melting point of the semi-crystalline polymer is preferably greater than or equal to 30°C and less than 100°C. More preferably, the melting point of the semi-crystalline polymer is greater than or equal to 30°C and less than 70°C.
The semi-crystalline polymer(s) according to the invention are solid at room temperature (25°C) and atmospheric pressure (760 mmHg), with a melting point of greater than or equal to 30°C. The melting point values correspond to the

melting point measured using a differential scanning calorimeter (DSC), such as the calorimeter sold under the name DSC 30 by the company Mettler, with a temperature rise of 5 or 10°C per minute (the melting point under consideration is the point corresponding to the temperature of the most endothermic peak in the thermogram).

The semi-crystalline polymer(s) according to the invention preferably have a melting point that is higher than the temperature of the keratinous support intended to receive said composition, in particular the skin, the lips or the eyebrows.

According to the invention, the semi-crystalline polymers are advantageously soluble in the fatty phase, especially to at least 1% by weight, at a temperature that is higher than their melting point. Besides the crystallizable chains or blocks, the blocks of the polymers are amorphous.

For the purposes of the invention, the term "crystallizable chain or block" is intended to mean a chain or block which, if it were alone, would change from the amorphous state to the crystalline state reversibly, depending on whether the temperature is above or below the melting point. For the purposes of the invention, a chain is a group of atoms, which are pendent or lateral relative to the polymer backbone. A "block" is a group of atoms belonging to the backbone, this group constituting one of the repeating units of the polymer.

Preferably, the polymer backbone of the semi-crystalline polymers is soluble in the fatty phase at a temperature above their melting point.

Preferably, the crystallizable blocks or chains of the semi-crystalline polymers represent at least 30% of the total weight of each polymer and better still at least 40%. The semi-crystalline polymers containing crystallizable side chains are homopolymers or copolymers. The semi-crystalline polymers of the invention containing crystallizable blocks are block or multiblock copolymers. They may be obtained via polymerization of a monomer containing reactive double bonds (or ethylenic bonds) or via polycondensation. When the polymers of the invention are polymers containing crystallizable side chains, these side chains are advantageously in random or statistical form.

Preferably, the semi-crystalline polymers of the invention are of synthetic origin.

According to a preferred embodiment, the semi-crystalline polymer is chosen from:

- homopolymers and copolymers comprising units resulting from the polymerization of one or more monomers bearing crystallizable hydrophobic side chain(s),
- polymers bearing in the backbone at least one crystallizable block,
- polycondensates of aliphatic or aromatic or aliphatic/aromatic polyester type,
- copolymers of ethylene and propylene prepared via metallocene catalysis, and
- acrylate/silicone copolymers.

The semi-crystalline polymers that may be used in the invention may be chosen in particular from:

- block copolymers of polyolefins of controlled crystallization, whose monomers are described in EP 0 951 897,
- polycondensates, in particular of aliphatic or aromatic or aliphatic/aromatic polyester type,
- copolymers of ethylene and propylene prepared via metallocene catalysis,
- homopolymers or copolymers bearing at least one crystallizable side chain and homopolymers or copolymers bearing in the backbone at least one crystallizable block, such as those described in document US 5 156 911, such as the ($C_{10}$-$C_{30}$)alkyl polyacrylates corresponding to the Intelimer® products from the company Landec described in the brochure *Intelimer® Polymers,* Landec IP22 (Rev. 4-97), for example the product Intelimer® IPA 13-1 from the company Landec, which is a polystearyl acrylate with a molecular weight of about 145 000 and a melting point of 49°C,
- homopolymers or copolymers bearing at least one crystallizable side chain, in particular containing fluoro group(s), as described in document WO 01/19333,
- acrylate/silicone copolymers, such as copolymers of acrylic acid and of stearyl acrylate bearing polydimethylsiloxane grafts, copolymers of stearyl methacrylate bearing polydimethylsiloxane grafts, copolymers of acrylic acid and of stearyl methacrylate bearing polydimethylsiloxane grafts, copolymers of methyl methacrylate, butyl methacrylate, 2-ethylhexyl acrylate and stearyl methacrylate bearing polydimethylsiloxane grafts. Mention may be made in particular of the copolymers sold by the company Shin-Etsu under the names KP-561 (CTFA name: acrylates/dimethicone), KP-541 (CTFA name: acrylates/dimethicone and isopropyl alcohol), KP-545 (CTFA name: acrylates/dimethicone and cyclopentasiloxane),
- and mixtures thereof.

Preferably, the amount of semi-crystalline polymer(s), preferably chosen from semi-crystalline polymers bearing crystallizable side chains, represents from 0.1% to 30% by weight of solids relative to the total weight of the oily phase, for example from 0.5% to 25% by weight, better still from 5% to 20% or even from 5% to 17% by weight, relative to the total weight of the oily phase.

## IV. Dextrin esters

The composition according to the invention may comprise as lipophilic gelling agent at least one dextrin ester.

In particular, the composition preferably comprises at least one preferably $C_{12}$ to $C_{24}$ and in particular $C_{14}$ to $C_{18}$ fatty acid ester of dextrin, or mixtures thereof.

Preferably, the dextrin ester is an ester of dextrin and of a $C_{12}$-$C_{18}$ and in particular $C_{14}$-$C_{18}$ fatty acid.

Preferably, the dextrin ester is chosen from dextrin myristate and/or dextrin palmitate, and mixtures thereof.

According to a particular embodiment, the dextrin ester is dextrin myristate, such as the product sold especially under the name Rheopearl MKL-2 by the company Chiba Flour Milling.

According to a preferred embodiment, the dextrin ester is dextrin palmitate. This product may be chosen, for example, from those sold under the names Rheopearl TL®, Rheopearl KL® and Rheopearl® KL2 by the company Chiba Flour Milling.

In a particularly preferred manner, the oily phase of a composition according to the invention may comprise from 0.1% to 30% by weight, preferably from 2% to 25% and preferably from 7.5% to 17% by weight of dextrin ester(s) relative to the total weight of the oily phase.

In a particularly preferred manner, the composition according to the invention may comprise between 0.1% and 10% by weight and preferably between 0.5% and 5% by weight of dextrin palmitate relative to the total weight of the oily phase. The dextrin palmitate may especially be the product sold under the names Rheopearl TL®, Rheopearl KL® or Rheopearl® KL2 by the company Chiba Flour Milling.

## V. Hydrogen bonding polymers

As representatives of hydrogen bonding polymers that are suitable for use in the invention, mention may be made most particularly of polyamides and in particular hydrocarbon-based polyamides and silicone polyamides.

### Polyamides

The oily phase of a composition according to the invention may comprise at least one polyamide chosen from hydrocarbon-based polyamides and silicone polyamides, and mixtures thereof.

Preferably, the total content of polyamide(s) is between 0.1% and 30% by weight expressed as solids, preferably between 0.1% and 20% by weight and preferably between 0.5% and 10% by weight relative to the total weight of the oily phase.

For the purposes of the invention, the term *"polyamide"* means a compound containing at least 2 repeating amide units, preferably at least 3 repeating amide units and better still 10 repeating amide units.

### a) Hydrocarbon-based polyamide

The term *"hydrocarbon-based polyamide"* means a polyamide formed essentially of, indeed even consisting of, carbon and hydrogen atoms, and optionally of oxygen or nitrogen atoms, and not comprising any silicon or fluorine atoms. It may contain alcohol, ester, ether, carboxylic acid, amine and/or amide groups.

For the purposes of the invention, the term *"functionalized chain"* means an alkyl chain comprising one or more functional groups or reagents chosen especially from hydroxyl, ether, ester, oxyalkylene and polyoxyalkylene groups.

Advantageously, this polyamide of the composition according to the invention has a weight-average molecular mass of less than 100 000 g/mol especially ranging from 1000 to 100 000 g/mol, in particular less than 50 000 g/mol especially ranging from 1000 to 50 000 g/mol and more particularly ranging from 1000 to 30 000 g/mol, preferably from 2000 to 20 000 g/mol and better still from 2000 to 10 000 g/mol.

This polyamide is insoluble in water, especially at 25°C.

According to a first embodiment of the invention, the polyamide used is a polyamide of formula (I):

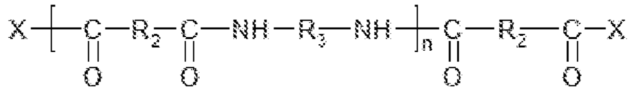

(I)

in which X represents a group $-N(R_1)_2$ or a group $-OR_1$ in which $R_1$ is a linear or branched $C_8$ to $C_{22}$, alkyl radical which may be identical or different, $R_2$ is a $C_{28}$-$C_{42}$ diacid dimer residue, $R_3$ is an ethylenediamine radical and n is between 2 and 5;

and mixtures thereof.

According to a particular mode, the polyamide used is an amide-terminated polyamide of formula (Ia):

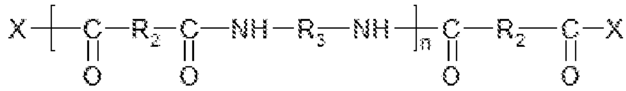

(Ia)

in which X represents a group $-N(R_1)_2$ in which $R_1$ is a linear or branched $C_8$ to $C_{22}$, alkyl radical which may be identical or different, $R_2$ is a $C_{28}$-$C_{42}$ diacid dimer residue, $R_3$ is an ethylenediamine radical and n is between 2 and 5; and mixtures thereof.

The oily phase of a composition according to the invention may also comprise, additionally in this case, at least one additional polyamide of formula (Ib):

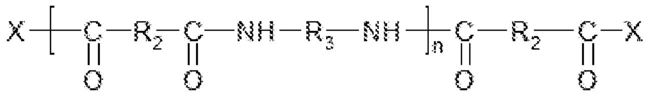

(Ib)

in which X represents a group $-OR_1$ in which $R_1$ is a linear or branched $C_8$ to $C_{22}$ and preferably $C_{16}$ to $C_{22}$, alkyl radical which may be identical or different, $R_2$ is a $C_{28}$-$C_{42}$ diacid dimer residue, $R_3$ is an ethylenediamine radical and n is between 2 and 5, such as the commercial products sold by the company Arizona Chemical under the names Uniclear 80 and Uniclear 100 or Uniclear 80 V, Uniclear 100 V and Uniclear 100 VG, the INCI name of which is Ethylenediamine/stearyl dimer dilinoleate copolymer.

b) Silicone polyamide

The silicone polyamides are preferably solid at room temperature (25°C) and atmospheric pressure (760 mmHg).

The silicone polyamides may preferentially be polymers comprising at least one unit of formula (III) or (IV):

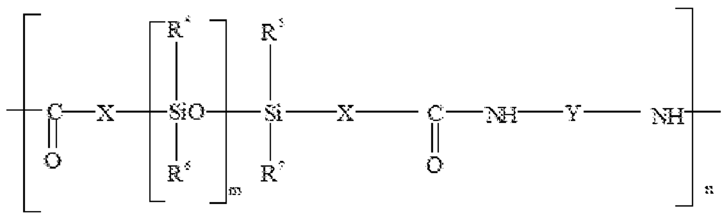

(III)

or

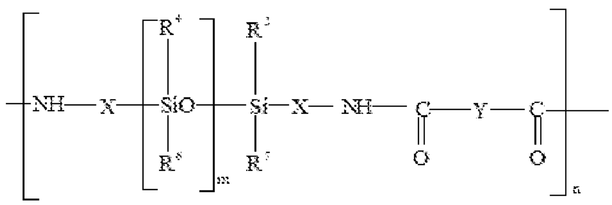

(IV)

in which:

- $R^4$, $R^5$, $R^6$ and $R^7$, which may be identical or different, represent a group chosen from:
    - saturated or unsaturated, $C_1$ to $C_{40}$ linear, branched or cyclic hydrocarbon-based groups, which may contain in their chain one or more oxygen, sulfur and/or nitrogen atoms, and which may be partially or totally substituted with fluorine atoms,
    - $C_6$ to $C_{10}$ aryl groups, optionally substituted with one or more $C_1$ to $C_4$ alkyl groups,

- polyorganosiloxane chains possibly containing one or more oxygen, sulfur and/or nitrogen atoms,

• the groups X, which may be identical or different, represent a linear or branched $C_1$ to $C_{30}$ alkylenediyl group, possibly containing in its chain one or more oxygen and/or nitrogen atoms,
• Y is a saturated or unsaturated $C_1$ to $C_{50}$ linear or branched alkylene, arylene, cycloalkylene, alkylarylene or arylalkylene divalent group, which may comprise one or more oxygen, sulfur and/or nitrogen atoms, and/or may bear as substituent one of the following atoms or groups of atoms: fluorine, hydroxyl, $C_3$ to $C_8$ cycloalkyl, $C_1$ to $C_{40}$ alkyl, $C_5$ to $C_{10}$ aryl, phenyl optionally substituted with one to three $C_1$ to $C_3$ alkyl, $C_1$ to $C_3$ hydroxyalkyl and $C_1$ to $C_6$ aminoalkyl groups, or
Y represents a group corresponding to the formula:

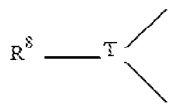

in which

- T represents a linear or branched, saturated or unsaturated, $C_3$ to $C_{24}$ trivalent or tetravalent hydrocarbon-based group optionally substituted with a polyorganosiloxane chain, and possibly containing one or more atoms chosen from O, N and S, or T represents a trivalent atom chosen from N, P and Al, and
- $R^8$ represents a linear or branched $C_1$ to $C_{50}$ alkyl group or a polyorganosiloxane chain, possibly comprising one or more ester, amide, urethane, thiocarbamate, urea, thiourea and/or sulfonamide groups, which may possibly be linked to another chain of the polymer,
• n is an integer ranging from 2 to 500 and preferably from 2 to 200, and m is an integer ranging from 1 to 1000, preferably from 1 to 700 and even better still from 6 to 200.

According to a particular mode, the silicone polyamide comprises at least one unit of formula (III) in which m ranges from 50 to 200, in particular from 75 to 150 and is preferably about 100.
More preferably, $R^4$, $R^5$, $R^6$ and $R^7$ independently represent a linear or branched $C_1$ to $C_{40}$ alkyl group, preferably a group $CH_3$, $C_2H_5$, n-$C_3H_7$ or an isopropyl group in formula (III).
As an example of silicone polymers that may be used, mention may be made of one of the silicone polyamides obtained in accordance with Examples 1 to 3 of document US 5 981 680.
Mention may be made of the compounds sold by the company Dow Corning under the names DC 2-8179 (DP 100) and DC 2-8178 (DP 15), the INCI name of which is Nylon-611/dimethicone copolymer, i.e. Nylon-611/dimethicone copolymers. The silicone polymers and/or copolymers advantageously have a temperature of transition from the solid state to the liquid state ranging from 45°C to 190°C. Preferably, they have a temperature of transition from the solid state to the liquid state ranging from 70 to 130°C and better still from 80°C to 105°C.
Preferably, the total content of polyamide(s) and/or silicone polyamide(s) is between 0.5% and 25% by weight of solids, in particular from 2% to 20% by weight and preferably between 2% and 17% by weight relative to the total weight of the oily phase.
Advantageously, the hydrogen bonding polymer is chosen from the ethylenediamine/stearyl dimer dilinoleate copolymer and Nylon-611/dimethicone copolymers.

## VI. Hydrocarbon-based block copolymers

As representatives of lipophilic gelling agents, mention may also be made of other polymeric gelling agents, namely hydrocarbon-based block copolymers, also known as block copolymers.
The polymeric gelling agent is capable of thickening or gelling the hydrocarbon-based phase of the composition.
The term "amorphous polymer" means a polymer that does not have a crystalline form.
The polymeric gelling agent is preferably also film-forming, i.e. it is capable of forming a film when it is applied to the skin and/or the lips.
The hydrocarbon-based block copolymer may especially be a diblock, triblock, multiblock, radial or star copolymer, or mixtures thereof.
Such hydrocarbon-based block copolymers are described in patent application US-A-2002/005 562 and in patent US-A-5 221 534.
The copolymer may have at least one block whose glass transition temperature is preferably less than 20°C, preferably less than or equal to 0°C, preferably less than or equal to -20°C, more preferably less than or equal to -40°C.

The glass transition temperature of said block may be between -150°C and 20°C and especially between -100°C and 0°C.

The hydrocarbon-based block copolymer present in the composition according to the invention is an amorphous copolymer formed by polymerization of an olefin. The olefin may especially be an elastomeric ethylenically unsaturated monomer.

Examples of olefins that may be mentioned include ethylenic carbide monomers, especially containing one or two ethylenic unsaturations, containing from 2 to 5 carbon atoms, such as ethylene, propylene, butadiene, isoprene or pentadiene.

Advantageously, the hydrocarbon-based block copolymer is an amorphous block copolymer of styrene and of olefin.

Block copolymers comprising at least one styrene block and at least one block comprising units chosen from butadiene, ethylene, propylene, butylene and isoprene or a mixture thereof are especially preferred.

According to a preferred embodiment, the hydrocarbon-based block copolymer is hydrogenated to reduce the residual ethylenic unsaturations after polymerization of the monomers.

In particular, the hydrocarbon-based block copolymer is an optionally hydrogenated copolymer bearing styrene blocks and ethylene/$C_3$-$C_4$ alkylene blocks.

According to a preferred embodiment, the composition according to the invention comprises at least one diblock copolymer, which is preferably hydrogenated, preferably chosen from styrene-ethylene/propylene copolymers, styrene-ethylene/butadiene copolymers and styrene-ethylene/butylene copolymers. Diblock polymers are sold especially under the name Kraton® G1701E by the company Kraton Polymers.

Advantageously, a diblock copolymer such as those described previously is used as polymeric gelling agent, in particular a diblock copolymer of styrene-ethylene/propylene or a diblock mixture, as described previously.

Thus, according to a preferred embodiment variant, a composition according to the invention comprises as lipophilic gelling agent at least one hydrocarbon-based block copolymer, preferably an optionally hydrogenated copolymer, bearing styrene blocks and bearing ethylene/$C_3$-$C_4$ alkylene blocks, even more preferentially a diblock copolymer, which is preferably hydrogenated, such as a styrene-ethylene/propylene copolymer or a styrene-ethylene/butadiene copolymer.

The hydrocarbon-based block copolymer (or the mixture of hydrocarbon-based block copolymers) may be present in a content ranging from 0.1% to 15% by weight, preferably ranging from 0.1% to 10% by weight, more preferentially ranging from 0.5% to 5% by weight and better still ranging from 0.5% to 3% by weight relative to the total weight of the composition.

According to an advantageous variant, a composition according to the invention comprises a lipophilic gelling agent chosen from particulate gelling agents, organopolysiloxane elastomers, semi-crystalline polymers, dextrin esters and hydrogen bonding polymers, hydrocarbon-based block copolymers, and mixtures thereof, and in particular at least one organopolysiloxane elastomer.

Preferably, a composition according to the invention comprises, as lipophilic gelling agent, at least one silicone elastomer, in combination with at least one other lipophilic gelling agent.

## HYDROPHILIC GELLING AGENT/LIPOPHILIC GELLING AGENT SYSTEM

As preferred synthetic polymeric hydrophilic gelling agents, mention may be made more particularly of 2-acrylamido-2-methylpropanesulfonic acid polymers, for instance AMPS, such as the ammonium 2-acrylamido-2-methylpropanesulfonate acid polymer sold under the trade name Hostacerin AMPS® by the company Clariant, and 2-acrylamido-2-methylpropanesulfonic acid copolymers and in particular copolymers of AMPS® and of hydroxyethyl acrylate, for instance the AMPS®/hydroxyethyl acrylate copolymer such as that used in the commercial product sold under the name Simulgel NS® by the company SEPPIC (CTFA name: Hydroxyethyl acrylate/Sodium acryloyldimethyltaurate copolymer (and) Squalane (and) Polysorbate 60), or such as the product sold under the name Sodium acrylamido-2-methylpropanesulfonate/Hydroxyethyl acrylate copolymer, such as the commercial product Sepinov EMT 10 (INCI name: Hydroxyethyl acrylate/Sodium acryloyldimethyltaurate copolymer).

As preferred lipophilic gelling agents, mention may be made of gelling agents of organopolysiloxane elastomer type, and more particularly organopolysiloxane elastomers chosen from Dimethicone Crosspolymer (INCI name), Dimethicone (and) Dimethicone Crosspolymer (INCI name), Vinyl Dimethicone Crosspolymer (INCI name), Dimethicone/Vinyl Dimethicone Crosspolymer (INCI name), Dimethicone Crosspolymer-3 (INCI name), and in particular Dimethicone Crosspolymer (INCI name) and Dimethicone (and) Dimethicone Crosspolymer (INCI name).

According to a preferred mode, as preferred lipophilic gelling agents, mention may be made more particularly of gels of silicone elastomer dispersed in a silicone oil.

Thus, according to a particular mode, use is made of a gel of silicone elastomer dispersed in a silicone oil chosen from a non-exhaustive list comprising cyclopentadimethylsiloxane, dimethicones, dimethylsiloxanes, methyl trimethicone, phenyl methicone, phenyl dimethicone, phenyl trimethicone and cyclomethicone, preferably a linear silicone

oil chosen from polydimethylsiloxanes (PDMS) or dimethicones with a viscosity at 25°C ranging from 1 to 500 cSt at 25°C, especially the following references:

- dimethicone/vinyl dimethicone crosspolymer (and) dimethicone, such as KSG-6 and KSG-16 from the company Shin-Etsu;
- dimethicone (and) dimethicone crosspolymer, such as DC9041 from the company Dow Corning; and
- dimethicone (and) dimethicone crosspolymer, such as Dow Corning EL-9240® Silicone Elastomer Blend from the company Dow Corning.

According to a particularly preferred embodiment, the composition according to the invention comprises as lipophilic gelling agent at least one crosslinked silicone elastomer having the INCI name "dimethicone (and) dimethicone crosspolymer", with, preferably, a dimethicone having a viscosity ranging from 1 to 100 cSt, in particular from 1 to 10 cSt at 25°C, such as the mixture of polydimethylsiloxane with hexadiene/polydimethylsiloxane (5 cSt) sold under the name DC 9041 by Dow Corning and the mixture of polydimethylsiloxane with hexadiene/polydimethylsiloxane (2 cSt) sold under the name EL-9240® Silicone Elastomer Blend by Dow Corning.

As non-limiting illustrations of hydrophilic gelling agent/lipophilic gelling agent systems that are most particularly suitable for use in the invention, mention may be made especially of the polymer or copolymer system of 2-acrylamido-2-methylpropanesulfonic acid/organopolysiloxane elastomer.

Thus, a composition according to the invention may advantageously comprise as hydrophilic gelling agent/lipophilic gelling agent systems, a copolymer(s) system of 2-acrylamido-2-methylpropanesulfonic acid and of hydroxyethyl acrylate/organopolysiloxane elastomer(s).

Preferably, a composition according to the invention may comprise as hydrophilic gelling agent/lipophilic gelling agent system, a copolymer(s) system of 2-acrylamido-2-methylpropanesulfonic acid and of hydroxyethyl acrylate/gel(s) of silicone elastomer dispersed in a silicone oil.

## AQUEOUS PHASE

The aqueous phase of a composition according to the invention comprises water and optionally a water-soluble solvent.

In the present invention, the term "water-soluble solvent" denotes a compound that is liquid at room temperature and water-miscible (miscibility with water of greater than 50% by weight at 25°C and atmospheric pressure).

The water-soluble solvents that may be used in the composition of the invention may also be volatile.

Among the water-soluble solvents that may be used in the composition in accordance with the invention, mention may be made especially of lower monoalcohols containing from 1 to 5 carbon atoms such as ethanol and isopropanol, glycols containing from 2 to 8 carbon atoms such as ethylene glycol, propylene glycol, 1,3-butylene glycol and dipropylene glycol, $C_3$ and $C_4$ ketones and $C_2$-$C_4$ aldehydes.

The aqueous phase (water and optionally the water-miscible solvent) may be present in the composition in a content ranging from 5% to 95%, better still from 30% to 80% by weight and preferably from 40% to 75% by weight relative to the total weight of said composition.

According to another embodiment variant, the aqueous phase of a composition according to the invention may comprise at least one $C_2$-$C_{32}$ polyol.

For the purposes of the present invention, the term "*polyol*" should be understood as meaning any organic molecule comprising at least two free hydroxyl groups.

Preferably, a polyol in accordance with the present invention is present in liquid form at room temperature.

A polyol suitable for the invention can be a compound of saturated or unsaturated and linear, branched or cyclic alkyl type carrying, on the alkyl chain, at least two -OH functional groups, in particular at least three -OH functional groups and more particularly at least four -OH functional groups.

The polyols advantageously suitable for the formulation of a composition according to the present invention are those exhibiting in particular from 2 to 32 carbon atoms and preferably from 3 to 16 carbon atoms.

Advantageously, the polyol may be chosen, for example, from ethylene glycol, pentaerythritol, trimethylolpropane, propylene glycol, 1,3-propanediol, butylene glycol, isoprene glycol, pentylene glycol, hexylene glycol, glycerol, polyglycerols such as glycerol oligomers, for instance diglycerol, and polyethylene glycols, and mixtures thereof.

According to a preferred embodiment of the invention, said polyol is chosen from ethylene glycol, pentaerythritol, trimethylolpropane, propylene glycol, glycerol, polyglycerols, polyethylene glycols and mixtures thereof.

According to a particular mode, the composition of the invention may comprise at least propylene glycol.

According to another particular mode, the composition of the invention may comprise at least glycerol.

## OILY PHASE

For the purposes of the invention, an oily phase comprises at least one oil.
The term "*oil*" means any fatty substance that is in liquid form at room temperature and atmospheric pressure.
An oily phase that is suitable for preparing the compositions, especially cosmetic compositions according to the invention, may comprise hydrocarbon-based oils, silicone oils, fluoro oils or non-fluoro oils, or mixtures thereof.
The oils may be volatile or non-volatile.
They may be of animal, plant, mineral or synthetic origin. According to one embodiment variant, oils of silicone origin are preferred.
For the purposes of the present invention, the term "non-volatile oil" means an oil with a vapour pressure of less than 0.13 Pa.
For the purposes of the present invention, the term "silicone oil" means an oil comprising at least one silicon atom, and in particular at least one Si-O group.
The term *"fluoro oil"* means an oil comprising at least one fluorine atom.
The term *"hydrocarbon-based oil"* means an oil mainly containing hydrogen and carbon atoms.
The oils may optionally comprise oxygen, nitrogen, sulfur and/or phosphorus atoms, for example in the form of hydroxyl or acid radicals.
For the purposes of the invention, the term *"volatile oil"* means any oil that is capable of evaporating on contact with the skin in less than one hour, at room temperature and atmospheric pressure. The volatile oil is a volatile cosmetic compound, which is liquid at room temperature, especially having a nonzero vapour pressure, at room temperature and atmospheric pressure, in particular having a vapour pressure ranging from 0.13 Pa to 40 000 Pa ($10^{-3}$ to 300 mmHg), in particular ranging from 1.3 Pa to 13 000 Pa (0.01 to 100 mmHg) and more particularly ranging from 1.3 Pa to 1300 Pa (0.01 to 10 mmHg).

## Volatile oils

The volatile oils may be hydrocarbon-based oils or silicone oils.
Among the volatile hydrocarbon-based oils containing from 8 to 16 carbon atoms, mention may be made especially of branched $C_8$-$C_{16}$ alkanes, such as $C_8$-$C_{16}$ isoalkanes (also known as isoparaffins), isododecane, isodecane, isohexadecane and, for example, the oils sold under the trade names Isopar or Permethyl, branched $C_8$-$C_{16}$ esters, such as isohexyl neopentanoate, and mixtures thereof. Preferably, the volatile hydrocarbon-based oil is chosen from volatile hydrocarbon-based oils containing from 8 to 16 carbon atoms, and mixtures thereof, in particular from isododecane, isodecane and isohexadecane, and is especially isohexadecane.
Mention may also be made of volatile linear alkanes comprising from 8 to 16 carbon atoms, in particular from 10 to 15 carbon atoms and more particularly from 11 to 13 carbon atoms, for instance n-dodecane ($C_{12}$) and n-tetradecane ($C_{14}$) sold by Sasol under the respective references Parafol 12-97 and Parafol 14-97, and also mixtures thereof, the undecane-tridecane mixture, mixtures of n-undecane ($C_{11}$) and of n-tridecane ($C_{13}$) obtained in Examples 1 and 2 of patent application WO 2008/155 059 from the company Cognis, and mixtures thereof.
Volatile silicone oils that may be mentioned include linear volatile silicone oils such as hexamethyldisiloxane, octamethyltrisiloxane, decamethyltetrasiloxane, tetradecamethylhexasiloxane, hexadecamethylheptasiloxane and dodecamethylpentasiloxane.
Volatile cyclic silicone oils that may be mentioned include hexamethylcyclotrisiloxane, octamethylcyclotetrasiloxane, decamethylcyclopentasiloxane and dodecamethylcyclohexasiloxane.

## Non-volatile oils

The non-volatile oils may be chosen especially from non-volatile hydrocarbon-based, fluoro and/or silicone oils.
Non-volatile hydrocarbon-based oils that may especially be mentioned include:

- hydrocarbon-based oils of animal origin,
- hydrocarbon-based oils of plant origin, synthetic ethers containing from 10 to 40 carbon atoms, such as dicapryl ether,
- synthetic esters, such as the oils of formula $R_1COOR_2$, in which $R_1$ represents a linear or branched fatty acid residue comprising from 1 to 40 carbon atoms and $R_2$ represents a hydrocarbon-based chain, which is especially branched, containing from 1 to 40 carbon atoms, on condition that $R_1 + R_2 \geq 10$. The esters may be chosen especially from fatty acid alcohol esters, for instance cetostearyl octanoate, isopropyl alcohol esters such as isopropyl myristate or isopropyl palmitate, ethyl palmitate, 2-ethylhexyl palmitate, isopropyl stearate, octyl stearate, hydroxylated esters, such as isostearyl lactate or octyl hydroxystearate, alkyl or polyalkyl ricinoleates, hexyl laurate, neopentanoic acid esters, such as isodecyl neopentanoate or isotridecyl neopentanoate, and isononanoic acid esters, such as isononyl

isononanoate or isotridecyl isononanoate,

- polyol esters and pentaerythritol esters, such as dipentaerythrityl tetrahydroxy stearate/tetraisostearate,
- fatty alcohols that are liquid at room temperature, with a branched and/or unsaturated carbon-based chain containing from 12 to 26 carbon atoms, for instance 2-octyldodecanol, isostearyl alcohol and oleyl alcohol,
- $C_{12}$-$C_{22}$ higher fatty acids, such as oleic acid, linoleic acid, linolenic acid, and mixtures thereof,
- non-phenyl silicone oils, for instance caprylyl methicone, and
- phenyl silicone oils, for instance phenyl trimethicones, phenyl dimethicones, phenyltrimethylsiloxydiphenylsiloxanes, diphenyl dimethicones, diphenylmethyldiphenyltrisiloxanes and 2-phenylethyl trimethylsiloxysilicates, dimethicones or phenyl trimethicone with a viscosity of less than or equal to 100 cSt, and trimethyl-pentaphenyl-trisiloxane, and mixtures thereof; and also mixtures of these various oils.

Preferably, a composition according to the invention comprises volatile and/or non-volatile silicone oils. Such silicone oils are particularly appreciated when the lipophilic gelling agent is an organopolysiloxane elastomer.
A composition according to the invention may comprise from 5% to 95% by weight, better still from 5% to 40% by weight and preferably from 7% to 35% by weight of oil(s) relative to the total weight of said composition.
As mentioned above, the gelled oily phase according to the invention may have a threshold stress of greater than 1.5 Pa and preferably greater than 10 Pa.
The gelled oily phase according to the invention may have a threshold stress of lower than 10 000 Pa preferably lower than 5 000 Pa.
This threshold stress value reflects a gel-type texture of this oily phase.

**DYESTUFFS**

A composition according to the invention may also comprise at least one particulate or non-particulate, water-soluble or water-insoluble dyestuff, preferably in a proportion of at least 0.01% by weight relative to the total weight of the composition.
For obvious reasons, this amount is liable to vary significantly with regard to the intensity of the desired colour effect and of the colour intensity afforded by the dyestuffs under consideration, and its adjustment clearly falls within the competence of a person skilled in the art.
A composition according to the invention may comprise from 0.01% to 30% by weight, especially from 0.1% to 25% by weight, in particular from 1% to 25% by weight and preferably from 5% to 25% by weight of dyestuffs relative to the total weight of said composition.
As stated above, the dyestuffs that are suitable for use in the invention may be water-soluble, but may also be liposoluble.
For the purposes of the invention, the term *"water-soluble dyestuff"* means any natural or synthetic, generally organic compound, which is soluble in an aqueous phase or water-miscible solvents and which is capable of imparting colour.
As water-soluble dyes that are suitable for use in the invention, mention may be made especially of synthetic or natural water-soluble dyes, for instance FDC Red 4, DC Red 6, DC Red 22, DC Red 28, DC Red 30, DC Red 33, DC Orange 4, DC Yellow 5, DC Yellow 6, DC Yellow 8, FDC Green 3, DC Green 5, FDC Blue 1, betanine (beetroot), carmine, copper chlorophylline, methylene blue, anthocyanins (enocianin, black carrot, hibiscus and elder), caramel and riboflavin.
The water-soluble dyes are, for example, beetroot juice and caramel.
For the purposes of the invention, the term *"liposoluble dyestuff"* means any natural or synthetic, generally organic compound, which is soluble in an oily phase or in solvents that are miscible with a fatty substance, and which is capable of imparting colour.
As liposoluble dyes that are suitable for use in the invention, mention may be made especially of synthetic or natural liposoluble dyes, for instance DC Red 17, DC Red 21, DC Red 27, DC Green 6, DC Yellow 11, DC Violet 2, DC Orange 5, Sudan red, carotenes (β-carotene, lycopene), xanthophylls (capsanthin, capsorubin, lutein), palm oil, Sudan brown, quinoline yellow, annatto and curcumin.
The colouring particulate materials may be present in a proportion of from 0.01% to 30% by weight, especially from 0.1% to 25% by weight, in particular from 1% to 25% by weight and preferably from 5% to 25% by weight relative to the total weight of said composition containing them.
They may especially be pigments, nacres and/or particles with metallic tints.
The term *"pigments"* should be understood as meaning white or coloured, mineral or organic particles that are insoluble in an aqueous solution, which are intended to colour and/or opacify the composition containing them.
A composition according to the invention may comprise from 0.01% to 30% by weight, especially from 0.1% to 25% by weight, in particular from 1% to 25% by weight and preferably from 5% to 25% by weight of pigments relative

to the total weight of said composition.

Preferably, when the composition according to the invention is a makeup composition, it may comprise at least 3% by weight of pigments, relative to the total weight of said composition.

The pigments may be white or coloured, and mineral and/or organic.

As mineral pigments that may be used in the invention, mention may be made of titanium oxide, titanium dioxide, zirconium oxide, zirconium dioxide, cerium oxide or cerium dioxide and also zinc oxide, iron oxide or chromium oxide, ferric blue, manganese violet, ultramarine blue and chromium hydrate, and mixtures thereof.

It may also be a pigment having a structure that may be, for example, of sericite/brown iron oxide/titanium dioxide/silica type. Such a pigment is sold, for example, under the reference Coverleaf NS or JS by the company Chemicals and Catalysts, and has a contrast ratio in the region of 30.

They may also be pigments having a structure that may be, for example, of silica microsphere type containing iron oxide. An example of a pigment having this structure is the product sold by the company Miyoshi under the reference PC Ball PC-LL-100 P, this pigment consisting of silica microspheres containing yellow iron oxide.

Advantageously, the pigments in accordance with the invention are iron oxides and/or titanium dioxides.

The term *"nacres"* should be understood as meaning iridescent or non-iridescent coloured particles of any shape, especially produced by certain molluscs in their shell or alternatively synthesized, which have a colour effect via optical interference.

A composition according to the invention may comprise from 0% to 15% by weight of nacres relative to the total weight of said composition.

The nacres may be chosen from nacreous pigments such as titanium mica coated with an iron oxide, titanium mica coated with bismuth oxychloride, titanium mica coated with chromium oxide, titanium mica coated with an organic dye and also nacreous pigments based on bismuth oxychloride. They may also be mica particles, at the surface of which are superposed at least two successive layers of metal oxides and/or of organic dyestuffs.

Examples of nacres that may also be mentioned include natural mica coated with titanium oxide, with iron oxide, with natural pigment or with bismuth oxychloride.

Among the commercially available nacres that may be mentioned are the nacres Timica, Flamenco and Duochrome (on mica base) sold by the company Engelhard, the Timiron nacres sold by the company Merck, the Prestige nacres on mica base sold by the company Eckart and the Sunshine nacres on synthetic mica base sold by the company Sun Chemical.

The nacres may more particularly have a yellow, pink, red, bronze, orange, brown and/or coppery colour or tint.

Advantageously, the nacres in accordance with the invention are micas coated with titanium dioxide or with iron oxide, and also bismuth oxychloride.

For the purposes of the present invention, the term *"particles with a metallic tint"* means any compound whose nature, size, structure and surface finish allow it to reflect the incident light, especially in a non-iridescent manner.

The particles with a metallic tint that may be used in the invention are in particular chosen from:

- particles of at least one metal and/or of at least one metal derivative,
- particles comprising a monomaterial or multimaterial, organic or mineral substrate, at least partially coated with at least one layer with a metallic tint comprising at least one metal and/or at least one metal derivative, and
- mixtures of said particles.

Among the metals that may be present in said particles, mention may be made, for example, of Ag, Au, Cu, Al, Ni, Sn, Mg, Cr, Mo, Ti, Zr, Pt, Va, Rb, W, Zn, Ge, Te and Se, and mixtures or alloys thereof. Ag, Au, Cu, Al, Zn, Ni, Mo and Cr and mixtures or alloys thereof (for example bronzes and brasses) are preferred metals.

The term *"metal derivatives"* denotes compounds derived from metals, especially oxides, fluorides, chlorides and sulfides.

Illustrations of these particles that may be mentioned include aluminium particles, such as those sold under the names Starbrite 1200 EAC® by the company Siberline and Metalure® by the company Eckart and glass particles coated with a metallic layer, especially those described in documents JP-A-09188830, JP-A-10158450, JP-A-10158541, JP-A-07258460 and JP-A-05017710.

Hydrophobic treatment of the dyestuffs

The pulverulent dyestuffs as described previously may be totally or partially surface-treated, with a hydrophobic agent, to make them more compatible with the oily phase of the composition of the invention, especially so that they have good wettability with oils. Thus, these treated pigments are well dispersed in the oily phase.

Hydrophobic-treated pigments are described especially in document EP-A-1 086 683.

The hydrophobic-treatment agent may be chosen from silicones such as methicones, dimethicones and perfluoroalkylsilanes; fatty acids, such as stearic acid; metal soaps, such as aluminium dimyristate, the aluminium salt of hydrogenated tallow glutamate; perfluoroalkyl phosphates, polyhexafluoropropylene oxides; perfluoropolyethers; amino acids; N-acylamino acids or salts thereof; lecithin, isopropyl triisostearyl titanate, isostearyl sebacate, and mixtures thereof.

The term "alkyl" mentioned in the compounds cited above especially denotes an alkyl group containing from 1 to 30 carbon atoms and preferably containing from 5 to 16 carbon atoms.

## POLAR ADDITIVE(S)

Advantageously, a composition according to the invention may also comprise one or more polar additive(s), especially when it contains pigments.

According to the present invention, the use of such a polar additive facilitates the homogenization of the dispersion in the presence of pigments.

The polar additive may be chosen from compounds considered as good hydrogen bond donors or acceptors, for instance fatty alcohols, fatty acids, diols and esters, and mixtures thereof.

According to one embodiment, the polar additives of the invention may be polar oils, for instance:

- hydrocarbon-based plant oils with a high content of triglycerides consisting of fatty ($C_8$ to $C_{24}$) acid esters of glycerol in which the fatty acids may have varied chain lengths, these chains possibly being linear or branched, and saturated or unsaturated; these oils are in particular wheatgerm oil, corn oil, sunflower oil, shea oil, castor oil, sweet almond oil, macadamia oil, apricot oil, soybean oil, rapeseed oil, cottonseed oil, alfalfa oil, poppy seed oil, pumpkin seed oil, sesame seed oil, marrow oil, avocado oil, hazelnut oil, grapeseed oil, blackcurrant seed oil, evening primrose oil, millet oil, barley oil, quinoa oil, olive oil, rye oil, safflower oil, candlenut oil, passion flower oil and musk rose oil; or caprylic/capric acid triglycerides such as those sold by the company Stearineries Dubois or those sold under the names Miglyol 810, 812 and 818 by the company Dynamit Nobel;
- synthetic oils of formula $R_5COOR_6$ in which $R_5$ represents a linear or branched higher fatty acid residue comprising from 7 to 40 carbon atoms and $R_6$ represents a branched hydrocarbon-based chain containing from 3 to 40 carbon atoms, for instance purcellin oil (cetostearyl octanoate), isononyl isononanoate or $C_{12}$ to $C_{15}$ alkyl benzoate;
- synthetic esters and ethers such as isopropyl myristate, 2-ethylhexyl palmitate, alcohol or polyalcohol octanoates, decanoates or ricinoleates, hydroxylated esters such as isostearyl lactate, diisostearyl malate; and pentaerythritol esters;
- fatty acids containing from 12 to 22 carbon atoms, for instance oleic acid, linoleic acid or linolenic acid; and
- mixtures thereof.

According to another embodiment, the polar additives of the invention may be amphiphilic compounds.

The amphiphilic compound(s) that may be used in the composition of the invention comprise a lipophilic part linked to a polar part, the lipophilic part possibly comprising a carbon-based chain containing at least 8 carbon atoms, especially from 18 to 32 carbon atoms and better still from 18 to 28 carbon atoms. Preferably, the polar part of this or these amphiphilic compound(s) is the residue of a compound chosen from alcohols and poly alcohols containing from 1 to 12 hydroxyl groups, polyoxyalkylenes comprising at least two oxyalkylene units and containing from 0 to 20 oxypropylene units and/or from 0 to 20 oxyethylene units.

In particular, the amphiphilic compound is an ester chosen from glyceryl, sorbitan or methylglucose hydroxystearates, oleates or isostearates, or alternatively branched $C_{12}$ to $C_{26}$ fatty alcohols such as octyldodecanol, and mixtures thereof. Among these esters, monoesters and mixtures of monoesters and diesters are particularly preferred.

The amphiphilic compounds may also be silicone-based. These amphiphilic silicones comprise a silicone part that is compatible with the highly silicone medium of the compositions of the invention, and a hydrophilic part that may be, for example, the residue of a compound chosen from alcohols and polyols, containing from 1 to 12 hydroxyl groups, polyoxyalkylenes comprising at least two oxyalkylene units and containing from 0 to 20 oxypropylene units and/or from 0 to 20 oxyethylene units. This hydrophilic part thus has affinity for the hydrophilic particles and promotes their dispersion in the silicone medium.

The polar additives of the invention may be agents for screening out UV-B and/or UV-A rays, the total amount of screening agents possibly being between 0.01% and 10% by weight relative to the total weight of the composition.

A composition according to the invention may comprise from 0.01% to 25% by weight, especially from 0.05% to 20% by weight and in particular from 0.05% to 15% by weight of polar additive(s).

## FILLERS

Advantageously, a composition according to the invention may also comprise one or more fillers conventionally

used in care and/or makeup compositions.

These fillers are colourless or white solid particles of any form, which are in a form that is insoluble and dispersed in the medium of the composition.

These fillers, of mineral or organic, natural or synthetic nature, give the composition containing them softness and give the makeup result a matt effect and uniformity. In addition, these fillers advantageously make it possible to combat various attacking factors such as sebum or sweat.

As illustrations of these fillers, mention may be made of talc, mica, silica, kaolin, poly-β-alanine powder and polyethylene powder, powders of tetrafluoroethylene polymers (Teflon®), lauroyllysine, starch, boron nitride, hollow polymer microspheres such as those of polyvinylidene chloride/acrylonitrile, for instance Expancel® (Nobel Industrie), acrylic acid copolymers, silicone resin microbeads (for example Tospearls® from Toshiba), polyorganosiloxane elastomer particles, precipitated calcium carbonate, magnesium carbonate, magnesium hydrogen carbonate, hydroxyapatite, barium sulfate, aluminium oxides, polyurethane powders, composite fillers, hollow silica microspheres, and glass or ceramic microcapsules. Use may also be made of particles which are in the form of hollow sphere portions, as described in patent applications JP-2003 128 788 and JP-2000 191 789.

In particular, such fillers may be present in a composition according to the invention in a content of between 0.01% and 40% by weight, especially between 0.1% and 35% by weight and in particular between 0.5% and 25% by weight relative to the total weight of the composition.

According to one embodiment of the invention, a composition may comprise at least solid particles such as pigments and/or fillers.

Advantageously, a composition according to the invention may comprise from 0.01% to 40% by weight, especially from 0.1% to 40% by weight, in particular from 0.1% to 35% by weight, preferably from 0.5% to 30% by weight and preferentially from 0.5% to 25% by weight of solid particles relative to the total weight of the composition.

## DISPERSANT

Advantageously, a composition according to the invention may also comprise a dispersant.

Such a dispersant may be a surfactant, an oligomer, a polymer or a mixture of several thereof.

According to one particular embodiment, a dispersant in accordance with the invention is a surfactant.

## ACTIVE AGENT

In particular for a care application, a composition according to the invention may comprise at least one moisturizer (also known as a humectant).

Preferably, the moisturizer is glycerol.

The moisturizer(s) may be present in the composition in a content ranging from 0.1% to 30% by weight, especially from 0.5% to 20% by weight or even from 1% to 15% by weight relative to the total weight of said composition.

As other active agents that may be used in the composition of the invention, examples that may be mentioned include vitamins and sunscreens, and mixtures thereof.

Preferably, a composition according to the invention comprises at least one active agent.

It is a matter of routine operations for a person skilled in the art to adjust the nature and the amount of the additives present in the compositions in accordance with the invention such that the desired cosmetic properties thereof are not thereby affected.

According to one embodiment, a composition of the invention may advantageously be in the form of a composition for caring for the skin and/or keratin fibres, the body or the face, in particular the face.

According to another embodiment, a composition of the invention may advantageously be in the form of a composition for making up the skin and/or keratin fibres, the body or the face, in particular the face.

Thus, according to a sub-mode of this embodiment, a composition of the invention may advantageously be in the form of a makeup base composition.

A composition of the invention may advantageously be in the form of a foundation.

According to another sub-mode of this embodiment, a composition of the invention may advantageously be in the form of a composition for making up the skin and especially the face. It may thus be an eyeshadow or a face powder.

According to yet another sub-mode of this embodiment, a composition of the invention may advantageously be in the form of a lip product, especially a lipstick.

Such compositions are especially prepared according to the general knowledge of a person skilled in the art.

As emerges from the foregoing text, the device under consideration according to the invention proves to be particularly advantageous for a user wishing to apply a composition as defined above and to perceive a sensation of lightness during the application.

Throughout the description, including the claims, the term *"comprising a"* should be understood as being synonymous with *"comprising at least one"*, unless otherwise specified.

The terms *"between... and* and "*ranging from... to..."* should be understood as being inclusive of the limits, unless otherwise specified.

The invention is illustrated in greater detail by the example below. Unless otherwise mentioned, the amounts indicated are expressed as mass percentages.

## METHODOLOGY FOR THE OSCILLATING DYNAMIC RHEOLOGY MEASUREMENTS

These are harmonic-regime rheology measurements for measuring the elastic modulus.

The measurements are taken using a Haake RS600 rheometer on a product at rest, at 25°C with a plate-plate rotor Ø 60 mm and a 2 mm gap.

The harmonic-regime measurements make it possible to characterize the viscoelastic properties of the products. The technique consists in subjecting a material to a stress which varies sinusoidally over time and in measuring the response of the material to this stress. In a range in which the behaviour is linear viscoelastic behaviour (zone in which the strain is proportional to the stress), the stress ($\tau$) and the strain ($\gamma$) are two sinusoidal functions of time which are written in the following manner:

$$\tau(t) = \tau_0 \sin(\omega t)$$

$$\gamma(t) = \gamma_0 \sin(\omega t + \delta)$$

in which:

$\tau_0$ represents the maximum amplitude of the stress (Pa);
$\gamma_0$ represents the maximum amplitude of the strain (-);
$\omega$ = 2IIN represents the angular frequency (rad.s$^{-1}$) with N representing the frequency (Hz); and
$\delta$ represents the phase shift of the stress relative to the strain (rad).

Thus, the two functions have the same angular frequency, but they are shifted by an angle $\delta$. Depending on the phase shift $\delta$ between $\tau$(t) and $\gamma$(t), the behaviour of the system may be apprehended:

- if $\delta = 0$, the material is purely elastic;
- if $\delta = \Pi/2$, the material is purely viscous (Newtonian fluid); and
- if $0 < \delta < \Pi/2$, the material is viscoelastic.

In general, the stress and the strain are written in complex form:

$$\tau^*(t) = \tau_0 e^{i\omega t}$$

$$\gamma^*(t) = \gamma_0 e^{(i\omega t + \delta)}$$

A complex stiffness modulus, representing the overall resistance of the material to the strain, whether it is of elastic or viscous origin, is then defined by:

$$G^* = \tau^* / \gamma^* = G' + iG''$$

in which:

G' is the storage modulus or elastic modulus, which characterizes the energy stored and totally restituted during a cycle, G' = $(\tau_0/ \gamma_0) \cos \delta$; and
G" is the loss modulus or viscous modulus, which characterizes the energy dissipated by internal friction during a cycle, G" = $(\tau_0/ \gamma_0) \sin \delta$.

The parameter retained is the mean stiffness modulus G* recorded at the plateau measured at a frequency of 1 Hz.

**EXAMPLE: Foundation composition according to the invention**

A foundation formulation in accordance with the invention is prepared as described below.

Preparation of the aqueous phase

Water, glycerol and the preserving agent are weighed out in a beaker and stirred with a Rayneri blender at room temperature.
The sodium acrylamido-2-methylpropanesulfonate/hydroxyethyl acrylate copolymer is added with stirring at room temperature. The stirring is adjusted so as not to incorporate air into the mixture.
The mixture is left under moderate stirring for about 10 minutes at room temperature.

Preparation of the oily phase

The pigments are ground with 15% of silicone oil using a three-roll mill.
The ground material and the remaining oil are placed in a beaker and stirred with a Rayneri blender at room temperature.
The gel of silicone elastomer in dimethicone is added with moderate stirring at room temperature.
The gel gradually thickens.
The mixture is stirred for 20 minutes.

Preparation of the foundation formulation

The formulation is obtained by mixing the phases intended to form the foundation in accordance with the invention, in the proportions described hereafter in the table below.
The aqueous and oily gels are weighed out and then mixed with a Rayneri blender.

| Phase | Compounds | Total weight/weight % of the composition |
|---|---|---|
| *Phase A* | Water | qs 100 |
| | Glycerol | 5.00 |
| | Preserving agent | 0.80 |
| | Sodium acrylamido-2-methylpropanesulfonate/hydroxyethyl acrylate copolymer in powder form (Sepinov® EMT 10 sold by the company SEPPIC) | 2.40 |
| *Phase B* | Dimethicone 5 cSt | 12.00 |
| | 84.5% dimethicone/15.5% dimethicone crosspolymer (DC9041® sold by the company Dow Corning) (*% of dimethicone crosspolymer solids) | 10.00 (1.55*) |
| | Iron oxides coated with aluminium stearoyl glutamate (NAI-C33-9001-10, NAI-C33-7001-10 and NAI-C33-8001-10 sold by the company Miyoshi Kasei) | 2.40 |
| | Titanium dioxide coated with aluminium stearoyl glutamate (NAI-TAO-77891 sold by the company Miyoshi Kasei) | 15.60 |

The formula has a thick, dense, finely dispersed texture of gel/gel type. It is smooth and homogeneous and has very good cosmetic qualities, such as very good coverage. In addition, the texture is fresh and light on application to the skin. It has a high viscosity of about 110 poises.
The viscosity is measured at 25°C with a Rheomat 180 viscometer equipped with a No. 4 spindle, the measurement being taken after 10 minutes of rotation of the spindle (after which time stabilization of the viscosity and of the spin speed of the spindle is observed), at a shear of 200 $min^{-1}$.

Evaluation of the technical effect of the compositions

The formula obtained was then packaged:

- either in a device in accordance with the invention as illustrated in the attached figures,
- or in a jar.

Thirteen female volunteers aged between 20 and 55, having all types of skin except extreme types, then tested this formula in the two abovementioned packagings.

It emerges therefrom that when the composition is presented in a jar, the foundation texture has for certain women a greasy, compact and sparingly fondant aspect. The foundation then transforms when taken up with the fingers into a surprising, light or creamy texture that is rather easy to spread uniformly. However, more than half of the women declared that they had a problem with dosing. For three women who did not succeed in finding the right amount, the application and the makeup result were unsatisfactory (sensation that the foundation dried too quickly or, on the contrary, remained on the surface, marked lines).

When the formula is placed in the device according to the invention described above, more than half of the women had the feeling that the tip modified the texture and made it lighter/more airy, thereby facilitating the dosing and the ease of spreading.

The texture packaged in this device thus appears light, non-greasy, non-tacky, moisturizing, with no "mask" effect and easy to dose.

The device according to the invention thus makes it possible to erase the high consistency and the thickness that the formula has in the bulk, when it is in ajar.

## Claims

**1.** Device (2) for packaging and applying a cosmetic or dermatological product, comprising:

- a composition, especially a cosmetic composition, for making up and/or caring for keratin materials, in particular the skin and/or the lips, comprising at least one aqueous phase gelled with at least one hydrophilic gelling agent chosen from 2-acrylamido-2-methylpropanesulfonic acid polymers and copolymers and at least one oily phase gelled with at least one lipophilic gelling agent, with said phases forming a macroscopically homogeneous mixture,
- a container (5) containing said composition,
- an applicator head (10) fed with composition by the container (5), the applicator head (10) being arranged to dispense said composition through a dispensing member having at least one passage for the composition imposing a shear on the composition passing through it, in which the applicator head comprises a grate (19) defining an application surface (19a) for applying the product to a surface to be treated and also comprises a partitioned support member (22) for the grate (19), against which the grate (19) may rest at least during the application, having at least one partition (72) between two zones (78) where the product may pass through the support member.

**2.** Device according to claim 1, in which said dispensing member has a single passage.

**3.** Device according to claim 1 or 2, said grate (19) being a woven or nonwoven textile or an injection-moulded or extruded net.

**4.** Device according to any one of the preceding claims, the grate (19) being made of thermoplastic material, especially of polyamide 6.6 or of PP, PA or PET.

**5.** Device according to any one of the preceding claims, the grate (19) comprising a plurality of yarns (81) with a diameter d of between 2 $\mu$m and 1000 $\mu$m and better still between 50 $\mu$m and 250 $\mu$m.

**6.** Device according to any one of the preceding claims, the grate (19) comprising between 20 yarns/cm and 70 yarns/cm and better still between 28 yarns/cm and 35 yarns/cm.

**7.** Device according to any one of the preceding claims, the grate (19) being formed by a woven or a net, the meshes of the grate (19) each having an aperture of cross section S between 0.01 $mm^2$ and 1 $mm^2$ and better still between

0.01 $mm^2$ and 0.1 $mm^2$.

**8.** Device according to any one of the preceding claims, comprising a cap (93) for covering the applicator head (10) when the device is not in use.

**9.** Device according to any one of the preceding claims, comprising, as hydrophilic gelling agent, at least one copolymer of 2-acrylamido-2-methylpropanesulfonic acid and of hydroxyethyl acrylate.

**10.** Device according to any one of the preceding claims, in which said lipophilic gelling agent is chosen from particulate gelling agents, organopolysiloxane elastomers, semi-crystalline polymers, dextrin esters and hydrogen bonding polymers, hydrocarbon-based block copolymers, and mixtures thereof.

**11.** Device according to any one of the preceding claims, comprising as lipophilic gelling agent at least one organopolysiloxane elastomer preferably chosen from Dimethicone Crosspolymer, Dimethicone (and) Dimethicone Crosspolymer, Vinyl Dimethicone Crosspolymer, Dimethicone/Vinyl Dimethicone Crosspolymer, Dimethicone Crosspolymer-3, and in particular Dimethicone Crosspolymer and Dimethicone (and) Dimethicone Crosspolymer, more particularly at least one crosslinked silicone elastomer of INCI name Dimethicone (and) Dimethicone Crosspolymer, preferably with a dimethicone having a viscosity ranging from 1 to 100 cSt and in particular from 1 to 10 cSt at 25°C.

**12.** Device according to any one of the preceding claims, comprising, as lipophilic gelling agent, at least one silicone elastomer, in combination with at least one other lipophilic gelling agent.

**13.** Device according to any one of the preceding claims, containing, as hydrophilic gelling agent/lipophilic gelling agent system, a 2-acrylamido-2-methylpropanesulfonic acid polymer or copolymer/organopolysiloxane elastomer system.

**14.** Device according to any one of the preceding claims, containing the aqueous and oily phases in a gelled aqueous phase/gelled oily phase weight ratio of greater than 1, especially ranging from 60/40 to 90/10, preferably ranging from 60/40 to 80/20, preferentially from 60/40 to 70/30 and even more preferentially favouring a gelled aqueous phase/gelled oily phase weight ratio of 60/40 or 70/30.

**15.** Device according to any one of the preceding claims, also comprising at least solid particles such as pigments and/or fillers in said composition.

**16.** Cosmetic process for making up and/or caring for keratin materials, in particular the skin and/or the lips, using the device as defined according to any one of the preceding claims, comprising at least one step which consists in applying to said keratin materials the composition contained in the container.

## Patentansprüche

**1.** Vorrichtung (2) zum Verpacken und Applizieren eines kosmetischen oder dermatologischen Produkts, umfassend:

- eine Zusammensetzung, insbesondere eine kosmetische Zusammensetzung, zum Schminken und/oder Pflegen von Keratinmaterialien, insbesondere der Haut und/oder der Lippen, umfassend mindestens eine wässrige Phase, geliert mit mindestens einem hydrophilen gelbildenden Mittel, ausgewählt aus 2-Acrylamido-2-methylpropansulfonsäurepolymeren und -copolymeren, und mindestens eine ölige Phase, geliert mit mindestens einem lipophilen gelbildenden Mittel, wobei die Phasen ein makroskopisch homogenes Gemisch bilden,
- einen Behälter (5), enthaltend die Zusammensetzung,
- einen Applikatorkopf (10), dem Zusammensetzung durch den Behälter (5) zugeführt wird, wobei der Applikatorkopf (10) so angeordnet ist, dass die Zusammensetzung durch einen Abgabebestandteil mit mindestens einem Durchgang für die Zusammensetzung, welcher eine Scherung auf die Zusammensetzung, die durch ihn passiert, aufbringt, abgegeben wird, wobei der Applikatorkopf ein Gitter (19), welches eine Applikatoroberfläche (19a) definiert, zum Applizieren des Produkts auf eine Oberfläche, welche behandelt wird, umfasst und auch einen abgetrennten Trägerbestandteil (22) für das Gitter (19), an welchem das Gitter (19) mindestens während dem Applizieren anliegen kann, mit mindestens einer Abtrennung (72) zwischen zwei Zonen (78) umfasst, wobei das Produkt durch den Trägerbestandteil passieren kann.

**2.** Vorrichtung gemäß Anspruch 1, wobei der Abgabebestandteil einen einzigen Durchgang aufweist.

3. Vorrichtung gemäß Anspruch 1 oder 2, wobei das Gitter (19) ein Gewebe- oder Vliestextil oder ein Spritzguss- oder extrudiertes Netz ist.

4. Vorrichtung gemäß einem der vorhergehenden Ansprüche, wobei das Gitter (19) aus einem thermoplastischen Material, insbesondere aus Polyamid 6.6 oder aus PP, PA oder PET hergestellt ist.

5. Vorrichtung gemäß einem der vorhergehenden Ansprüche, wobei das Gitter (19) eine Vielzahl von Fäden (81) mit einem Durchmesser d von zwischen 2 $\mu$m und 1000 $\mu$m und noch besser zwischen 50 $\mu$m und 250 $\mu$m umfasst.

6. Vorrichtung gemäß einem der vorhergehenden Ansprüche, wobei das Gitter (19) zwischen 20 Fäden/cm und 70 Fäden/cm und noch besser zwischen 28 Fäden/cm und 35 Fäden/cm umfasst.

7. Vorrichtung gemäß einem der vorhergehenden Ansprüche, wobei das Gitter (19) durch ein Gewebe oder ein Netz gebildet wird, wobei die Maschen des Gitters (19) jeweils einen Öffnungsquerschnitt S zwischen 0,01 $mm^2$ und 1 $mm^2$ und noch besser zwischen 0,01 $mm^2$ und 0,1 $mm^2$ aufweisen.

8. Vorrichtung gemäß einem der vorhergehenden Ansprüche, umfassend einen Deckel (93) zum Bedecken des Applikatorkopfes (10), wenn die Vorrichtung nicht in Verwendung ist.

9. Vorrichtung gemäß einem der vorhergehenden Ansprüche, umfassend als hydrophiles gelbildendes Mittel mindestens ein Copolymer von 2-Acrylamido-2-methylpropansulfonsäure und von Hydroxyethylacrylat.

10. Vorrichtung gemäß einem der vorhergehenden Ansprüche, wobei das lipophile gelbildende Mittel aus teilchenförmigen gelbildenden Mitteln, Organopolysiloxanelastomeren, semikristallinen Polymeren, Dextrinestern und wasserstoffbindenden Polymeren, Blockcopolymeren auf Kohlenwasserstoff-Basis und Gemischen davon ausgewählt ist.

11. Vorrichtung gemäß einem der vorhergehenden Ansprüche, umfassend als lipophiles gelbildendes Mittel mindestens ein Organopolysiloxanelastomer, bevorzugt ausgewählt aus Dimethicone Crosspolymer, Dimethicone (and) Dimethicone Crosspolymer, Vinyl Dimethicone Crosspolymer, Dimethicone/Vinyl Dimethicone Crosspolymer, Dimethicone Crosspolymer-3, und insbesondere Dimethicone Crosspolymer und Dimethicone (and) Dimethicone Crosspolymer, stärker besonders mindestens einem vernetzten Siliconelastomer mit INCI-Name Dimethicone (and) Dimethicone Crosspolymer, bevorzugt mit einem Dimethicon mit einer Viskosität in einem Bereich von 1 bis 100 cSt und insbesondere von 1 bis 10 cSt bei 25°C.

12. Vorrichtung gemäß einem der vorhergehenden Ansprüche, umfassend als lipophiles gelbildendes Mittel mindestens ein Siliconelastomer in Kombination mit mindesten einem weiteren lipophilen gelbildenden Mittel.

13. Vorrichtung gemäß einem der vorhergehenden Ansprüche, enthaltend als hydrophiles gelbildendes Mittel/lipophiles gelbildendes Mittel-System ein 2-Acrylamido-2-methylpropansulfonsäurepolymer oder -copolymer/Organopolysiloxanelastomer-System.

14. Vorrichtung gemäß einem der vorhergehenden Ansprüche, enthaltend die wässrigen und öligen Phasen in einem gelierte wässrige Phase/gelierte ölige Phase-Gewichtsverhältnis von höher als 1, insbesondere in einem Bereich von 60/40 bis 90/10, vorzugsweise in einem Bereich von 60/40 bis 80/20, bevorzugt von 60/40 bis 70/30, und wobei noch stärker bevorzugt ein gelierte wässrige Phase/gelierte ölige Phase-Gewichtsverhältnis von 60/40 oder 70/30 favorisiert ist.

15. Vorrichtung gemäß einem der vorhergehenden Ansprüche, auch umfassend mindestens feste Teilchen wie Pigmente und/oder Füllstoffe in der Zusammensetzung.

16. Kosmetisches Verfahren zum Schminken und/oder Pflegen von Keratinmaterialien, insbesondere der Haut und/oder der Lippen, unter Verwendung der Vorrichtung wie gemäß einem der vorhergehenden Ansprüche definiert, umfassend mindestens einen Schritt, der in Applizieren der in dem Behälter enthaltenen Zusammensetzung auf die Keratinmaterialien besteht.

## Revendications

**1.** Dispositif (2) de conditionnement et d'application d'un produit cosmétique ou dermatologique, comprenant :

- une composition, notamment une composition cosmétique, de maquillage et/ou de soin des matières kératiniques, en particulier de la peau et/ou des lèvres, comprenant au moins une phase aqueuse gélifiée par au moins un agent gélifiant hydrophile choisi parmi des polymères et copolymères d'acide 2-acrylamido-2-méthylpropanesulfonique et au moins une phase huileuse gélifiée par au moins un agent gélifiant lipophile, lesdites phases formant un mélange macroscopiquement homogène,
- un récipient (5) contenant ladite composition,
- une tête applicatrice (10) alimentée avec la composition par le récipient (5), la tête applicatrice (10) étant agencée pour distribuer ladite composition à travers un élément de distribution comportant au moins un passage pour la composition imposant un cisaillement à la composition le traversant, dans lequel la tête applicatrice comprend une grille (19) définissant une surface d'application (19a) pour appliquer le produit sur une surface à traiter et comprend en outre un élément cloisonné de soutien (22) de la grille (19), contre lequel la grille (19) peut reposer au moins lors de l'application, comportant au moins une cloison (72) entre deux zones (78) où le produit peut traverser l'élément de soutien.

**2.** Dispositif selon la revendication 1, dans lequel ledit élément de distribution comporte un passage unique.

**3.** Dispositif selon la revendication 1 ou 2, ladite grille (19) étant un textile tissé ou non-tissé ou un filet moulé par injection ou extrudé.

**4.** Dispositif selon l'une quelconque des revendications précédentes, la grille (19) étant constituée de matériau thermoplastique, en particulier de polyamide 6.6 ou de PP, PA ou PET.

**5.** Dispositif selon l'une quelconque des revendications précédentes, la grille (19) comprenant une pluralité de fils (81) ayant un diamètre d compris entre 2 $\mu$m et 1000 $\mu$m et de préférence entre 50 $\mu$m et 250 $\mu$m.

**6.** Dispositif selon l'une quelconque des revendications précédentes, la grille (19) comprenant entre 20 fils/cm et 70 fils/cm et de préférence entre 28 fils/cm et 35 fils/cm.

**7.** Dispositif selon l'une quelconque des revendications précédentes, la grille (19) étant formée d'un tissé ou d'un filet, les mailles de la grille (19) ayant chacune une ouverture de section transversale S comprise entre 0,01 $mm^2$ et 1 $mm^2$ et plus préférablement entre 0,01 $mm^2$ et 0,1 $mm^2$.

**8.** Dispositif selon l'une quelconque des revendications précédentes, comprenant un capuchon (93) pour recouvrir la tête applicatrice (10) lorsque le dispositif n'est pas utilisé.

**9.** Dispositif selon l'une quelconque des revendications précédentes, comprenant, en tant qu'agent gélifiant hydrophile, au moins un copolymère d'acide 2-acrylamido-2-méthylpropanesulfonique et d'acrylate d'hydroxyéthyle.

**10.** Dispositif selon l'une quelconque des revendications précédentes, dans lequel ledit agent gélifiant lipophile est choisi parmi des agents gélifiants particulaires, des élastomères d'organopolysiloxane, des polymères semi-cristallins, des esters de dextrine et des polymères à liaison hydrogène, des copolymères séquencés hydrocarbonés, et des mélanges de ceux-ci.

**11.** Dispositif selon l'une quelconque des revendications précédentes, comprenant, en tant qu'agent gélifiant lipophile, au moins un élastomère d'organopolysiloxane de préférence choisi parmi Dimethicone Crosspolymer, Dimethicone (and) Dimethicone Crosspolymer, Vinyl Dimethicone Crosspolymer, Dimethicone/Vinyl Dimethicone Crosspolymer, Dimethicone Crosspolymer-3, et en particulier Dimethicone Crosspolymer et Dimethicone (and) Dimethicone Crosspolymer, plus particulièrement au moins un élastomère de silicone réticulé de nom INCI Dimethicone (and) Dimethicone Crosspolymer, de préférence avec une diméthicone ayant une viscosité dans la plage de 1 à 100 cSt et en particulier de 1 à 10 cSt à 25 °C.

**12.** Dispositif selon l'une quelconque des revendications précédentes, comprenant, en tant qu'agent gélifiant lipophile, au moins un élastomère de silicone, en combinaison avec au moins un autre agent gélifiant lipophile.

**13.** Dispositif selon l'une quelconque des revendications précédentes, contenant, en tant que système agent gélifiant hydrophile/agent gélifiant lipophile, un système de polymère ou copolymère d'acide 2-acrylamido-2-méthylpropanesulfonique / élastomère d'organopolysiloxane.

**14.** Dispositif selon l'une quelconque des revendications précédentes, contenant les phases aqueuse et huileuse dans un rapport en poids phase aqueuse gélifiée /phase huileuse gélifiée supérieur à 1, en particulier dans la plage de 60/40 à 90/10, de préférence dans la plage de 60/40 à 80/20, plus préférablement de 60/40 à 70/30 et encore plus préférablement favorisant un rapport en poids phase aqueuse gélifiée /phase huileuse gélifiée de 60/40 ou 70/30.

**15.** Dispositif selon l'une quelconque des revendications précédentes, comprenant en outre au moins des particules solides telles que des pigments et/ou des charges dans ladite composition.

**16.** Procédé cosmétique pour le maquillage et/ou le soin des matières kératiniques, en particulier la peau et/ou les lèvres, utilisant le dispositif tel que défini selon l'une quelconque des revendications précédentes, comprenant au moins une étape qui consiste à appliquer sur lesdites matières kératiniques la composition contenue dans le récipient.

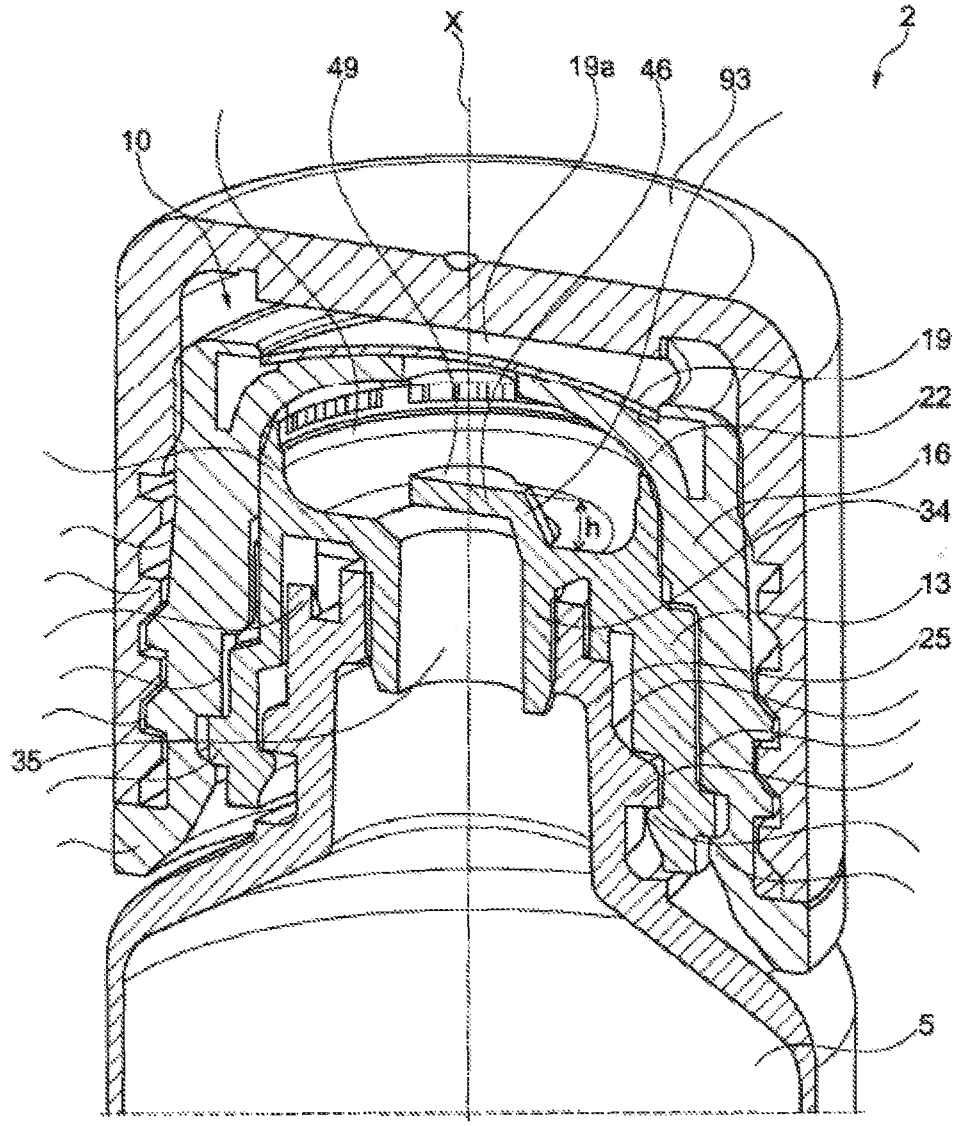


Fig. 1

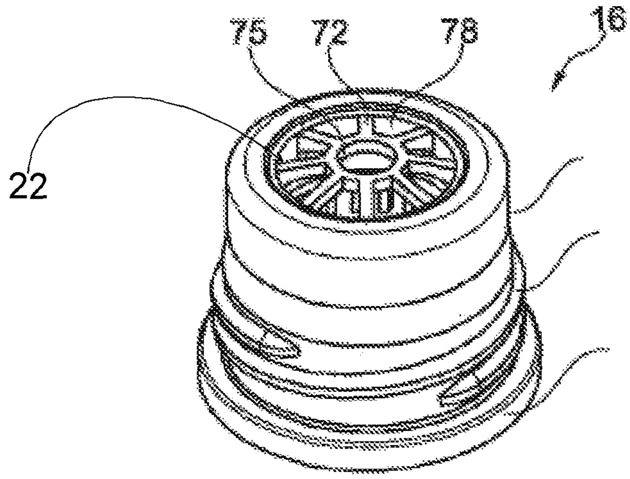


Fig. 2

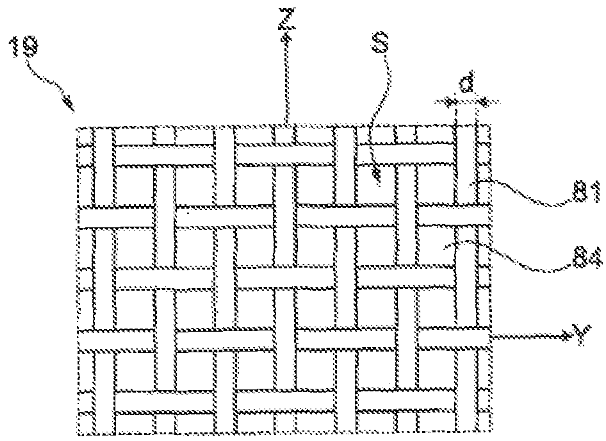


Fig. 3

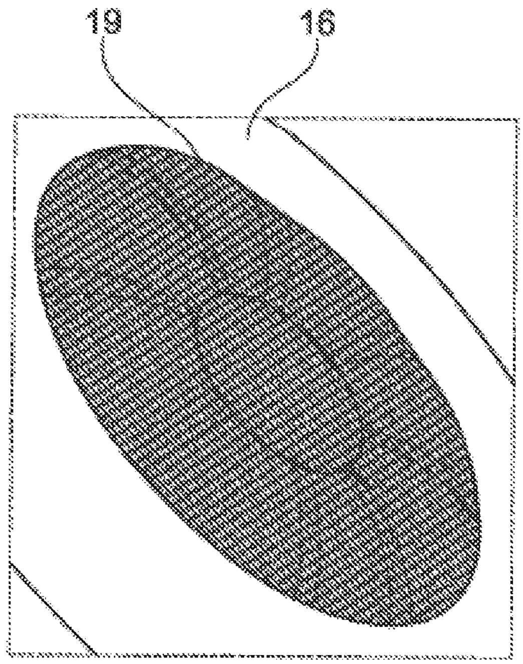


Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013093869 A
- US 2009311035 A
- US 5455340 A
- US 4017460 A
- US 3301848 A
- US 3589578 A
- US 4031307 A
- EP 0216479 A
- US 3915921 A
- US 4509949 A
- WO 9844012 A
- EP 0815928 B1
- US 7470725 B
- EP 295886 A
- EP 242219 A
- EP 285886 A
- EP 765656 A
- JP 61194009 A
- US 5538793 A
- EP 0951897 A
- US 5156911 A
- WO 0119333 A
- US 5981680 A
- US 2002005562 A
- US 5221534 A
- WO 2008155059 A
- JP 09188830 A
- JP 10158450 A
- JP 10158541 A
- JP 07258460 A
- JP 05017710 A
- EP 1086683 A
- JP 2003128788 B
- JP 2000191789 B


### Non-patent literature cited in the description


- Remington: The Science and Practice of Pharmacy. 1995, 282
- Kirk-Othmer's Encyclopaedia of Chemical Technology. Wiley Interscience, 1983, vol. 21, 492-507
- Kirk-Othmer's Encyclopaedia of Chemical Technology. 1982, vol. 3, 896-900
- KIRK-OTHMER'S ENCYCLOPAEDIA OF CHEMICAL TECHNOLOGY. vol. 15, 439-458
- **E.A. MACGREGOR ; C.T. GREENWOOD.** Polymers in Nature. John Wiley & Sons, 1980, 240-328
- **ROBERT L. DAVIDSON.** Handbook of Water-Soluble Gums and Resins. McGraw Hill Book Company, 1980
- **ROY L. WHISTLER.** Industrial Gums - Polysaccharides and their Derivatives. Academic Press Inc,
- **G. FONNUM ; J. BAKKE ; FK. HANSEN.** *Colloid Polym. Sci.,* 1993, vol. 271, 380-389
- **C.M. HANSEN.** The three-dimensional solubility parameters. *J. Paint Technol.,* 1967, vol. 39, 105
- **BRINKER C.J. ; SCHERER G.W.** Sol-Gel Science. Academic Press, 1990
- *Journal of the American Chemical Society,* February 1938, vol. 60, 309
- **VAN DE HULST, H.C.** Light Scattering by Small Particles. Wiley, 1957